# EUROPÄISCHE PATENTSCHRIFT

(11) **EP 1 381 596 B1**
(45) Veröffentlichungstag und Bekanntmachung des Hinweises auf die Patenterteilung: **27.09.2006**
(21) Anmeldenummer: 02766566.0
(22) Anmeldetag: 02.04.2002
(51) Int. Cl.: C07D 277/24, A01N 43/78, C07D 417/12

(54) **THIAZOLYLSUBSTITUIERTE CARBOCYCLISCHE 1,3-DIONE ALS SCHÄDLINGSBEKÄMPFUNGSMITTEL**
THIAZOLYL-SUBSTITUTED CARBOCYCLIC 1,3-DIONES AS PESTICIDAL AGENTS
1,3-DIONES CARBOCYCLIQUES A SUBSTITUTION THIAZOLYL EN TANT QUE PESTICIDES

(30) Priorität: 12.04.2001 DE 10118310
(43) Veröffentlichungstag der Anmeldung: 21.01.2004
(73) Patentinhaber: Bayer CropScience AG, 40789 Monheim (DE)
(72) Erfinder: FISCHER, Reiner, 40789 Monheim (DE); ULLMANN, Astrid, 50677 Köln (DE); TRAUTWEIN, Axel, 51467 Bergisch Gladbach (DE); DREWES, Mark, Wilhelm, 40764 Langenfeld (DE); ERDELEN, Christoph, verstorben (DE); DAHMEN, Peter, 41470 Neuss (DE); FEUCHT, Dieter, 40789 Monheim (DE); PONTZEN, Rolf, 42799 Leichlingen (DE); KUCK, Karl-Heinz, 40764 Langenfeld (DE); WACHENDORFF-NEUMANN, Ulrike, 56566 Neuwied (DE)
(86) Internationale Anmeldenummer: PCT/EP2002/003620
(87) Internationale Veröffentlichungsnummer: WO 2002/088098

(56) Entgegenhaltungen:
- EP-A- 0 368 592
- GB-A- 1 567 300
- US-A- 4 659 372

## Beschreibung

Die vorliegende Erfindung betrifft neue hetarylsubstituierte Cyclopentan- und Cyclohexan-1,3-dion-Derivate, mehrere Verfahren zu ihrer Herstellung und ihre Verwendung als bioaktive Wirkstoffe in der Landwirtschaft.

Bekannt ist 2-(2-Amino-thiazol-4-yl)-3-hydroxy-5,5-dimethyl-cyclohexen-2-enon; Pshenichnyi, V.N. et al; CNCIALi; Chem. Heterocycl. Compd. (Engl. Transl.) EN; 26, 1175-1178 (1990).

Insektizide Verbindungen, deren Struktur eine Thiazolylgruppe aufweist, sind in EP-A-368 592 beschrieben.

US4,659,372-A und GB-A-1 567 300 offenbaren 2-Aryl-1,3-cyclohexandion-enolester-Derivate unit insektizider Wirkung

Es wurden nun neue Verbindungen der Formel (I) gefunden, 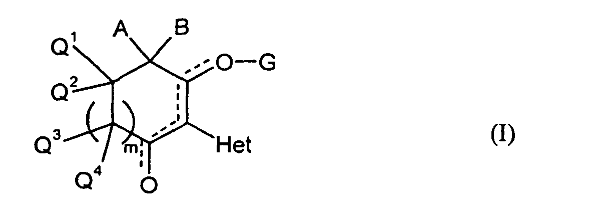 in welcher
- Het: für 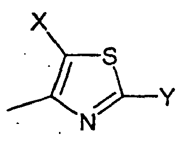 steht,
- m: für die Zahlen 0 oder 1 steht,
- X: für Wasserstoff, Chlor, Brom oder C₁-C₄-Alkyl steht,
- Y: für Chlor, Brom, C₁-C₄-Alkyl, C₁-C₄-Halogenalkyl, C₁-C₄-Alkoxy, C₁-C₄-Halogenalkoxy oder für die Gruppe 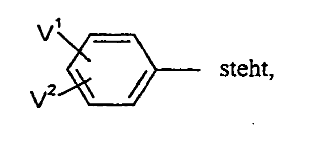
- V¹: für Wasserstoff, Fluor, Chlor, C₁-C₆-Alkyl, C₁-C₄-Alkoxy, C₁-C₂₋Halogenalkyl, C₁-C₂-Halogenalkoxy, Nitro, Cyano oder gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy steht,
- V²: für Wasserstoff, Fluor, Chlor, Brom, C₁-C₄-Alkyl, C₁ -C₄-Alkoxy, C₁₋C₂-Halogenalkyl oder C₁-C₂-Halogenalkoxy steht,
- V ¹ und V²: zusammen gemeinsam mit den Kohlenstoffatomen, an die sie gebunden sind, für einen gegebenenfalls durch Fluor oder Methyl substituierten 5- oder 6-gliedrigen Cyclus stehen, in welchem gegebenenfalls ein bis zwei Kohlenstoffatome durch Sauerstoff ersetzt sein können,
- A: für Wasserstoff, für jeweils gegebenenfalls einfach bis dreifach durch Fluor substituiertes C₁-C₈-Alkyl oder C₁-C₄-Alkoxy-C₁-C₂-alkyl, für jeweils gegebenenfalls einfach bis zweifach durch Fluor, Chlor, Methyl, Ethyl oder Methoxy substituiertes C₅-C₆-Cycloalkyl oder C₃₋C₆-Cycloalkyl-C₁-C₂-alkyl, in welchem gegebenenfalls ein Ringglied durch Sauerstoff oder Schwefel ersetzt ist oder jeweils gegebenenfalls einfach bis zweifach durch Fluor, Chlor, Brom, C₁-C₄-Alkyl, C₁-C₂₋Halogenalkyl, C₁-C₄-Alkoxy oder C₁-C₂-Halogenalkoxy substituiertes Phenyl oder Benzyl steht,
- B: für Wasserstoff oder C₁-C₄-Alkyl steht,
- A, B: und das Kohlenstoffatom an das sie gebunden sind, für gesättigtes C₅₋C₆-Cycloalkyl stehen, worin gegebenenfalls ein Ringglied durch Sauerstoff ersetzt ist und welches gegebenenfalls einfach durch C₁₋C₄-Alkyl, Trifluormethyl oder C₁-C₄-Alkoxy substituiert ist,
mit der Maßgabe, dass dann Q¹ nur für Wasserstoff oder C₁-C₄-Alkyl steht,
- A und Q¹: gemeinsam für gegebenenfalls einfach oder zweifach durch Methyl, Ethyl, Methoxy oder Ethoxy substitutiertes C₃-C₄-Alkandiyl stehen,
- Q¹: für Wasserstoff, C₁-C₄-Alkyl, C₁-C₄-Alkoxy-C₁-C₂-alkyl, oder gegebenenfalls durch Methyl oder Methoxy substituiertes C₃-C₆₋Cycloalkyl, worin gegebenenfalls eine Methylengruppe durch Sauerstoff ersetzt ist, oder jeweils gegebenenfalls einfach bis zweifach durch Fluor, Chlor, Brom, C₁-C₄-Alkyl, C₁-C₄-Alkoxy, Trifluormethyl oder Trifluormethoxy substituiertes Phenyl oder Benzyl steht,
- Q², Q³, Q⁴: unabhängig voneinander für Wasserstoff, Methyl oder Ethyl stehen,
- Q¹ und: Q² gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl stehen, in welchem gegebenenfalls ein Ringglied durch Sauerstoff ersetzt ist,
mit der Maßgabe, dass dann A nur für Wasserstoff oder C₁-C₄-Alkyl steht,
- G: für Wasserstoff (a) oder für eine der Gruppen 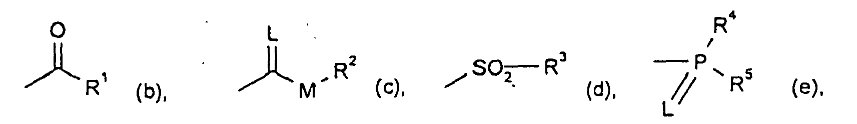 E (f) oder 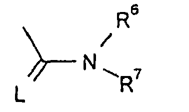 (g) steht
in welchen
E für ein Metallion oder ein Ammoniumion steht,
L für Sauerstoff oder Schwefel steht und
M für Sauerstoff oder Schwefel steht,
- R¹: für jeweils gegebenenfalls einfach bis fünffach durch Fluor oder Chlor substituiertes C₁-C₁₆-Alkyl, C₂-C₁₆-Alkenyl, C₁-C₄-Alkoxy-C₁₋C₂-alkyl, C₁-C₄-Alkylthio-C₁-C₂-alkyl, oder gegebenenfalls einfach bis zweifach durch Fluor, Chlor, C₁-C₄-Alkyl oder C₁-C₅-Alkoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls einfach bis zweifach durch Fluor, Chlor, Brom, Cyano, Nitro, C₁-C₄-Alkyl, C₁-C₄-Alkoxy, Trifluormethyl oder Trifluormethoxy substituiertes. Phenyl,
für jeweils gegebenenfalls einfach durch Fluor, Chlor, Brom, Methyl, Ethyl oder Trifluormethyl substituiertes Pyridyl oder Thienyl steht,
- R²: für C₁-C₆-Alkyl, C₂-C₁₆-Alkenyl oder C₁-C₄-Alkoxy-C₂-C₄-alkyl,
für gegebenenfalls einfach bis zweifach durch Methyl, Ethyl oder Methoxy substituiertes C₃-C₆-Cycloalkyl,
für jeweils gegebenenfalls einfach bis zweifach, durch Fluor, Chlor, Brom, Cyano, Nitro, C₁-C₄-Alkyl, C₁-C₄-Alkoxy, Trifluormethyl oder Trifluormethoxy substituiertes Phenyl oder Benzyl steht,
- R³: für gegebenenfalls einfach bis fünffach durch Fluor substituiertes C₁₋C₄-Alkyl oder für gegebenenfalls einfach bis zweifach durch Fluor, Chlor, Brom, C₁-C₄-Alkyl. C₁-C₄-Alkoxy, Trifluormethyl, Trifluormethoxy, Cyano oder Nitro substituiertes Phenyl steht,
- R⁴: für C₁-C₆-Alkyl, C₁-C₆-Alkoxy, C₁-C₆-Alkylamino, Di-(C₁-C₆₋alkyl)amino, C₁-C₆-Alkylthio,oder für jeweils gegebenenfalls einfach bis zweifach durch Fluor, Chlor, Brom, Nitro, Cyano, C₁-C₃-Alkoxy, Trifluormethoxy, C₁-C₃-Alkyl oder Trifluormethyl substituiertes Phenyl, Benzyl, Phenoxy oder Phenylthio steht,
- R⁵: für C₁-C₄-Alkyl, C₁-C₄-Alkoxy oder C₁-C₄-Alkylthio steht,
- R⁶: für Wasserstoff, C₁-C₆-Alkyl, C₃-C₆-Cycloalkyl, C₁-C₆-Alkoxy, C₃₋C₆-Alkenyl, C₁-C₆-Alkoxy-C₁-C₆-alkyl, für gegebenenfalls einfach bis zweifach durch Fluor, Chlor, Brom, Trifluormethyl, C₁-C₄-Alkyl oder C₁-C₄-Alkoxy substituiertes Phenyl, für gegebenenfalls durch Fluor, Chlor, Brom, Methyl, Ethyl, Trifluormethyl oder Methoxy substituiertes Benzyl steht,
- R⁷: für Wasserstoff, C₁-C₆-Alkyl oder C₃-C₆-Alkenyl steht,
- R⁶ und R⁷: zusammen mit dem Stickstoffatom, an das sie gebunden sind, für einen gegebenenfalls einfach bis zweifach durch Methyl oder Ethyl substituierten C₅-C₆-Alkylenrest stehen, in welchem gegebenenfalls eine Methylengruppe durch Sauerstoff oder Schwefel ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Die Verbindungen der Formel (I) können in Abhängigkeit von der Stellung des Substituenten G in den zwei isomeren Formen der Formeln (I-α) und (I-β) vorliegen, 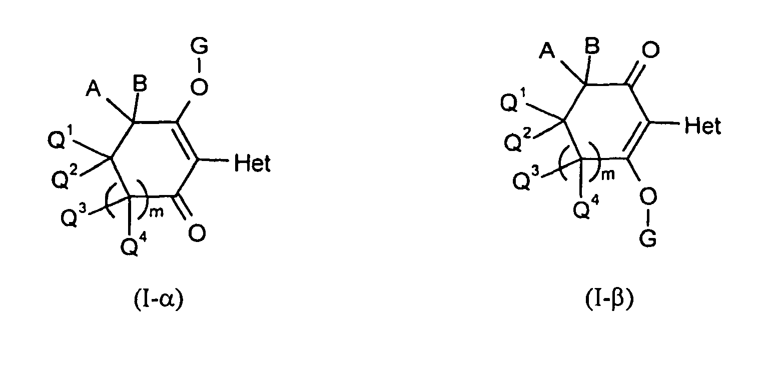 was durch die gestrichelte Linie in der Formel (I) zum Ausdruck gebracht werden soll.

Die Verbindungen der Formeln (I-α) und (I-β) können sowohl als Gemische als auch in Form ihrer reinen Isomeren vorliegen. Gemische der Verbindungen der Formeln (I-α) und (I-β) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung von Het für Thiazolyl und m für die Zahlen 0 und 1 ergeben sich folgende hauptsächliche Strukturen (I-1) und (1-2) 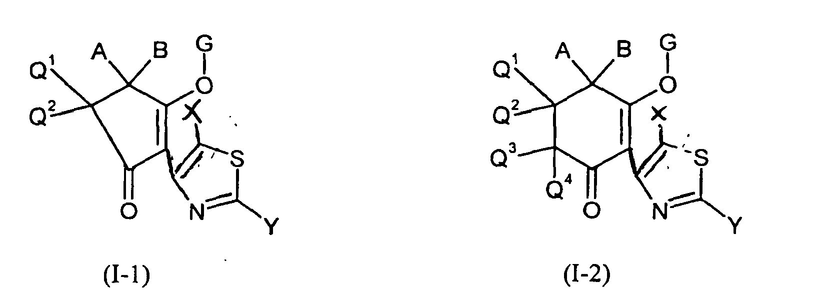 Unter Einbeziehung der verschiedenen Bedeutungen (a), (b), (c), (d), (e), (f) und (g) der Gruppe G ergeben sich folgende hauptsächliche Strukturen (I-1-a) bis (I-1-g) wenn m für die Zahl 0 (I-1) steht, 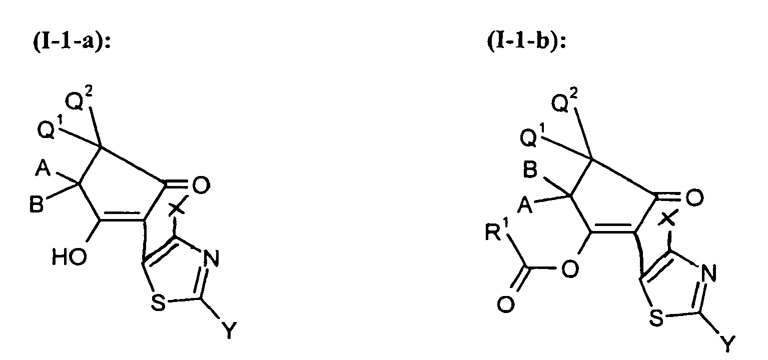 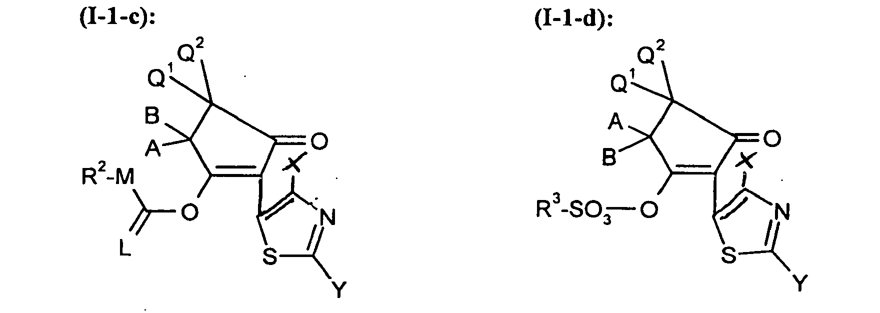 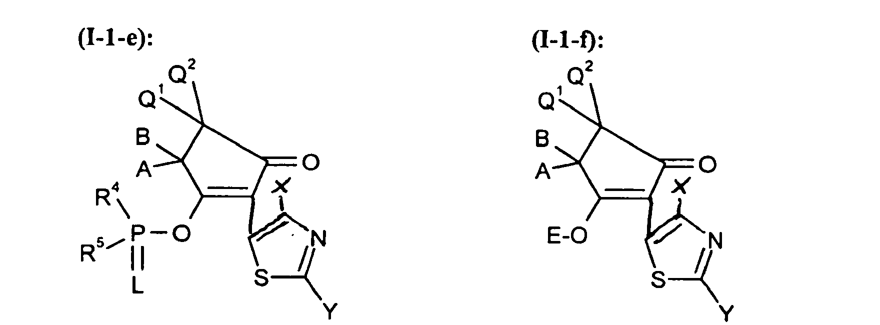 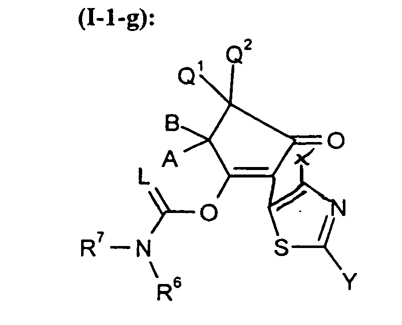 worin
X, Y, A, B, E, L, M, Q¹, Q², R¹, R², R³, R⁴, R⁵, R⁶ und R⁷ die oben angegebenen Bedeutungen besitzen, und

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g) wenn m für die Zahl 1 (I-2) steht, 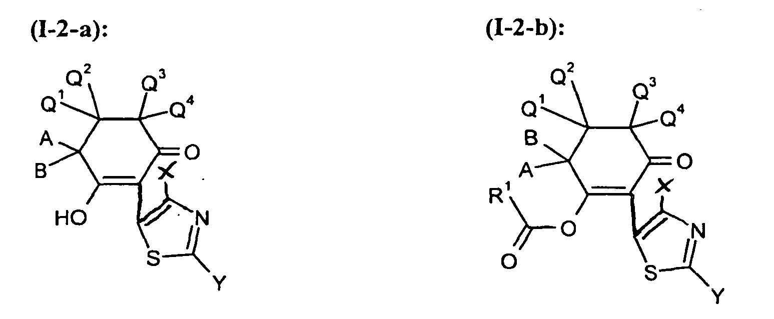 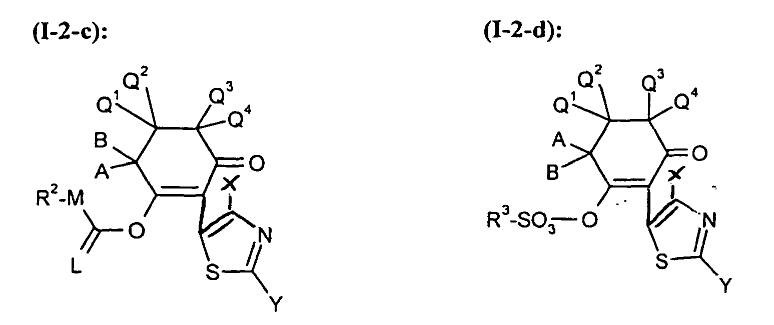 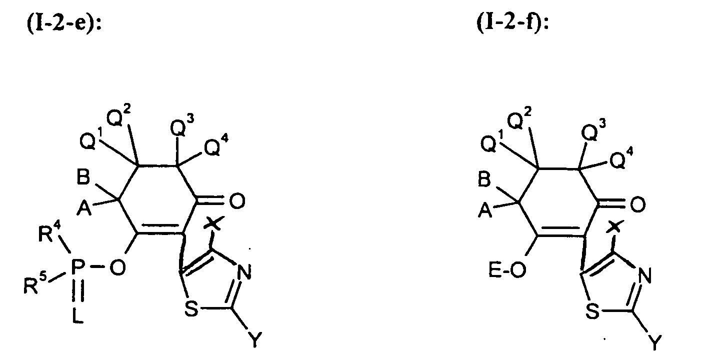 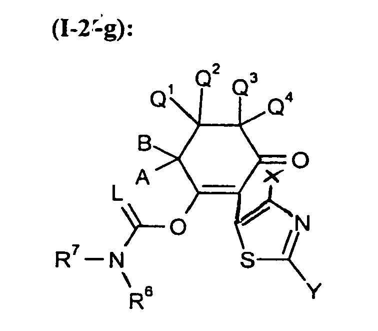 worin

A, B, E, L, M, Q¹, Q², Q³, Q⁴, X, Y, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte Cyclopentan-1,3-dione bzw. deren Enole der Formel (I-1-a) 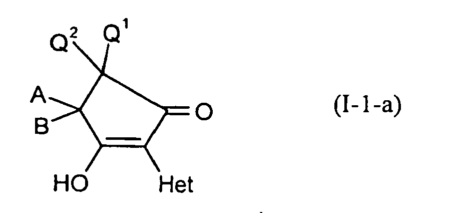 in welcher
   - A, B, Q¹, Q² und Het: die oben angegebenen Bedeutungen haben, wenn man
   Ketocarbonsäureester der Formel (II) 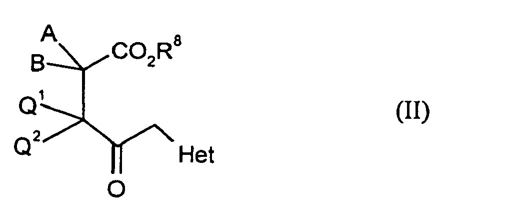 in welcher
   A, B, Q¹, Q² und Het die oben angegebenen Bedeutungen haben,
   und
   R⁸ für Alkyl (bevorzugt C₁ -C₆-Alkyl) steht,
   gegebenenfalls in Gegenwart eines Verdünnungsmittels und in Gegenwart einer Base intramolekular kondensiert.
   Weiterhin wurde gefunden,
(B) dass man substituierte Cylohexan-1,3-dione der Formel (1-2-a) erhält 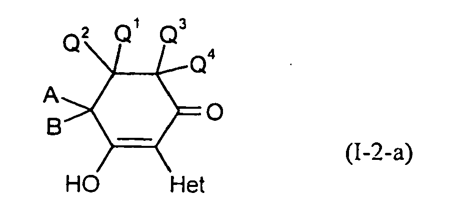 in welcher
   - A, B, Q¹, Q², Q³, Q⁴, und Het: die oben angegebene Bedeutungen haben, wenn man
   Ketocarbonsäureester der Formel (III) 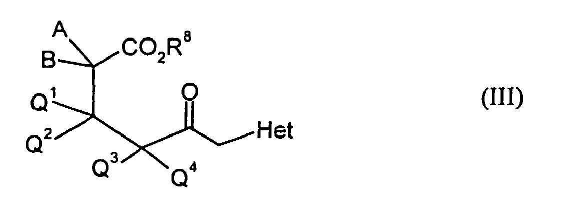 in welcher
   A, B, Q¹, Q², Q³, Q⁴, und Het die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Weiterhin wurde gefunden,
(C) dass man die Verbindungen der oben gezeigten Formeln (I-1-b) bis (I-2-b), in welcher A, B, Q¹, Q², Q³, Q⁴, m, R¹ und Het die oben angebenen Bedeutungen haben, erhält, wenn man Verbindungen der oben gezeigten Formeln (I-1-a) bis (1-2-a), in welchen A, B, Q¹, Q², Q³, Q⁴, m und Het die oben angegebenen Bedeutungen haben, jeweils
   (α) mit Säurehalogeniden der Formel (IV) 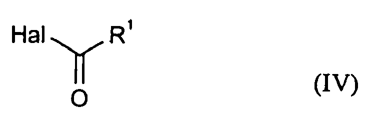 in welcher
      - R¹: die oben angegebene Bedeutung hat und
      - Hal: für Halogen (insbesondere Chlor oder Brom) steht
      oder
   (β) mit Carbonsäureanhydriden der Formel (V)

      R¹-CO-O-CO-R¹ (V)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
   (D) dass man die Verbindungen der oben gezeigten Formeln (I-1-c) bis (I-2-c), in welchen A, B, Q¹, Q², Q³, Q⁴, m, R², M und Het die oben angegebenen Bedeutungen haben und L für Sauerstoff steht, erhält, wenn man Verbindungen der oben gezeigten Formeln (I-1-a) bis (1-2-a), in welcher A, B, Q¹, Q², Q³, Q⁴, m und Het die oben angegebenen Bedeutungen haben, jeweils
      mit Chlorameisensäureestem oder Chlorameisensäurethioestern der Formel (VI)

      R²-M-CO-Cl (VI)

      in welcher
      - R² und M: die oben angegebenen Bedeutungen haben,
      gegebenenfalls in Gegenwart eines Verdünnungsmittels und gegebenenfalls in Gegenwart eines Säurebindemittels umsetzt;
   (E) dass man Verbindungen der oben gezeigten Formeln (I-1-c) bis (I-2-c), in welcher A, B, Q¹, Q², Q³, Q⁴, m, R², M und Het die oben angegebenen Bedeutungen haben und L für Schwefel steht, erhält, wenn man Verbindungen der oben gezeigten Formeln (I-1-a) bis (1-2-a), in welcher A, B, Q¹, Q², Q³, Q⁴, m und Het die oben angegebenen Bedeutungen haben, jeweils
      mit Chlormonothioameisensäureestern oder Chlordithioameisensäureestern der Formel (VII) 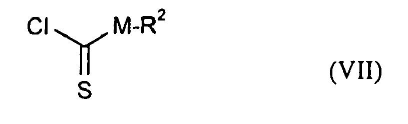 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      und
   (F) dass man Verbindungen der oben gezeigten Formeln (I-1-d) bis (I-2-d), in welcher A, B, Q¹, Q², Q³, Q⁴, m, R³ und Het die oben angegebenen Bedeutungen haben, erhält, wenn man Verbindungen der oben gezeigten Formeln (I-1-a) und (I-2-a), in welcher A, B, Q¹, Q², Q³, Q⁴, m und Het die oben angegebenen Bedeutungen haben, jeweils
      mit Sulfonsäurechloriden der Formel (VIII)

      R³-SO₂-Cl (VIII)

      in welcher
      - R³: die oben angegebene Bedeutung hat,
      gegebenenfalls in Gegenwart eines Verdünnungsmittels und gegebenenfalls in Gegenwart eines Säurebindemittels umsetzt,
   (G) dass man Verbindungen der oben gezeigten Formeln (I-1-e) bis (I-2-e), in welcher A, B, L, Q¹, Q², Q³, Q⁴, m, R⁴, R⁵ und Het die oben angegebenen Bedeutungen haben, erhält, wenn man Verbindungen der oben gezeigten Formeln (I-1-a) bis (1-2-a), in welcher A, B, Q¹, Q², Q³, Q⁴, m und Het die oben angegebenen Bedeutungen haben, jeweils
      mit Phosphorverbindungen der Formel (IX) 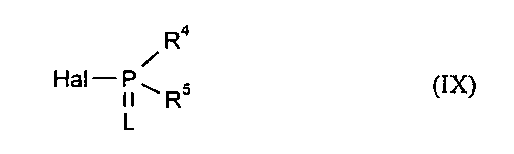 in welcher
      - L, R⁴ und R⁵: die oben angegebenen Bedeutungen haben und
      - Hal: für Halogen (insbesondere Chlor oder Brom) steht,
      gegebenenfalls in Gegenwart eines Verdünnungsmittels und gegebenenfalls in Gegenwart eines Säurebindemittels umsetzt,
   (H) dass man Verbindungen der oben gezeigten Formeln (I-1-f) bis (1-2-f), in welcher A, B, E, Q¹, Q², Q³, Q⁴, m und Het die oben angegebenen Bedeutungen haben, erhält, wenn man Verbindungen der Formeln (I-1-a) bis (I-2-a), in welchen A, B, Q¹, Q², Q³, Q⁴, m und Het die oben angegebenen Bedeutungen haben, jeweils
      mit Metallverbindungen oder Aminen der Formeln (X) oder (XI)

      Me(OR¹¹)ₜ (X)

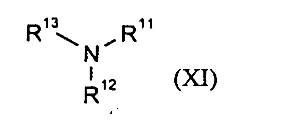

      in welchen
      - Me: für ein ein- oder zweiwertiges Metall (bevorzugt ein Alkali- oder Erdalkalimetall wie Lithium, Natrium, Kalium, Magnesium oder Calcium),
      - t: für die Zahl 1 oder 2 und
      - R¹¹, R¹², R¹³: unabhängig voneinander für Wasserstoff oder Alkyl (bevorzugt C₁-C₈-Alkyl) stehen,
      gegebenenfalls in Gegenwart eines Verdünnungsmittels umsetzt,
   (I) dass man Verbindungen der oben gezeigten Formeln (I-1-g) bis (I-2-g), in welcher A, B, L, Q¹, Q², Q³, Q⁴, m, R⁶, R⁷ und Het die oben angegebenen Bedeutungen haben, erhält, wenn man Verbindungen der oben gezeigten Formeln (I-1-a) bis (I-2-a), in welcher A, B, Q¹, Q², Q³, Q⁴, m und Het die oben angegebenen Bedeutungen haben, jeweils
      (α) mit Isocyanaten oder Isothiocyanaten der Formel (XII)

         R⁶-N=C=L (XII)

         in welcher
         - R⁶ und L: die oben angegebenen Bedeutungen haben,
         gegebenenfalls in Gegenwart eines Verdünnungsmittels und gegebenenfalls in Gegenwart eines Katalysators umsetzt oder
      (ß) mit Carbamidsäurechloriden oder Thiocarbamidsäurechloriden der Formel (XIII) 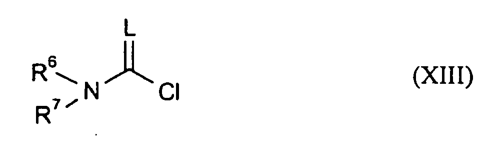 in welcher
         L, R⁶ und R⁷ die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) in Abhängigkeit von den Substituenten eine sehr gute Wirksamkeit sowohl als Schädlingsbekämpfungsmittel, vorzugsweise als Insektizide und Akarizide als auch als Herbizide und Fungizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- Het: steht ganz besonders bevorzugt für 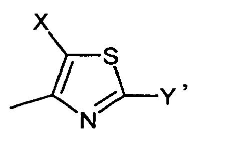
- m: steht ganz besonders bevorzugt für die Zahlen 0 oder 1,
- X: steht ganz besonders bevorzugt für Wasserstoff, Chlor, Brom, Methyl, Ethyl, n-Propyl oder iso-Propyl,
- Y: steht ganz besonders bevorzugt für die Gruppe 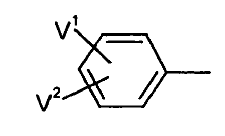
- V¹: steht ganz besonders bevorzugt für Wasserstoff, Fluor, Chlor, Brom, Methyl, Ethyl, n-Propyl, iso-Propyl, n-Butyl, iso-Butyl, tert-Butyl, Methoxy, Ethoxy, n-Propoxy, iso-Propoxy, Trifluormethyl, Trifluormethoxy oder 4-Chlorphenoxy,
- V²: steht ganz besonders bevorzugt für Wasserstoff, Fluor, Chlor, Brom, Methyl, Methoxy oder Trifluormethyl,
- A: steht ganz besonders bevorzugt für Wasserstoff, Methyl, Ethyl, n-Propyl, iso-Propyl, n-Butyl, iso-Butyl, tert-Butyl, Methoxymethyl, Ethoxymethyl, Phenyl oder Cyclohexyl,
- B: steht ganz besonders bevorzugt für Wasserstoff, Methyl oder Ethyl,
- A, B: und das Kohlenstoffatom, an das sie gebunden sind, stehen ganz besonders bevorzugt für gesättigtes C₅-C₆-Cycloalkyl, in welchem gegebenenfalls ein Ringglied durch Sauerstoff ersetzt ist und welches gegebenenfalls einfach durch Methyl, Ethyl, n-Propyl, Isopropyl, n-Butyl, Isobutyl, tert.-Butyl, Trifluormethyl, Methoxy, Ethoxy, n-Propoxy, n-Butoxy oder Isobutoxy substituiert ist,
mit der Maßgabe, dass dann Q¹ nur für Wasserstoff steht,
- A und Q: ¹ stehen gemeinsam ganz besonders bevorzugt für C₃-C₄-Alkandiyl,
- Q¹: steht ganz besonders bevorzugt für Wasserstoff, Methyl, Ethyl, n-Propyl, iso-Propyl, Cyclopropyl, Cyclopentyl, Cyclohexyl oder 4-Chlorphenyl,
- Q², Q³, Q⁴: stehen ganz besonders bevorzugt unabhängig voneinander für Wasserstoff, Methyl oder Ethyl,
- Q¹ und Q²: stehen ganz besonders bevorzugt gemeinsam mit dem Kohlenstoffatom, an das sie gebunden sind, für gegebenenfalls durch Methyl, Ethyl, Methoxy, Ethoxy, n-Propoxy oder n-Butoxy substituiertes gesättigtes C₅-C₆₋Cycloalkyl, in welchem gegebenenfalls ein Ringglied durch Sauerstoff ersetzt ist,
mit der Maßgabe, dass dann A nur für Wasserstoff steht,
- G: steht ganz besonders bevorzugt für Wasserstoff (a) oder für eine der Gruppen 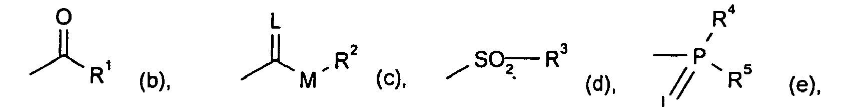 E (f) oder 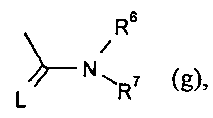 insbesondere für (a), (b) oder (c), in welchen
E für ein Metallion oder ein Ammoniumion steht,
L für Sauerstoff oder Schwefel steht und
M für Sauerstoff oder Schwefel steht,
- R¹: steht ganz besonders bevorzugt für jeweils gegebenenfalls durch Fluor oder Chlor substituiertes C₁-C₈-Alkyl, C₂-C₈-Alkenyl, C₁-C₂-Alkoxy-C₁-C₂₋alkyl, C₁-C₂-Alkylthio-C₁-C₂-alkyl oder jeweils gegebenenfalls einfach durch Fluor, Chlor, Methyl, Ethyl oder Methoxy substituiertes Cyclopropyl oder Cyclohexyl,
für gegebenenfalls einfach durch Fluor, Chlor, Brom, Cyano, Nitro, Methyl, Ethyl, iso-Propyl, tert-Butyl, Methoxy, Trifluormethyl oder Trifluormethoxy substituiertes Phenyl,
für jeweils gegebenenfalls einfach durch Fluor, Chlor, Brom oder Methyl substituiertes Thienyl oder Pyridyl,
- R²: steht ganz besonders bevorzugt für C₁-C₈-Alkyl, C₂-C₈-Alkenyl oder C₁₋C₄-Alkoxy-C₂-alkyl,
für gegebenenfalls einfach durch Methyl, Ethyl oder Methoxy substituiertes C₃-C₆-Cycloalkyl,
für jeweils gegebenenfalls einfach durch Fluor, Chlor, Cyano, Nitro, Methyl, Ethyl, iso-Propyl, tert-Butyl, Methoxy, Trifluormethyl oder Trifluormethoxy substituiertes Phenyl oder Benzyl,
- R³: steht ganz besonders bevorzugt für jeweils gegebenenfalls dreifach durch Fluor substituiertes Methyl oder Ethyl oder gegebenenfalls einfach durch Fluor, Chlor, Brom, Methyl, tert-Butyl, Methoxy, Trifluormethyl, Trifluormethoxy, Cyano oder Nitro substituiertes Phenyl,
- R⁴: steht ganz besonders bevorzugt für C₁-C₄-Alkyl, C₁-C₄-Alkoxy, C₁-C₄₋Alkylamino, Di-(C₁-C₄-alkyl)amino, C₁-C₄-Alkylthio oder für jeweils gegebenenfalls einfach durch Fluor, Chlor, Brom, Nitro, Cyano, C₁-C₂-Alkoxy, Trifluormethoxy oder C₁-C₃-Alkyl substituiertes Phenyl, Phenoxy oder Phenylthio,
- R⁵: steht ganz besonders bevorzugt für Methyl, Ethyl, Methoxy, Ethoxy, Methylthio oder Ethylthio,
- R⁶: steht ganz besonders bevorzugt für Wasserstoff, für C₁-C₄-Alkyl, C₃-C₆₋Cycloalkyl, C₁-C₄-Alkoxy, C₃-C₄-Alkenyl oder C₁-C₄-Alkoxy-C₁-C₄₋alkyl,
- R⁷: steht ganz besonders bevorzugt für Wasserstoff, C₁-C₄-Alkyl oder C₃-C₄₋Alkenyl,
- R⁶ und R⁷: stehen ganz besonders bevorzugt zusammen mit dem Stickstoffatom, an das sie gebunden sind, für einen C₅-Alkylenrest, in welchem gegebenenfalls eine Methylengruppe durch Sauerstoff ersetzt ist.
- Het: steht hervorgehoben für

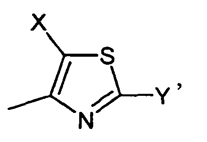

- m: steht hervorgehoben für die Zahlen 0 oder 1,
- X: steht hervorgehoben für Wasserstoff, Methyl oder Ethyl,
- Y: steht hervorgehoben für die Gruppe 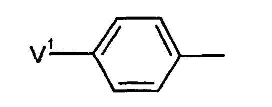
- V¹: steht hervorgehoben für Wasserstoff, Chlor, Methyl, Ethyl, n-Propyl, iso-Propyl, n-Butyl, iso-Butyl, tert.-Butyl, Trifluormethyl oder für 4-Chlorphenoxy,
- A: steht hervorgehoben für Wasserstoff, Methyl, Ethyl, n-Propyl, iso-Propyl, n-Butyl, iso-Butyl, Phenyl oder Cyclohexyl,
- B: steht hervorgehoben für Wasserstoff, Methyl oder Ethyl,
- A, B: und das Kohlenstoffatom, an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methyl, Ethyl, n-Propyl, Isopropyl, n-Butyl, Isobutyl, tert.-Butyl, Methoxy, Ethoxy, n-Propoxy, n-Butoxy oder Isobutoxy substituiert ist,
mit der Maßgabe, dass dann Q¹ nur für Wasserstoff steht,
- A und Q¹: stehen gemeinsam hervorgehoben für C₃-C₄-Alkandiyl,
- Q¹: steht hervorgehoben für Wasserstoff, Methyl, Ethyl, n-Propyl, iso-Propyl oder 4-Chlorphenyl,
- Q²: steht hervorgehoben für Wasserstoff, Methyl oder Ethyl,
- Q³: steht hervorgehoben für Wasserstoff,
- Q⁴: steht hervorgehoben für Wasserstoff,
- Q¹ und Q²: stehen hervorgehoben gemeinsam mit dem Kohlenstoffatom, an das sie gebunden sind, für gesättigtes C₅-C₆-Cycloalkyl,
mit der Maßgabe, dass dann A nur für Wasserstoff steht,
- G: steht hervorgehoben für Wasserstoff (a) oder für eine der Gruppen 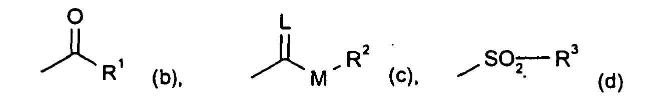 oder 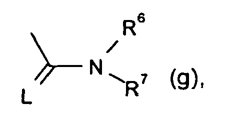 in welchen
L für Sauerstoff steht und
M für Sauerstoff oder Schwefel steht,
- R¹: steht hervorgehoben für C₁-C₈-Alkyl, C₁-C₂-Alkoxy-C₁-C₂-alkyl oder gegebenenfalls einfach durch Chlor substituiertes Cyclopropyl,
für gegebenenfalls einfach durch Chlor substituiertes Phenyl,
für gegebenenfalls einfach durch Chlor substituiertes Pyridyl,
- R²: steht hervorgehoben für C₁-C₈-Alkyl,
für Phenyl oder Benzyl,
- R³: steht hervorgehoben für Methyl oder Ethyl,
- R⁶ und R⁷: stehen hervorgehoben zusammen mit dem Stickstoffatom, an das sie gebunden sind, für einen C₅-Alkylenrest, in welchem gegebenenfalls eine Methylengruppe durch Sauerstoff ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Insbesonders bevorzugt sind Verbindungen der Formel (I), in denen G für Wasserstoff steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Verwendet man beispielsweise gemäß Verfahren (A) 1-[4-(5-Methyl)-2-(4-chlorphenyl)-thiazolyl]3-(1-ethoxycarbonyl-cyclohexyl)-propanon als Ausgangsstoff, so kann der Verlauf des erfindungsgemäßen Verfahrens durch folgendes Reaktionsschema wiedergegeben werden:

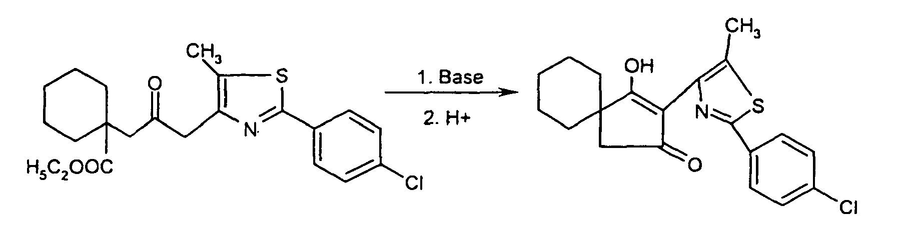

Verwendet man beispielsweise gemäß Verfahren (B) 1-[4-(5-Methyl)-2-(4-chlorphenyl)-thiazolyl]-4-(1-ethoxycarbonyl-cyclohexyl)-butan-2-on als Ausgangsstoff, so kann der Verlauf des erfindungsgemäßen Verfahrens durch folgendes Reaktionsschema wiedergegeben werden:

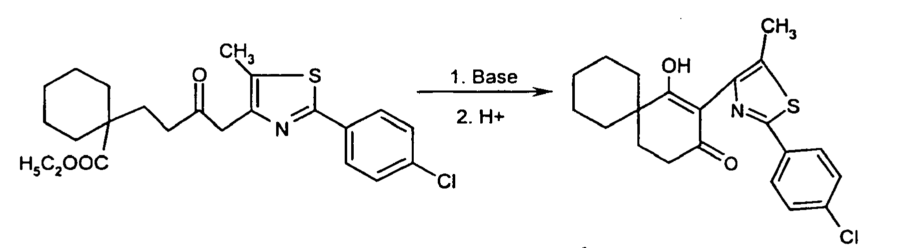

Verwendet man beispielsweise gemäß Verfahren (Cα) 2-[4-(5-Methyl-2-(3-chlorphenyl)-thiazolyl]-5,5-dimethylcyclohexan-1,3-dion und Pivaloylchlorid als Ausgangsstoffe, so kann der Verlauf des erfindungsgemäßen Verfahrens durch folgendes Reaktionsschema wiedergegeben werden:

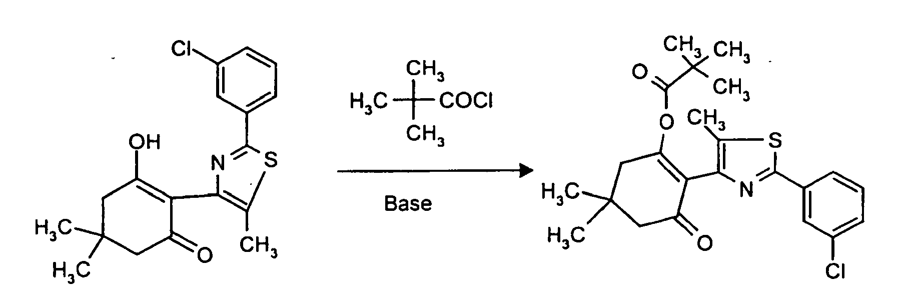

Verwendet man beispielsweise gemäß Verfahren (Cβ) 2-[4-(5-Ethyl-2-(4-methoxyphenyl))-thiazolyl]-5,5-dimethyl-cyclohexan-1,3-dion und Acetanhydrid als Ausgangsverbindungen, so kann der Verlauf des erfindungsgemäßen Verfahrens durch folgendes Reaktionsschema wiedergegeben werden:

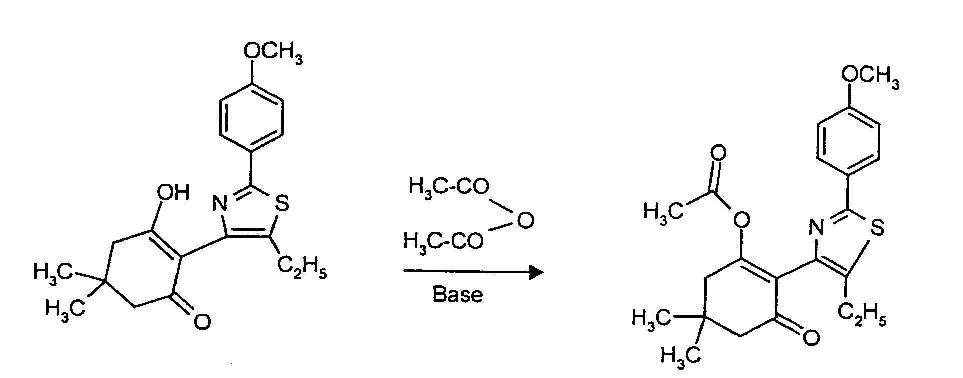

Verwendet man beispielsweise gemäß Verfahren (D) 2-[4-(5-Methyl-2-phenyl)-thiazolyl]-5,5-dimethyl-cyclohexan-1,3-dion und Chlorameisensäureethoxyethylester als Ausgangsverbindungen, so kann der Verlauf des erfindungsgemäßen Verfahrens durch folgendes Reaktionsschema wiedergegeben werden:

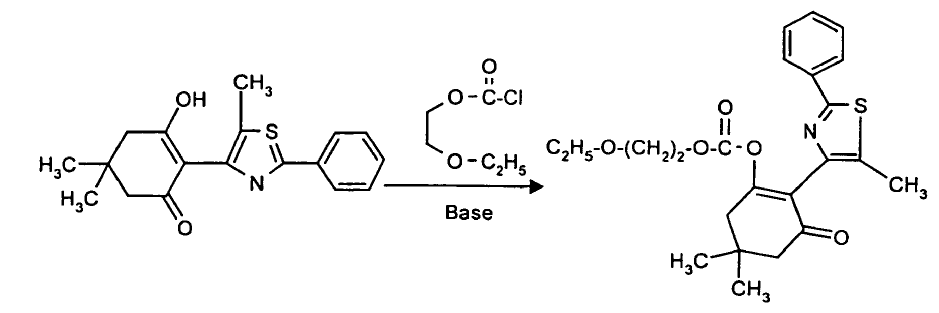

Verwendet man beispielsweise gemäß Verfahren (E), 2-[4-(5-Methyl-2-(4-fluorphenyl))-thiazolyl]-4,4-dimethyl-cyclopentan-1,3-dion und Chlormonothioameisensäuremethylester als Ausgangsprodukte, so kann der Reaktionsverlauf folgendermaßen wiedergegeben werden:

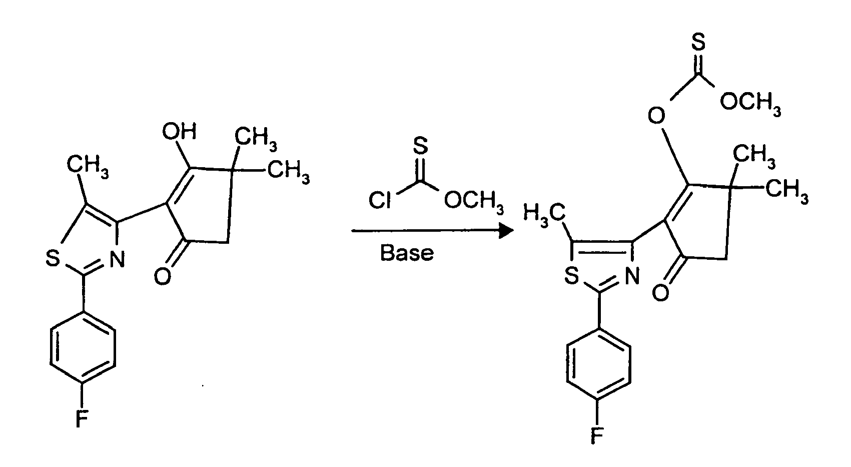

Verwendet man beispielsweise gemäß Verfahren (F) 2-[4-(5-Methyl-3-(4-methylphenyl)-thiazolyl]-5,5-dimethyl-cyclohexan-1,3-dion und Methansulfonsäurechlorid als Ausgangsprodukte, so kann der Reaktionsverlauf durch folgendes Reaktionsschema wiedergegeben werden:

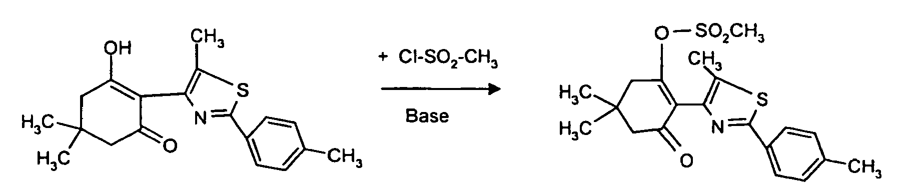

Verwendet man beispielsweise gemäß Verfahren (G) 2-[4-(5-Methyl-2-phenyl)-thiazolyl]-4,4-dimethyl-cyclopentan-1,3-dion und Methanthio-phosphonsäurechlorid-(2,2,2-trifluorethylester) als Ausgangsprodukte, so kann der Reaktionsverlauf durch folgendes Reaktionsschema wiedergegeben werden:

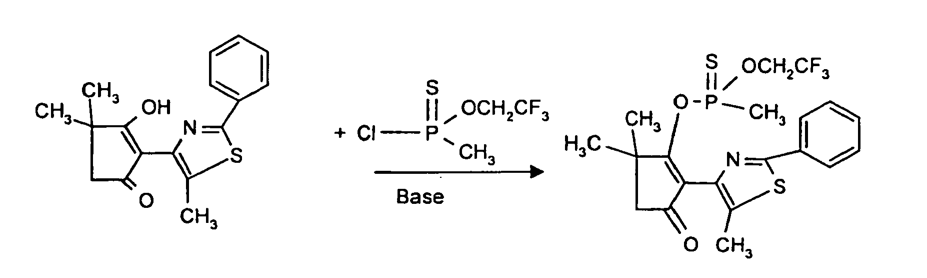

Verwendet man beispielsweise gemäß Verfahren (H) 2-[4-(5-Methyl-2-(4-trifluormethyl-phenyl))-thiazolyl]-5,5-dimethyl-cyclohexan-1,3-dion und NaOH als Komponenten, so kann der Verlauf des erfindungsgemäßen Verfahrens durch folgendes Reaktionsschema wiedergegeben werden:

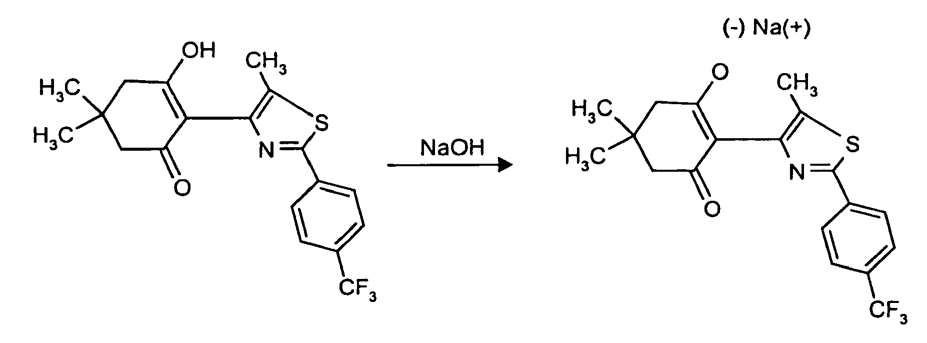

Verwendet man beispielsweise gemäß Verfahren (Iα) 2-[4-(5-Methyl-2-(3-trifluormethyl-phenyl))-thiazolyl]-4,4-dimethyl-cyclohexan-1,3-dion und Ethylisocyanat als Ausgangsprodukte, so kann der Reaktionsverlauf durch folgendes Reaktionsschema wiedergegeben werden:

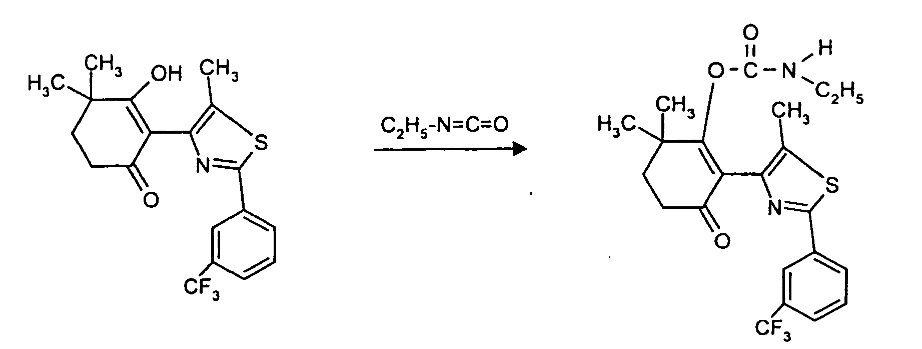

Verwendet man beispielsweise gemäß Verfahren (Iβ) 2-[4-(5-Methyl-2-phenyl)-thiazolyl]-5,5-dimethyl-cyclohexyl-1,3-dion und Dimethylcarbamidsäurechlorid als Ausgangsprodukte, so kann der Reaktionsverlauf durch folgendes Schema wiedergegeben werden:

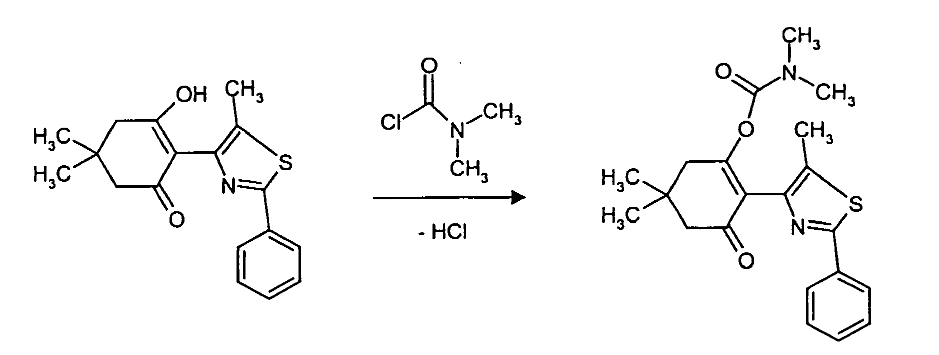

Die bei dem obigen Verfahren (A) als Ausgangsstoffe benötigten Ketocarbonsäureester der Formel (II) 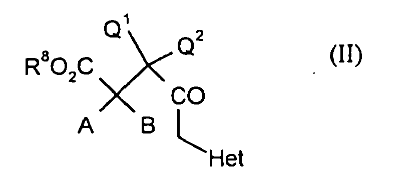 in welcher
- A, B, Het, Q¹, Q² und R⁸: die oben angegebene Bedeutung haben,
sind neu.

Sie lassen sich nach im Prinzip bekannten Methoden herstellen.

Man erhält die 5-Hetaryl-4-ketocarbonsäureester der Formel (II) beispielsweise, wenn man 5-Hetaryl-4-ketocarbonsäuren der Formel (XIV) 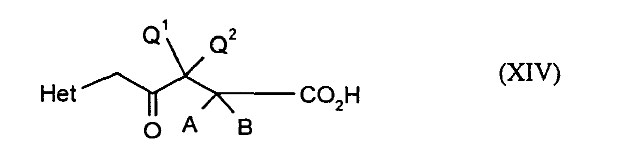 in welcher
A, B, Het, Q¹ und Q² die oben angegebene Bedeutung haben,
verestert (vgl. z.B. Organikum, 15. Auflage, Berlin, 1977, Seite 499) oder alkyliert (siehe Herstellungsbeispiel).

Die 5-Hetaryl-4-ketocarbonsäuren der Formel (XIV) 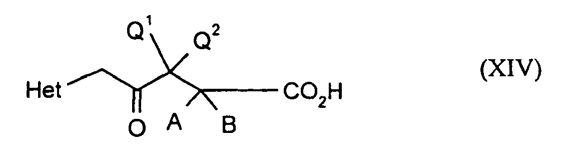 in welcher
A, B, Het, Q¹ und Q² die oben angegebene Bedeutung haben,
sind neu, lassen sich aber nach im Prinzip bekannten Methoden herstellen (siehe Herstellungsbeispiel).

Man erhält die 5-Hetaryl-4-ketocarbonsäuren der Formel (XIV) beispielsweise, wenn man 2-Hetaryl-3-oxo-adipinsäureester der Formel (XV) 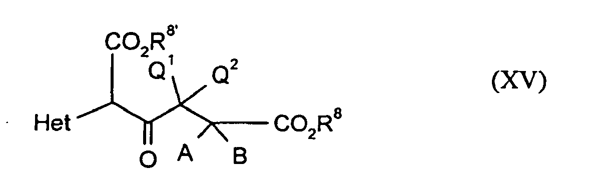 in welcher
- A, B, Het, Q¹ und Q²: die oben angegebene Bedeutung haben und
- R⁸ und R^{8'}: für Alkyl (insbesondere C₁-C₈-Alkyl) stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XV) 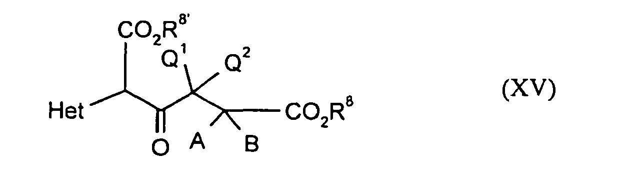 in welcher
- A, B, Het, Q¹, Q², R⁸ und R^{8'}: die oben angegebene Bedeutung haben,
oder wenn die Verbindung der Formel (XVII) als Ausgangsstoff zur Herstellung verwendet wurde,
und R⁸ dann für Wasserstoff steht, sind neu.

Man erhält die Verbindungen der Formel (XV) beispielsweise,
wenn man Dicarbonsäurehalbesterchloride der Formel (XVI), 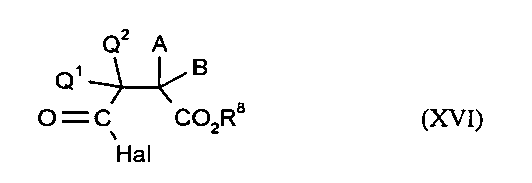 in welcher
- A, B, Q¹, Q² und R⁸: die oben angegebene Bedeutung haben und
- Hal: für Chlor oder Brom steht,
oder Carbonsäureanhydride der Formel (XVII) 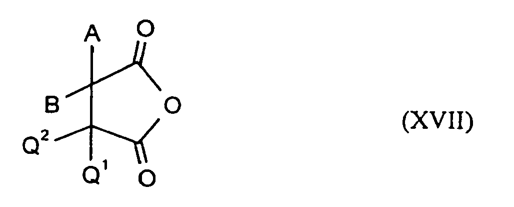 in welcher
A, B, Q¹ und Q² die oben angegebene Bedeutung haben,
mit einem Hetarylessigsäureester der Formel (XVIII) 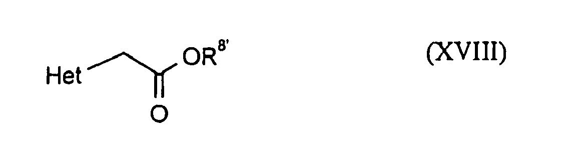 in welcher
- Het und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XVI) und (XVII) sind teilweise bekannte Verbindungen der Organischen Chemie und/oder lassen sich nach im Prinzip bekannten Methoden in einfacher Weise herstellen.

Die bei dem obigen Verfahren (B) als Ausgangsstoffe benötigten Ketocarbonsäureester der Formel (III) 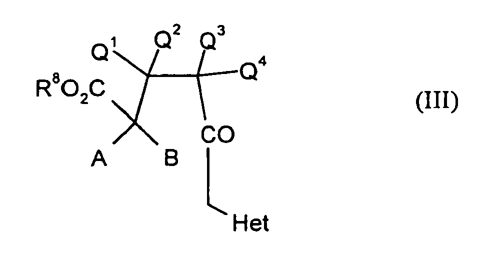 in welcher
- A, B, Het, Q¹, Q², Q³, Q⁴ und R⁸: die oben angegebene Bedeutung haben,
sind neu.

Sie lassen sich nach im Prinzip bekannten Methoden herstellen.

Man erhält die 6-Hetaryl-5-ketocarbonsäureester der Formel (III) beispielsweise, wenn man 6-Hetaryl-5-ketocarbonsäuren der Formel (XIX) 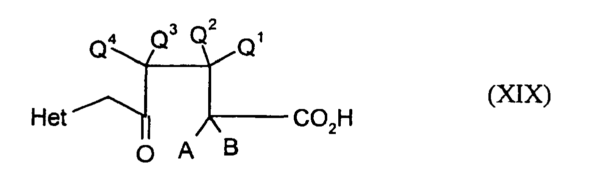 in welcher
A, B, Het, Q¹, Q², Q³ und Q⁴ die oben angegebene Bedeutung haben,
verestert (vgl. z.B. Organikum, 15. Auflage, Berlin, 1977, Seite 499) oder alkyliert (s. Herstellungsbeispiel).

Die 6-Hetaryl-5-ketocarbonsäuren der Formel (XIX) 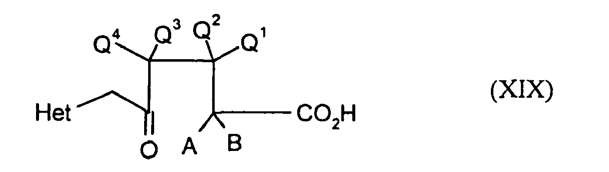 in welcher
A, B, Het, Q¹, Q², Q³ und Q⁴ die oben angegebene Bedeutung haben,
sind neu. Sie lassen sich nach im Prinzip bekannten Methoden herstellen, beispielsweise wenn man
substituierte 2-Hetaryl-3-oxo-heptandisäureester der Formel (XX) 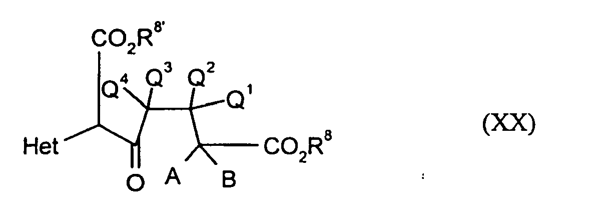 in welcher
- A, B, Het, Q¹, Q², Q³ und Q⁴: die oben angegebene Bedeutung haben und
- R⁸ und R^{8'}: für Alkyl (bevorzugt C₁-C₆-Alkyl), stehen,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521).

Die Verbindungen der Formel (XX) 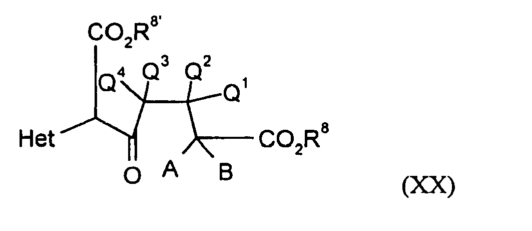 in welcher
A, B, Het, Q¹*,* Q², Q³, Q⁴, R⁸ und R^{8'} die oben angegebene Bedeutung haben,
oder wenn die Verbindung der Formel (XXII) als Ausgangsstoff zur Herstellung verwendet wurde,
und R⁸ dann für Wasserstoff steht, sind neu.

Man erhält die Verbindungen der Formel (XX) beispielsweise,
wenn man Dicarbonsäureester der Formel (XXI), oder Dicarbonsäureanhydride der Formel (XXII) 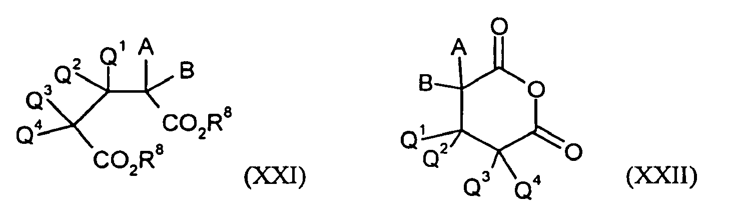 in welcher
A, B, Q¹, Q², Q³, Q⁴ und R⁸ die oben angegebene Bedeutung haben,
mit einem substituierten Hetarylessigsäureester der Formel (XVIII) 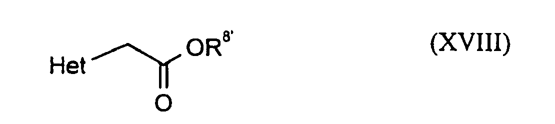 in welcher
- Het und R⁸': die oben angegebene Bedeutung haben,
in Gegenwart eines Verdünnungsmittels und in Gegenwart einer Base kondensiert.

Die Verbindungen der Formel (XXI) und (XXII) sind teilweise käuflich, teilweise bekannt und/oder lassen sich nach bekannten Verfahren darstellen.

Die Verbindungen der Formel (XVIII) sind teilweise neu, lassen sich aber nach im Prinzip bekannten Verfahren darstellen:
1. C.S. Rooney et al. *J. Med. Chem.* **1983**, *26,* 700-714.
2. M.S. Malamas et al. *J. Med. Chem.* **1996**, 39, 237-245.
3. J.L. Collins et al. *J. Med. Chem.* **1998**, *41*, 5037-5054.
4. EP-A-0 177 353.
5. EP-A-0 368 592.
6. NL-A-6614130.

Die zur Durchführung der erfindungsgemäßen Verfahren (C), (D), (E), (F), (G), (H), und (I) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride oder Thiocarbamidsäurechloride der Formel (XIII) sind allgemein bekannte Verbindungen der Organischen bzw. Anorganischen Chemie.

Die Verbindungen der Formeln (XVI), (XVII) und (XXI) sind teilweise käuflich, teilweise bekannt und/oder lassen sich nach im Prinzip bekannten Methoden herstellen.

Das Verfahren (A) ist dadurch gekennzeichnet, dass man Verbindungen der Formel (II), in welcher A, B, Q¹, Q², Het und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, daß Verbindungen der Formel (III), in welcher A, B, Q¹, Q², Q³, Q⁴, Het und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C-α) ist dadurch gekennzeichnet, dass man Verbindungen der Formeln (I-1-a) bis (1-2-a) jeweils mit Carbonsäurehalogeniden der Formel (IV) gegebebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (C-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (C-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-α) werden die Ausgangsstoffe der Formeln (I-1-a) bis (I-2-a) und das Carbonsäurehalogenid der Formel (IV) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C-β) ist dadurch gekennzeichnet, dass man Verbindungen der Formeln (I-1-a) bis (I-2-a) mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (C-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-β) werden die Ausgangsstoffe der Formeln (I-1-a) bis (1-2-a) und das Carbonsäureanhydrid der Formel (V) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formeln (I-1-a) bis (1-2-a) jeweils mit Chlorameisensäureestern oder Chlorameisensäurethiolestem der Formel (VI) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan.

Die Reaktionstemperaturen können bei der Durchführung des erfindungsgemäßen Verfahrens (D) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formeln (I-1-a) bis (1-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (1-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Nitrile, Ester, Ether, Amide, Sulfone, Sulfoxide, aber auch Halogenalkane.

Vorzugsweise werden Acetonitril, Essigsäureethylester, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formeln (I-1-a) bis (1-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a bis I-2-a) ca. 1 Mol Sulfonsäurechlorid der Formel (VIII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe wie Methylenchlorid.

Vorzugsweise werden Acetonitril, Essigsäureethylester, Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (1-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-a) bis (I-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formeln (I-1-e) bis (I-2-e) auf 1 Mol der Verbindungen (I-1-a) bis (1-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (H) ist dadurch gekennzeichnet, dass man Verbindungen der Formeln (I-1-a) bis (1-2-a) mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (H) wird im allgemeinen unter Normaldruck durchgeführt.

Die Reaktionstemperaturen liegen im allgemeinen zwischen -20°C und 100°C, vorzugsweise zwischen 0°C und 50°C.

Das erfindungsgemäße Verfahren (I) ist dadurch gekennzeichnet, dass man Verbindungen der Formeln (I-1-a) bis (I-2-a) jeweils mit Verbindungen der Formel (XII) (I-α) gegebenenfalls in Gegenwart eines Verdünnungsmittels und gegebenenfalls in Gegenwart eines Katalysators oder (I-β) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (I-α) setzt man pro Mol Ausgangsverbindung der Formeln (I-1-a) bis (I-2-a) ca. 1 Mol Isocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (I-β) setzt man pro Mol Ausgangsverbindung der Formeln (I-1-a) bis (1-2-a) ca. 1 Mol Carbamidsäurechlorid der Formel (XIII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe.

Vorzugsweise werden Acetonitril, Essigsäureethylester, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-2-a) dar, kann auf den weiteren Zusatz von Säurebindemitteln verzichtet werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindunsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlonnethyl)-1,3,4-thiadiazol,
   2-([6-Deoxy-4-O-(4-O-methyl-β-D-glycopyranosyl)-a-D-glucopyranosyl]-amino]-4-methoxy-1 H-pyrrolo[2,3-d]pyrimidin-5-carbonitril,
   2-Aminobutan,
   2-Brom-2-(brommethyl)-pentandinitril,
   2-Chlor-N-(2,3-dihydro-1, 1,3 -trimethyl- 1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1 H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1 H-imidazol-1-sulfonamid,
   4-Methyl-tetrazolo[1,5-a]quinazolin-5(4H)-on,
   8-(1, 1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamine)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, BromophosA, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]- 1 -fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Emteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylhamstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").
Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
Sirex juvencus, Urocerus gigas, Urocerus gigas taignus, Urocerus augur.
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-FungizidGemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb; oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind.* **1985**, *37*, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden.

Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Omithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
   Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

### Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulf uron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, lodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazit, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise: AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
   (Konidienform: Drechslera, Syn: Helminthosporium);
Cochliobolus-Arten, wie beispielsweise Cochliobolus sativus
   (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokolation mit unerwünschten Mikroorgansimen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Emteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschrniermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäure-ester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blästicidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
   OK-8705,
   OK-8801,
α-(1,1-Dimethylethyl)-β-(2-phenoxyethyl)-1 H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1 H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
{2-Methyl-1-[[[1-(4-methylphenyl)-ethyl]-amino]-carbonyl]-propyl }-carbaminsäure-1-isopropylester
1-(2,4-Dichlorphenyl)-2-(1 H-1,2,4-triazol-1-yl)-ethanon-O-(phenylmethyl)-oxim,
   1-(2-Methyl-1-naphthalenyl)-1 H-pyrrol-2,5-dion,
1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1 H-imidazol,
   1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1 H-inden-2-carbonitril,
   3-[2-(4-Chlorphenyl)-5-ethoxy-3-isoxazolidinyl]-pyridin,
4-Chlor-2-cyan-N,N-dimethyl-5-(4-methylphenyl)-1 H-imidazol-1-sulfonamid,
   4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
   8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
O,O-Diethyl-[2-(dipropylamino)-2-oxoethyl]-ethylphosphoramidothioat,
   O-Methyl-S-phenyl-phenylpropylphosphoramidothioate,
S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos, Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1 -phenylethoxy)-phenyl]-amino]-carbonyl]-benzamid
   2-Chlor-N-[[[4-(2,2-dichlor-1,1-difluorethoxy)-phenyl]-aminol]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon
   Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1 H-pyrazol-1-carboxamid
   N-[(2-Chlor-5-thiazolyl)methyl]-N'-methyl-N"-nitro-guanidin
   N-Methyl-N'-(1-methyl-2-propenyl)- 1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden.

Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Herstellungsbeispiele

### Beispiel I-1-a-1

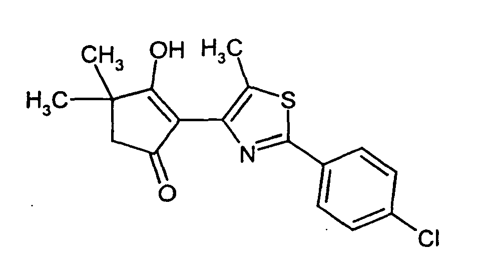

1,65 g (14,4 mmol) Kalium-tert-butylat werden in 30 ml wasserfreien Dimethylformamid (DMF) vorgelegt und 3,5 g (9,6 mmol) der Verbindung gemäß Beispiel (II-1) in 10 ml wasserfreiem DMF zugetropft. Man rührt 3 h bei 50°C. Anschließend wird in die Reaktionslösung 100 ml Eiswasser gegeben. Dieses Gemisch wird in 600 ml kalte 1 N HCl-Lösung gegeben. Der Niederschlag wird abgesaugt und getrocknet.
Ausbeute: 2,9 g (90 % d. Th.), Fp. 139-141°C

In Analogie zu Beispiel (I-1-a-1) und gemäß den allgemeinen Angaben zur Herstellung wurden folgende Verbindungen der Formel (I-1-a) hergestellt:

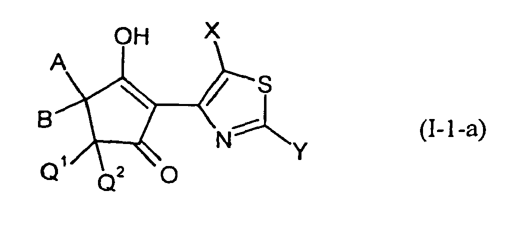

| **Bsp. Nr.** | **X** | **Y** | **B** | **A** | **Q**^{**1**} | **Q**^{**2**} | **Fp.°C** |
|---|---|---|---|---|---|---|---|
| I-1-a-2 | CH₃ | 4-Cl-C₆H₄ | -(CH₂)₅- | | H | H | 105-107°C |
| I-1-a-3 | C₂H₅ | 4-Cl-C₆H₄ | -(CH₂)₅- | | H | H | 121-123°C |
| I-1-a-4 | CH₃ | 4-Cl-C₆H₄ | -(CH₂)₂-O-(CH₂)₂- | | H | H | 130 |
| I-1-a-5 | CH₃ | 4-CF₃-C₆H₄ | -(CH₂)₅- | | H | H | Öl |
| I-1-a-6 | CH₃ | 4-Cl-C₆H₄ | H | -(CH2)₄ | | H | 145-148 |
| I-1-a-7 | CH₃ | 4-CH₃-C₆H₄ | -(CH₂)₅- | | H | H | 85 |
| I-1-a-8 | CH₃ | 4-Cl-C₆H₄-O-C₆H₄ | -(CH₂)₅- | | H | H | 75-77 |
| I-1-a-9 | CH₃ | 4-Cl-C₆H₄ | -(CH₂)₂-CHCH₃-(CH₂)₂- | | H | H | 142-144 |
| I-1-a-10 | CH₃ | 4-Cl-C₆H₄ | -(CH₂)₂-CHOCH₃-(CH₂)₂- | | H | H | 127 |
| I-1-a-11 | CH₃ | 4-Cl-C₆H₄ | H | C₆H₅- | H | H | 130 |
| I-1-a-12 | C₂H₅ | 4-Cl-C₆H₄ | -(CH₂)₂-O-(CH₂)₂- | | H | H | 210 |
| I-1-a-13 | CH₃ | 4-Cl-C₆H₄ | H | C₆H₁₁- | H | H | 105 |
| I-1-a-14 | CH₃ | 4-Cl-C₆H₄ | -(CH₂)₂-CH-C₃H₇-(CH₂)₂- | | H | H | Öl |
| I-1-a-15 | CH₃ | 4-Cl-C₆H₄ | -(CH₂)₂-CH-t-C₄H₉-(CH₂)₂- | | H | H | Öl |
| I-1-a-16 | CH₃ | 4-Cl-C₆H₄ | -(CH₂)₂-CH-C₂H₅-(CH₂)₂- | | H | H | Öl |
| I-1-a-17 | H | 4-Cl-C₆H₄ | -(CH₂)₅- | | H | H | 185 |

### Beispiel I-1-b-1

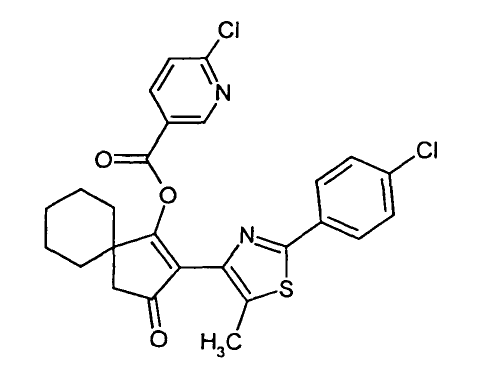

500 mg (1,33 mmol) der Verbindung gemäß Beispiel I-1-a-2 werden in 5 ml absolutem Dichlormethan vorgelegt und mit 0,27 ml (2,0 mmol) Triethylamin versetzt. Bei 0°C werden 0,30 g (1,74 mmol) 6-Chlornicotinylchlorid zugegeben und 2 h bei Raumtemperatur gerührt. Anschließend gibt man weitere 0,15 g Chlornicotinylchlorid und 0,13 ml Triethylamin zu.

Das Reaktionsgemisch wird mit 10 %iger Zitronensäurelösung gewaschen, die wässrige Phase wird mit Dichlormethan extrahiert und anschließend wird die organische Phase mit 1 N Natriumhydroxidlösung gewaschen und die wässrige Phase mit Dichlormethan extrahiert.

Die organische Phase wird getrocknet und das Lösungmittel abdestilliert. Der Rückstand wird mit Petrolether verrührt und filtriert und anschließend getrocknet.
Ausbeute: 0,7 g (100 % d. Th.), Fp. 133-134°C

In Analogie zu Beispiel (I-1-b-1) und gemäß den allgemeinen Angaben zur Herstellung wurden folgende Verbindungen der Formel (I-1-b) erhalten:

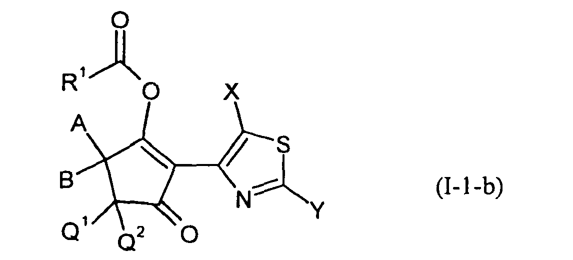

| **Bsp. Nr.** | **X** | **Y** | **B A** | | **Q**^{**1**} | **Q**^{**2**} | **R**^{**1**} | **Fp.°C** |
|---|---|---|---|---|---|---|---|---|
| I-1-b-2 | CH₃ | 4-Cl-C₆H₄ | -(CH₂)₅- | | H | H | H₅C₂-O-CH₂- | 135 |
| I-1-b-3 | CH₃ | 4-Cl-C₆H₄ | -(CH₂)₅- | | H | H | i-C₃H₇ | Öl |
| I-1-b-4 | CH₃ | 4-Cl-C₆H₄ | -(CH₂)₅- | | H | H | 4-Cl-C₆H₄ | 129-130 |
| I-1-b-5 | CH₃ | 4-Cl-C₆H₄ | -(CH₂)₅- | | H | H | 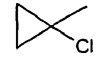 | Öl |

### Beispiel I-1-c-1

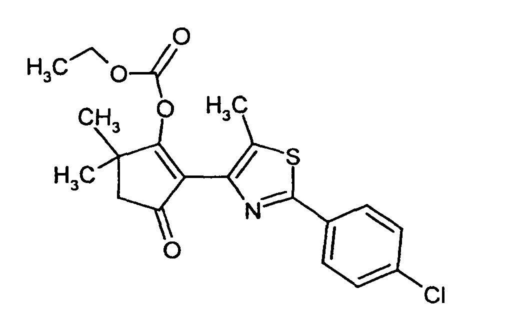

500 mg (1,5 mmol) der Verbindung gemäß Beispiel (I-1-a-1) werden in 5 ml absolutem Dichlormethan vorgelegt und mit 0,3 ml (2,25 mmol) Triethylamin versetzt. Bei 0°C werden 0,225 g (1,95 mmol) Chlorameisensäureethylester zugegeben und 2 h bei Raumtemperatur gerührt.

Die Reaktionslösung wird mit 10 %iger Zitronensäure gewaschen und die wässrige Phase mit Dichlormethan (CH₂Cl₂) extrahiert. Die organische Phase wird mit 1 N Natriumhydroxidlösung gewaschen, getrocknet und eingeengt.
Ausbeute: 0,45g (75 % der Theorie), 132-134°C

In Analogie zu Beispiel (I-1-c-1) und gemäß den allgemeinen Angaben zur Herstellung wurden folgende Verbindungen der Formel (I-1-c) erhalten:

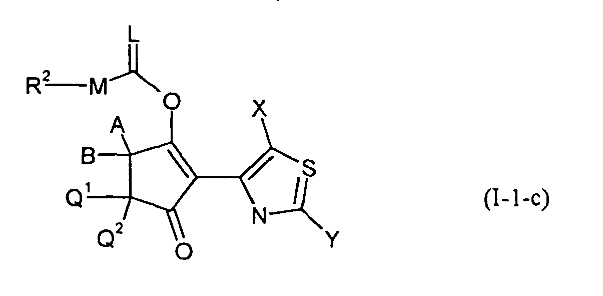

| **Bsp.-Nr** | **X** | **Y** | **A** | **B** | **Q**^{**1**} | **Q**^{**2**} | **L** | **M** | **R**^{**2**} | **Fp.°C** |
|---|---|---|---|---|---|---|---|---|---|---|
| I-1-c-2 | CH₃ | 4-Cl-C₆H₄ | -(CH₂)₅- | | H | H | O | O | C₂H₅ | Öl |
| I-1-c-3 | C₂H₅ | 4-Cl-C₆H₄ | -(CH₂)₅- | | H | H | O | O | C₂H₅ | Öl |
| I-1-c-4 | CH₃ | 4-Cl-C₆H₄ | -(CH₂)₅- | | H | H | O | O | C₆H₅ | 139-140 |
| I-1-c-5 | CH₃ | 4-Cl-C₆H₄ | -(CH₂)₅- | | H | H | O | S | C₆H₅-CH₂ | 144 |
| I-1-c-6 | CH₃ | 4-Cl-C₆H₄ | -(CH₂)₅- | | H | H | O | O | C₆H₅-CH₂ | 137 |

### Beispiel I-1-d-1

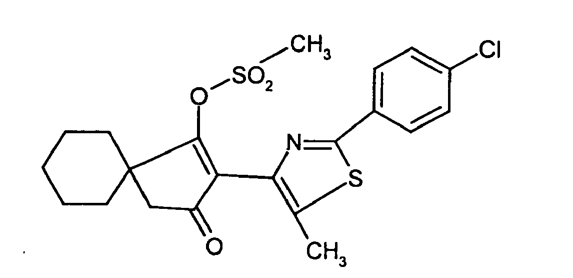

0,5 g (1,33 mmol) der Verbindung gemäß Beispiel I-1-a-2 werden in 5 ml wasserfreiem Dichlormethan vorgelegt und mit 0,27 ml (2,0 mmol) Triethylamin versetzt. Unter Eiskühlung werden 0,135 ml (1,74 mmol) Methansulfonsäurechlorid zugegeben. Man rührt 2 h bei Raumtemperatur.

Das Reaktionsgemisch wird mit 10 %iger Zitronensäure extrahiert, die wässrige Phase mit Dichlormethan gewaschen, die organische Phase mit 1 N Natriumhydroxid-Lösung extrahiert und die wässrige Phase mit Dichlormethan gewaschen, getrocknet und eingeengt.
Ausbeute: 0,6 g (100 % d. Th.), Fp. 134°C
¹H-NMR (d₆-DMSO, 400 MHz): δ = 1.02 - 1.75 (m, 10H, cyc-Hexyl-CH₂), 2,30 (s, 3H, Thiazolyl-CH₃), 3.05 (s, 2H, CH₂-CO), 3,61 (s, 3H, SO₂CH₃), 7,54 (d, 2H, Ar-H), 7,86 (d, 2H, Ar-H) ppm.

### Beispiel I-1-g-1

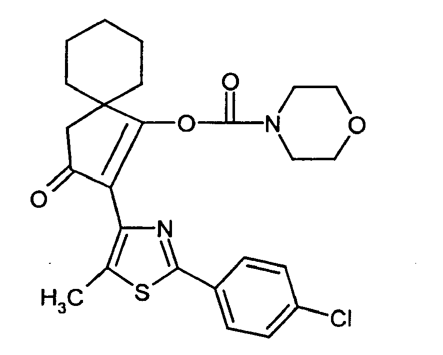

0,5 g (1,33 mmol) der Verbindung gemäß Beispiel I-1-a-2 und 0,27 ml (2,0 mmol) Triethylamin werden in 5 ml wasserfreiem Dichlormethan gelöst und bei Eiskühlung 0,26 g (1,73 mmol) Morpholin-N-carbonsäurechlorid zugegeben. Es wird über Nacht bei Raumtemperatur gerührt.

Das Reaktionsgemisch wird mit 10 %iger Zitronensäure extrahiert, die wässrige Phase mit Dichlormethan gewaschen, die organische Phase mit 1 N Natriumhydroxid-Lösung extrahiert und die wässrige Phase mit Dichlormethan gewaschen, getrocknet und eingeengt.
Ausbeute: 0,69 g (93 % d. Th.), Wachs
¹H-NMR (d₆-DMSO, 400 MHz): δ = 1.02 - 1.74 (m, 10H, cyc-Hexyl-CH₂), 2,30 (s, 3H, Thiazolyl-CH₃), 3.01 (s, 2H, CH₂-CO), 3,05 - 3,79 (m, 8H, Morpholin-CH₂), 7,54 (d, 2H, Ar-H), 7,85 (d, 2H, Ar-H) ppm.

### Beispiel II-1

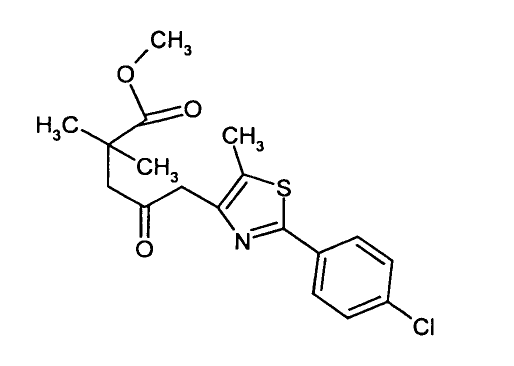

17,3 g (30 mmol) der Verbindung gemäß Beispiel XIV-1 werden in 200 ml wasserfreiem Aceton vorgelegt und 6,8 g (49 mmol) Kaliumcarbonat und 20,5 g (147 mmol) Methyliodid zugesetzt. Es wird 16 h unter Rückfluss gerührt.

Man filtriert die Lösung, destilliert das Lösungmittel ab und reinigt säulenchromatographisch über Kieselgel (Dichlormethan: Petrolether, 4:1 → Dichlormethan → Dichlormethan : Essigsäureethylester, 30 : 1 → 10 : 1).
Ausbeute: 3,5 g (31 % der Theorie), Öl
¹H-NMR (d₆-DMSO, 400 MHz):
δ = 1,11 (s, 6H, 2-CH₃-C-aliph.), 2,31 (s, 3H, CH₃-C-Heteroarom.) 3,50 (s, 3H, CH₃-O), 7,50 (d, 2H, 2-CH-arom.), 7,84 (d, 2H, 2-CH-arom) ppm.

### Beispiel XIV-1

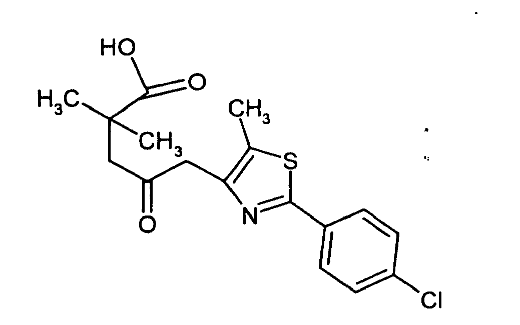

Zu einer Lösung von 25 ml LDA*-Lösung (2 molar) in 50 ml wasserfreiem Tetrahydrofuran wird eine Lösung von 12,7 g (45 mmol) 4-[2-(4-Chlorphenyl)-5-methyl]-thiazolyl-essigsäure-methylester in 15 ml Tetrahydrofuran bei -15°C zugetropft und 60 min bei 0°C gerührt.

Dann wird bei -15°C eine Lösung von 3,8 g (30 mmol) 2,2-Dimethylbemsteinsäureanhydrid in 10 ml wasserfreiem Tetrahydrofuran zugetropft.

Die Lösung wird zwei Stunden bei Raumtemperatur gerührt, dann werden 75 ml Wasser und 20 g Ammoniumchlorid zugegeben und mit konzentrierter Salzsäure eine saure Lösung eingestellt.

Das Zwischenprodukt wird mit Ether extrahiert und das Lösungsmittel abdestilliert. Der Rückstand wird mit 25 g KOH und 170 ml Wasser einen Tag unter Rückfluss gekocht.

Man kühlt ab, säuert mit konz. Salzsäure an und extrahiert mit Ether. Das Rohprodukt wird direkt ohne weitere Charakterisierung und Reinigung in die Verbindung II-1 überführt.
Ausbeute: 17,5 g (100 % der Theorie), Öl
*LDA = Lithium-diisopropylamid

In Analogie zu Beispiel (II-1) und gemäß den allgemeinen Angaben zur Herstellung wurden folgende Verbindungen der Formel (II) erhalten:

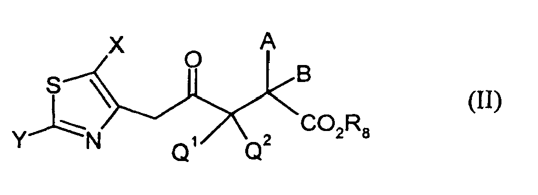

| **Bsp.- Nr** | **X** | **Y** | **B** | **A** | **Q**^{**1**} | **Q**^{**2**} | **R**^{**8**} | **Fp. °C** |
|---|---|---|---|---|---|---|---|---|
| II-2 | CH₃ | 4-Cl-C₆H₄ | -(CH₂)₅- | | H | H | CH₃ | Öl |
| II-3 | C₂H₅ | 4-Cl-C₆H₄ | -(CH₂)₅- | | H | H | CH₃ | Öl |
| II-4 | CH₃ | 4-Cl-C₆H₄ | -(CH₂)₂-O-(CH₂)₂- | | H | H | CH₃ | Öl |
| II-5 | CH₃ | 4-CF₃-C₆H₄ | -(CH₂)₅- | | H | H | CH₃ | Öl |
| II-6 | CH₃ | 4-Cl-C₆H₄ | H | -(CH₂)₄- | | H | CH₃ | Öl |
| II-7 | CH₃ | 4-CH₃-C₆H₄ | -(CH₂)₅- | | H | H | CH₃ | Öl |
| II-8 | CH₃ | 4-Cl-C₆H₄-O-C₆H₄. | -(CH₂)₅- | | H | H | CH₃ | Öl |
| II-9 | CH₃ | 4-Cl-C₆H₄ | -(CH₂)₂-CHCH₃-(CH₂)₂- | | H | H | CH₃ | Öl |
| II-10 | CH₃ | 4-Cl-C₆H₄ | -(CH₂)₂-CHOCH₃-(CH₂)₂- | | H | H | CH₃ | Öl |
| II-11 | CH₃ | 4-Cl-C₆H₄ | H | C₆H₅- | H | H | CH₃ | Öl |
| II-12 | C₂H₅ | 4-Cl-C₆H₄ | -(CH₂)₂-O-(CH₂)₂- | | H | H | CH₃ | Öl |
| II-13 | CH₃ | 4-Cl-C₆H₄ | H | C₆H₁₁ | H | H | CH₃ | Öl |
| II-14 | CH₃ | 4-Cl-C₆H₄ | -(CH₂)₂-CH-C₃H₇-(CH₂)₂- | | H | H | CH₃ | Öl |
| II-15 | CH₃ | 4-Cl-C₆H₄ | -(CH₂)₂-CH-t-C₄H₉-(CH₂)₂- | | H | H | CH₃ | Öl |
| II-16 | CH₃ | 4-Cl-C₆H₄ | -(CH₂)₂-CH-C₂H₅-(CH₂)₂- | | H | H | CH₃ | Öl |
| II-17 | H | 4-Cl-C₆H₄ | -(CH₂)₅- | | H | H | CH₃ | Öl |

In Analogie zu Beispiel (XIV-1) und gemäß den allgemeinen Angaben zur Herstellung wurden folgende Verbindungen der Formel (XIV) erhalten:

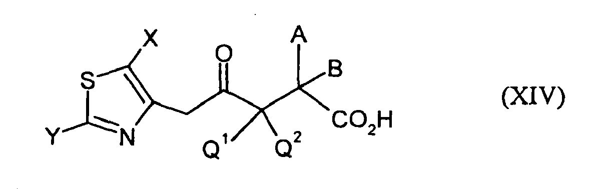

| **Bsp.-Nr.** | **X** | **Y** | **B** | **A** | **Q**^{**1**} | **Q**^{**2**} | **Fp. °C** |
|---|---|---|---|---|---|---|---|
| XIV-2 | CH₃ | 4-Cl-C₆H₄ | -(CH₂)₅- | | H | H | Wachs |
| XIV-3 | C₂H₅ | 4-Cl-C₆H₄ | -(CH₂)₅- | | H | H | Wachs |
| XIV-4 | CH₃ | 4-Cl-C₆H₄ | -(CH₂)₂-O-(CH₂)₂- | | H | H | Öl |
| XIV-5 | CH₃ | 4-CF₃-C₆H₄ | -(CH₂)₅- | | H | H | Öl |
| XIV-6 | CH₃ | 4-Cl-C₆H₄ | H | -(CH₂)₄- | | H | Öl |
| XIV-7 | CH₃ | 4-CH₃-C₆H₄ | -(CH₂)₅- | | H | H | Öl |
| XIV-8 | CH₃ | 4-Cl-C₆H₄-O-C₆H₄ | -(CH₂)₅- | | H | H | Öl |
| XIV-9 | CH₃ | 4-Cl-C₆H₄ | -(CH₂)₂-CHCH₃-(CH₂)₂- | | H | H | Öl |
| XIV-10 | CH₃ | 4-Cl-C₆H₄ | -(CH₂)₂-CHOCH₃-(CH₂)₂- | | H | H | Öl |
| XIV-11 | CH₃ | 4-Cl-C₆H₄ | H | C₆H₅- | H | H | Öl |
| XIV-12 | C₂H₅ | 4-Cl-C₆H₄ | -(CH₂)₂-O-(CH₂)₂- | | H | H | Öl |
| XIV-13 | CH₃ | 4-Cl-C₆H₄ | H | C₆H₁₁- | H | H | Öl |
| XIV-14 | CH₃ | 4-Cl-C₆H₄ | -(CH₂)₂-CH-C₃H₇-(CH₂)₂- | | H | H | Öl |
| XIV-15 | CH₃ | 4-Cl-C₆H₄ | -(CH₂)₂-CH-t-C₄H₉-(CH₂)₂- | | H | H | Öl |
| XIV-16 | CH₃ | 4-Cl-C₆H₄ | -(CH₂)₂-CH-C₂H₅-(CH₂)₂- | | H | H | Öl |
| XIV-17 | H | 4-Cl-C₆H₄ | -(CH₂)₅- | | H | H | Öl |

### Beispiel I-2-a-1

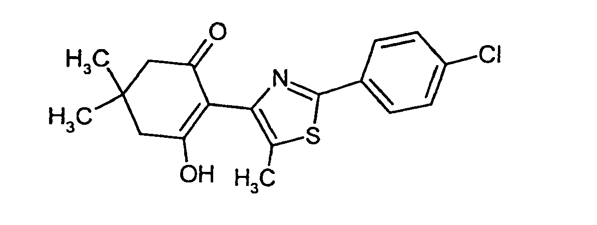

2,16 g (18,1 mmol) Kalium-tert.-butylat werden in 20 ml wasserfreien Dimethylformamid vorgelegt und 4,6 g (12,1 mmol) der Verbindung gemäß Beispiel III-1 in 2 ml wasserfreiem Dimethylformamid zugetropft.

Es wird 2 Stunden bei 50°C gerührt.

Die Reaktionslösung wird in 100 ml Eiswasser gegeben und diese in 500 ml kalte 1N Salzsäure-Lösung eingerührt. Der Niederschlag wird abgesaugt und in Dichlormethan aufgenommen. Nach Abdestillieren des Lösungsmittels wird der Rückstand säulenchromatographisch an Kieselgel gereinigt (Cyclohexan/Essigsäureethylester, 5:1).
Ausbeute: 1,4 g (13 % der Theorie), Fp. 165-167°C

In Analogie zu Beispiel (I-2-a-1) und gemäß den allgemeinen Angaben zur Herstellung wurden folgende Verbindungen der Formel (I-2-a) erhalten:

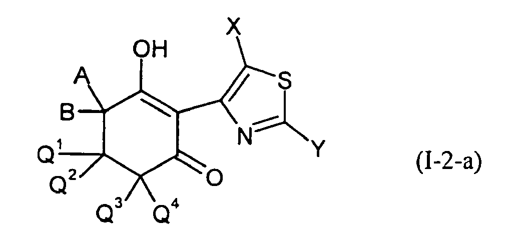

| **Bsp. Nr.** | **X** | **Y** | **A** | **B** | **Q**^{**1**} | **Q**^{**2**} | **Q**^{**3**} | **Q**^{**4**} | **Fp.°C** |
|---|---|---|---|---|---|---|---|---|---|
| I-2-a-2 | CH₃ | 4-Cl-C₆H₄ | H | H | H | H | H | H | 106 |
| I-2-a-3 | CH₃ | 4-Cl-C₆H₄ | CH₃ | CH₃ | H | H | H | H | Öl |
| I-2-a-4 | CH₃ | 4-Cl-C₆H₄ | H | H | -(CH₂)₄- | | H | H | 122 |
| I-2-a-5 | CH₃ | 4-Cl-C₆H₄ | H | H | 4-Cl-C₆H₄ | H | H | H | 195-197 |

### Beispiel I-2-b-1

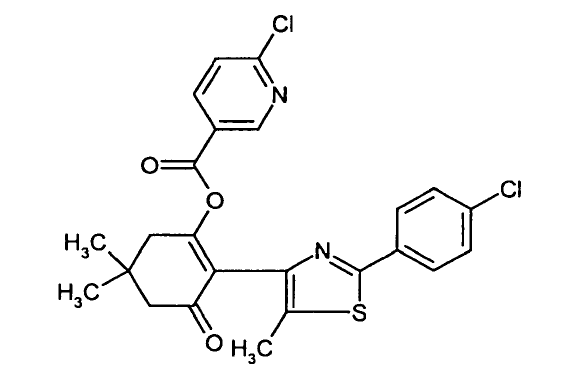

500 mg (1,44 mmol) der Verbindung gemäß Beispiel I-2-a-1 werden in 5 ml wasserfreiem Dichlormethan vorgelegt und mit 0,29 ml (2,16 mmol) Triethylamin versetzt. Bei 0°C werden 0,33 g (1,87 mmol) 6-Chlornicotinylchlorid zugegeben und es wird 2 h bei Raumtemperatur gerührt.

Das Reaktionsgemisch wird mit 10 %iger Zitronensäure extrahiert, die wässrige Phase mit Dichlormethan gewaschen, die organische Phase mit 1 N Natriumhydroxid-Lösung extrahiert und die wässrige Phase mit Dichlormethan gewaschen, getrocknet und eingeengt.
Ausbeute: 0,7 g (100 % d. Th.), Fp. 127-130°C

In Analogie zu Beispiel (I-2-b-1) und gemäß den allgemeinen Angaben zur Herstellung wurden folgende Verbindungen der Formel (I-2-b) erhalten:

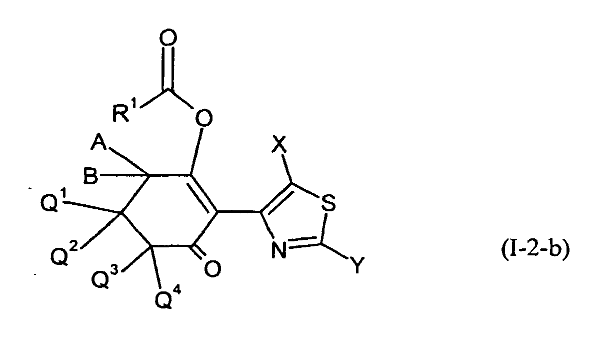

| **Bsp. Nr.** | **X** | **Y** | **A** | **B** | **Q**^{**1**} | **Q**^{**2**} | **Q**^{**3**} | **Q**^{**4**} | **R**^{**1**} | **Fp.°C** |
|---|---|---|---|---|---|---|---|---|---|---|
| I-2-b-2 | CH₃ | 4-Cl-C₆H₄ | H | H | CH₃ | CH₃ | H | H | i-C₃H₇- | 96 |
| I-2-b-3 | CH₃ | 4-Cl-C₆H₄ | H | H | CH₃ | CH₃ | H | H | H₅C₂-O-CH₂- | 142 |
| I-2-b-4 | CH₃ | 4-Cl-C₆H₄ | H | H | CH₃ | CH₃ | H | H | 4-Cl-C₆H₄ | 123-125 |
| I-2-b-5 | CH₃ | 4-Cl-C₆H₄ | H | H | CH₃ | CH₃ | H | H | 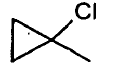 | 128 |

### Beispiel I-2-c-1

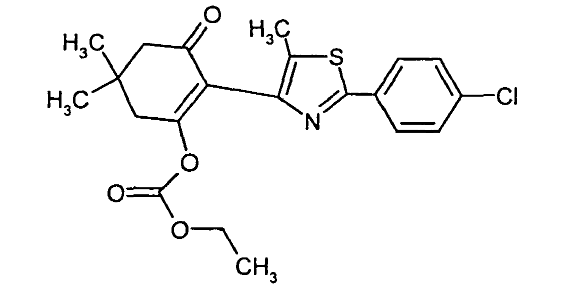

600 mg (1,7 mmol) der Verbindung gemäß Beispiel I-2-a-1 werden in 5 ml wasserfreiem Dichlormethan vorgelegt und mit 0,35 ml (2,60 mmol) Triethylamin versetzt. Bei 0°C werden 0,255 g (2,21 mmol) Chlorameisensäureethylester zugegeben und 2 Stunden bei Raumtemperatur gerührt.

Die Lösung wird mit 10 %iger Zitronensäure gewaschen und die wässrige Phase mit Dichlormethan extrahiert. Die organische Phase wird mit 1 N NatriumhydroxidLösung gewaschen, getrocknet und eingeengt.
Ausbeute: 0,62 g (87 % d.Th.), Fp. 82-86°C

In Analogie zu Beispiel (I-2-c-1) und gemäß den allgemeinen Angaben zur Herstellung wurden folgende Verbindungen der Formel (1-2-c) erhalten:

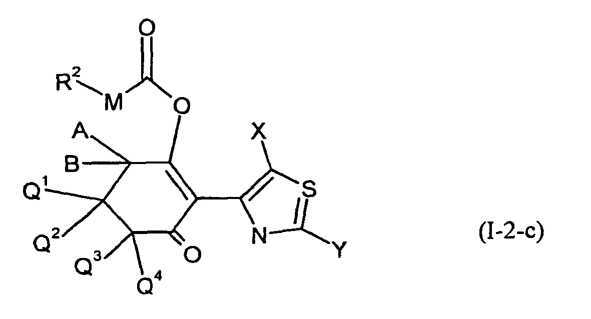

| **Bsp.- Nr.** | **X** | **Y** | **A** | **B** | **Q**^{**1**} | **Q**^{**2**} | **Q**^{**3**} | **Q**^{**4**} | **M** | **R**^{**2**} | **Fp.°C** |
|---|---|---|---|---|---|---|---|---|---|---|---|
| I-2-c-2 | CH₃ | 4-Cl-C₆H₄ | H | H | CH₃ | CH₃ | H | H | O | C₆H₅- | 123-125 |
| I-2-c-3 | CH₃ | 4-Cl-C₆H₄ | H | H | CH₃ | CH₃ | H | H | O | C₆H₅-CH₂ | 129-131 |
| I-2-c-4 | CH₃ | 4-Cl-C₆H₄ | H | H | CH₃ | CH₃ | H | H | S | C₆H₅-CH₂ | 94 |

### Beispiel Nr. I-2-d-1

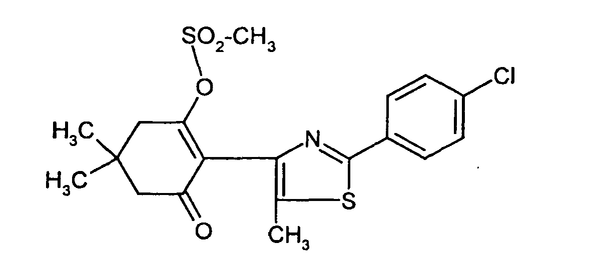

500 mg (1,44 mmol) der Verbindung gemäß Beispiel I-2-a-1 werden in 5 ml wasserfreiem Dichlormethan vorgelegt und mit 0,29 ml (2,16 mmol) Triethylamin versetzt. Bei 0°C werden 0,22 g (1,87 mmol) Methylsulfonsäurechlorid zugegeben und es wird 2 h bei Raumtemperatur gerührt.

Das Reaktionsgemisch wird mit 10 %iger Citronensäure extrahiert, die wässrige Phase mit Dichlormethan gewaschen, die organische Phase mit 1 N NaOH extrahiert und die wässrige Phase mit Dichlormethan gewaschen, getrocknet und eingeengt.
Ausbeute: 0,65 g (100 % d. Th.), Wachs
1H-NMR (d₆-DMSO, 400 MHz):
δ = 1,17 (s, 6H, cyc-Hexyl-CH₃), 2,25 (s, 3H, Thiazolyl-CH₃), 2.48 (s, 2H, cyc-Hexyl-CH₂), 2.86 (s, 2H, cyc-Hexyl-CH₂), 3.21 (s, 3H, SO₂-CH₃), 7.52 (d, 2H, Ar-H), 7.83 (d, 2H, Ar-H) ppm.

### Beispiel Nr. I-2-g-1

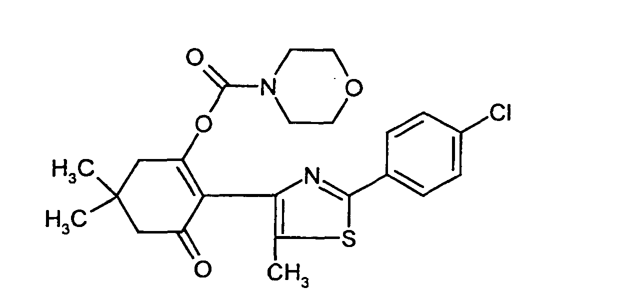

500 mg (1,44 mmol) der Verbindung gemäß Beispiel I-2-a-1 werden in 5 ml wasserfreiem Dichlormethan vorgelegt und mit 0,29 ml (2,16 mmol) Triethylamin versetzt. Bei 0°C werden 0,27 g (1,87 mmol) Morpholincarbonsäurechlorid zugegeben und es wird 2 h bei Raumtemperatur gerührt.

Das Reaktionsgemisch wird mit 10 %iger Zitronensäure extrahiert, die wässrige Phase mit Dichlormethan gewaschen, die organische Phase mit 1 N Natriumhydroxid-Lösung extrahiert und die wässrige Phase mit Dichlormethan gewaschen, getrocknet und eingeengt.
Ausbeute: 0,65 g (100 % d. Th.), Fp. 128°C
1H-NMR (d₆-DMSO, 400 MHz):
δ = 1.13 (s, 6H, cyc-Hexyl-CH₃), 2.21 (s, 3H, Thiazolyl-CH₃), 2.46 (s, 2H, cyc-Hexyl-CH₂), 2.71 (s, 2H, cyc-Hexyl-CH₂), 3.06-3.80 (m, 8H, Morpholin-CH₂), 7.53 (d, 2H, Ar-H), 7.84 (d, 2H, Ar-H) ppm.

### Beispiel III-1

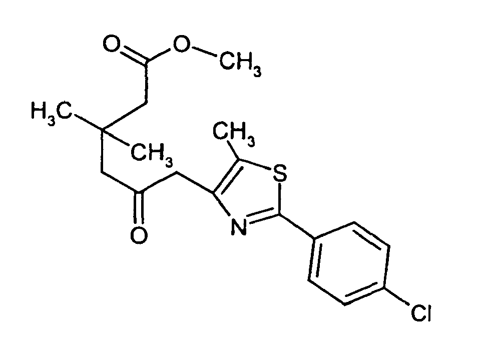

17,0 g (30 mmol) Rohprodukt der Verbindung gemäß Beispiel XIX-1 werden in 200 ml wasserfreiem Aceton vorgelegt und 6,42 g (46,5 mmol) Kaliumcarbonat und 19,5 g (139,5 mmol) Methyliodid zugesetzt. Es wird 16 h unter Rückfluß gerührt.

Man filtriert und engt ein. Der Rückstand wird säulenchromatographisch an Kieselgel gereinigt (Dichlormethan : Petrolether, 4 : 1 → Dichlormethan → Dichlormethan : Essigsäureethylester, 30 : 1 → 10 : 1)
Ausbeute: 4,6 g (16 % der Theorie), Öl
¹H-NMR (400 MHz, d₆-DMSO):
δ = 1,03 (s, 6H, 2-CH₃-C-aliph.), 2,32 (s, 3H, CH₃-C-arom.) 7,50 (d, 2H, Ar-H.), 7,81 (d, 2H, Ar-H) ppm.

### Beispiel XIX-1

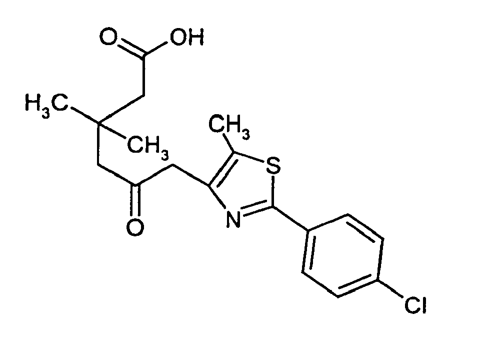

Zu einer Lösung von 25 ml LDA-Lösung (2 molar) in 50 ml wasserfreiem Tetrahydrofuran wird eine Lösung von 12,7 g (45 mmol) 4-[2-(4-Chlorphenyl)-5-methyl]-thiazolyl-essigsäure-methylester in 15 ml wasserfreiem Tetrahydrofuran bei -15°C zugetropft und 60 min bei 0°C gerührt.

Dann wird bei -15°C eine Lösung von 4,26 g (30 mmol) 3,3-Dimethylglutarsäureanhydrid in 10 ml Tetrahydrofuran zugetropft.

Die Lösung wird zwei Stunden bei Raumtemperatur gerührt, dann werden 75 ml Wasser und 20 g Ammoniumchlorid zugegeben und anschließend mit konzentrierter Salzsäure eine saure Lösung eingestellt.

Das Zwischenprodukt wird mit Ether extrahiert und das Lösungsmittel abdestilliert. Der Rückstand wird mit 25 g Kaliumhydroxid und 170 ml Wasser einen Tag unter Rückfluss gekocht.

Man kühlt ab, säuert mit konzentrierter Salzsäure an und extrahiert mit Ether. Das Rohprodukt wird direkt ohne Reinigung weiter umgesetzt.
Ausbeute: 17 g (100 % der Theorie), Öl

In Analogie zu Beispiel (III-1) und gemäß den allgemeinen Angaben zur Herstellung wurden folgende Verbindungen der Formel (III) erhalten:

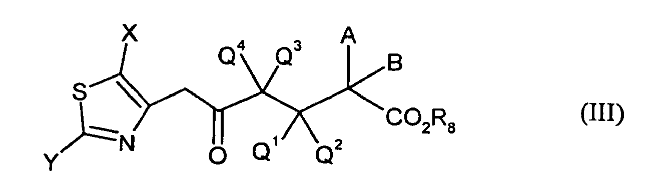

| **Bsp. Nr.** | **X** | **Y** | **A** | **B** | **Q**^{**1**} | **Q**^{**2**} | **Q**^{**3**} | **Q**^{**4**} | **R**^{**8**} | **Fp.°C** |
|---|---|---|---|---|---|---|---|---|---|---|
| III-2 | CH₃ | 4-Cl-C₆H₄ | H | H | H | H | H | H | CH₃ | Öl |
| III-3 | CH₃ | 4-Cl-C₆H₄ | CH₃ | CH₃ | H | H | H | H | CH₃ | Öl |
| III-4 | CH₃ | 4-Cl-C₆H₄ | H | H | -(CH₂)₄- | | H | H | CH₃ | Öl |
| III-5 | CH₃ | 4-Cl-C₆H₄ | H | H | 4-Cl-C₆H₄ | H | H | H | CH₃ | Öl |

In Analogie zu Beispiel (XIX-1) und gemäß den allgemeinen Angaben zur Herstellung wurden folgende Verbindungen der Formel (XIX) erhalten:

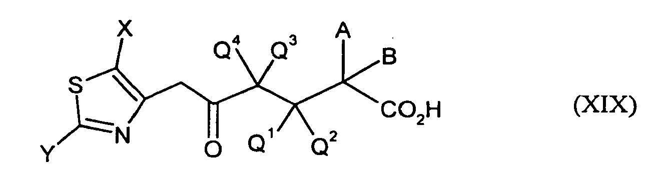

| **Bsp. Nr.** | **X** | **Y** | **A** | **B** | **Q**^{**1**} | **Q**^{**2**} | **Q**^{**3**} | **Q**^{**4**} | **Fp.°C** |
|---|---|---|---|---|---|---|---|---|---|
| XIX-2 | CH₃ | 4-Cl-C₆H₄ | H | H | H | H | H | H | Öl |
| XIX-3 | CH₃ | 4-Cl-C₆H₄ | CH₃ | CH₃ | H | H | H | H | Öl |
| XIX-4 | CH₃ | 4-Cl-C₆H₄ | H | H | -(CH₂)₄- | | H | H | Öl |
| XIX-5 | CH₃ | 4-Cl-C₆H₄ | H | H | 4-Cl-C₆H₄ | H | H | H | Öl |

### Anwendungsbeispiele

### Beispiel A

### Meloidogyne-Test

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100%, dass keine Gallen gefunden wurden; 0 % bedeutet, dass die Zahl der Gallen an den behandelten Pflanzen der der unbehandelten Kontrolle entspricht.

Bei diesem Test zeigte z. B. die folgenden Verbindungen der Herstellungsbeispiele gute Wirksamkeit:

| Wirkstoffe | Wirkstoffkonzentration in ppm | Abtötungsgrad In % nach 14^{d} |
|---|---|---|
| **Bsp. I-1-c-3** | 20 | 100 |
| **Bsp. I-1-a-1** | 20 | 95 |

### Beispiel B

### Myzus-Test (systemische Wirkung)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Mit je 10 ml Wirkstoffzubereitung der gewünschten Konzentration werden Wirsingkohl-Pflanzen (Brassica oleracea), die stark von der Grünen Pfirsichblattlaus (Myzus persicae) befallen sind, angegossen, so dass die Wirkstoffzubereitung in den Boden eindringt, ohne den Spross zu benetzen. Der Wirkstoff wird von den Wurzeln aufgenommen und in den Spross weitergeleitet.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele gute Wirksamkeit:

| Wirkstoffe | WirkstoffkonZentration in ppm | Abtötungsgrad in % nach 10^{d} |
|---|---|---|
| **Bsp.I-1-c-2** | 20 | 95 |

### Beispiel C

### Phaedon-Larven-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100%, dass alle Käferlarven abgetötet wurden; 0% bedeutet, dass keine Käferlarven abgetötet wurden.

Bei diesem Test zeigen z. B. die folgenden Verbindungen der Herstellungsbeispiele gute Wirksamkeit:

| Wirkstoffe | Wirkstoffkonzentration in ppm | Abtötungsgrad in % nach 7^{d} |
|---|---|---|
| **Bsp. 1-1-c-1** | 500 | 100 |
| **Bsp. 1-2-c-1** | 500 | 100 |

### Beispiel D

### Spodoptera frugiperda-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die folgenden Verbindungen der Herstellungsbeispiele gute Wirksamkeit:

| Wirkstoff | Wirkstoffkonzentration in ppm | Abtötungsgrad in % nach 7^{d} |
|---|---|---|
| **Bsp. I-1-a-2** | 500 | 100 |
| **Bsp. I-2-a-1** | 500 | 100 |
| **Bsp. I-1-c-1** | 500 | 100 |
| **Bsp. I-2-c-1** | 500 | 100 |

### Beispiel E

### Tetranychus-Test (OP-resistent/Tauchbehandlung)

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100%, dass alle Spinnmilben abgetötet wurden; 0% bedeutet, dass keine Spinnmilben abgetötet wurden.

Bei diesem Test zeigen z. B. die folgenden Verbindungen der Herstellungsbeispiele gute Wirksamkeit:

| Wirkstoff | Wirkstoffkon-Zentration in ppm | Abtötungsgrad in % nach 7^{d} |
|---|---|---|
| **Bsp. I-1-c-3** | 100 | 98 |
| **Bsp. I-1-a-1** | 100 | 100 |
| **Bsp. I-2-a-2** | 100 | 99 |
| **Bsp. I-2-a-1** | 100 | 98 |
| **Bsp. I-1-c-1** | 100 | 95 |
| **Bsp. I-2-c-1** | 100 | 100 |

### Beispiel F1

### Post-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, dass die jeweils gewünschten Wirkstoffmengen pro Flächeneinheit ausgebracht werden. Die Konzentration der Spritzbrühe wird so gewählt, dass in 1000 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

### Beispiel F2

### Pre-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 1000 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

| **post emergence/ Gewächshaus** | **g a.i./ha** | **Alopecurus** | **Avena fatua** | **Setaria** | **Sinapis** |
|---|---|---|---|---|---|
| I-1-a-2 | 250 | 90 | 100 | 100 | 80 |
| 1-1-a-3 | 250 | 90 | 100 | 100 | 80 |
| I-1-c-3 | 250 | 100 | 100 | 100 | 80 |
| I-1-a-1 | 250 | 100 | 100 | 100 | 70 |

| **post-emergence/ Gewächshaus** | **g a.i./ha** | **Zuckerrüben** | **Alopecurus** | **Avena fatua** | **Echinochloa** | **Setaria** |
|---|---|---|---|---|---|---|
| I-2-a-1 | 250 | 0 | 90 | 95 | 100 | 100 |

| **post-emergence/ Gewächshaus** | **g a.i./ha** | **Zuckerrüben** | **Avena fatua** | **Echinochloa** | **Setaria** | **Sinapis** |
|---|---|---|---|---|---|---|
| I-1-c-1 | 250 | 0 | 90 | 100 | 100 | 70 |

| **post-emergence/ Gevächshaus** | **g a.i./ha** | **Alopecurus** | **Avena fatua** | **Setaria** | **Galium** |
|---|---|---|---|---|---|
| I-2-c- 1 | 250 | 90 | 100 | 100 | 80 |

| **pre-emergence/Gewächshaus** | **g a.i./ha** | **Weizen** | **Soja** | **Digitaria** | **Lolium** | **Setaria** | **Matricaria** |
|---|---|---|---|---|---|---|---|
| I-1-a-2 | 60 | 0 | 0 | 100 | 100 | 100 | 95 |

| **pre-emergence/Gewächshaus** | **g a.i./ha,** | **Zuckerrüben** | **Alopecurus** | **Avena fatua** | **Digitaria** | **Polygonum** |
|---|---|---|---|---|---|---|
| I-1-a-3 | 125 | 0 | 95 | 100 | 100 | 100 |

| **pre-emergence/ Gewächsshaus** | **g a.i./ha** | **Raps** | **Alopecurus** | **Digitaria** | **Lolium** | **Stellaria** |
|---|---|---|---|---|---|---|
| I-1-c-2 | 125 | 0 | 95 | 100 | 100 | 100 |

| **pre-emergence/ Gewächsshaus** | **g a.i./ha** | **Weizen** | **Soja** | **Alopecurus** | **Lolium** | **Setaria** | **Stellaria** |
|---|---|---|---|---|---|---|---|
| I-1-c-3 | 125 | 10 | 0 | 95 | 100 | 100 | 70 |

| **pre-emergence/ Gewächsshaus** | **g a.i./ha** | **Soja** | **Lolium** | **Setaria** | **Matricaria** | **Stellaria** |
|---|---|---|---|---|---|---|
| I-1-a-1 | 125 | 0 | 100 | 100 | 100 | 100 |

| **pre-emergence/ Gewächsshaus** | **g a.i./ha** | **Soja** | **Bromus** | **Lolium** | **Setaria** | **Abutilon** |
|---|---|---|---|---|---|---|
| I-2-a-1 | 125 | 0 | 95 | 100 | 100 | 70 |

### Beispiel G

### In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS 16, pipettiert. Nachdem das Lösungsmittel abgedampft ist, werden je Kavität 200 µl Potatoe-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0,1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet.

| Wirkstoff | Mikroorganismus | ED₅₀-Wert |
|---|---|---|
| Bsp. I-1-a-2 | Pyricularia oryzae | < 0,1 |
| | Septoria tritici | < 0,1 |
| | Ustilago avenae | < 0,1 |
| Bsp. I-2-a-2 | Pyricularia oryzae | 1,96 |
| | Septoria tritici | <0,1 |
| | Ustilago avenae | < 0,1 |
| Bsp. I-2-a-3 | Pyricularia oryzae | < 0,1 |
| | Septoria tritici | < 0,1 |
| | Ustilago avenae | 0,1 |

### Beispiel H

### Sphaerotheca-Test (Gurke) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkstoffgrad, der demjenigen der Kontrolle entspricht, während ein Wirkungsgrad von 100 % bedeutet, dass kein Befall beobachtet wird.

| Wirkstoff | Aufwandmenge an Wirkstoff in g/ha | % Wirkungsgrad |
|---|---|---|
| Bsp. I-1-a-2 | 100 | 100 |

### Beispiel I

### Venturia-Test (Apfel)/protektiv

| | | |
|---|---|---|
| Lösungsmittel: | 24,5 | Gewichtsteile Aceton |
| | 24, 5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 %iger relativer Luftfeuchtigkeit in einer Inukubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| Wirkstoff | Aufwandmenge Wirkstoff in g/ha | % Wirkungsgrad |
|---|---|---|
| Bsp. I-1-a-2 | 100 | 91 |

### Beispiel J

### Alternaria-Test (Tomate) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünsche Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Alternaria solani inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| Wirkstoff | Aufwandmenge an Wirkstoff in g/ha | % Wirkungsgrad |
|---|---|---|
| Bsp. I-1-a-2 | 100 | 93 |

### Beispiel K

### Botrytis-Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

| Wirkstoff | Aufwandmenge an Wirkstoff in g/ha | % Wirkungsgrad |
|---|---|---|
| Bsp. I-1-a-2 | 500 | 92 |

### Beispiel L

### Grenzkonzentrations-Test / Bodeninsekten - Behandlung transgener Pflanzen

| | |
|---|---|
| Testinsekt: | Diabrotica balteata- Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel M

### Heliothis virescens - Test - Behandlung transgener Pflanzen

| | | |
|---|---|---|
| Lösungsmittel: | 7 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 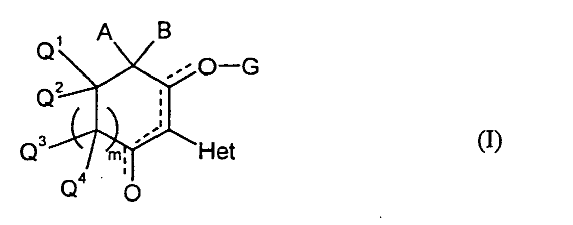 in welcher
Het für 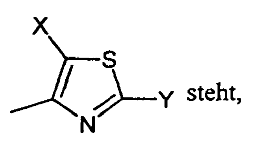
m für die Zahlen 0 oder 1 steht,
X für Wasserstoff, Chlor, Brom oder C₁-C₄-Alkyl steht,
Y für Chlor, Brom, C₁-C₄-Alkyl, C₁-C₄-Halogenalkyl, C₁-C₄-Alkoxy, C₁-C4-Halogenalkoxy oder für die Gruppe 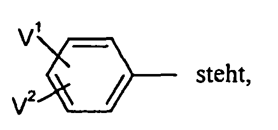
V¹ für Wasserstoff, Fluor, Chlor, C₁-C₆-Alkyl, C₁-C₄-Alkoxy, C₁-C₂₋Halogenalkyl, C₁-C₂-Halogenalkoxy, Nitro, Cyano oder gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy steht,
V² für Wasserstoff, Fluor, Chlor, Brom, C₁-C₄-Alkyl, C₁-C₄-Alkoxy, C₁₋C₂-Halogenalkyl oder C₁-C₂-Halogenalkoxy steht,
V¹ und V² zusammen gemeinsam mit den Kohlenstoffatomen, an die sie gebunden sind, für einen gegebenenfalls durch Fluor oder Methyl substituierten 5- oder 6-gliedrigen Cyclus stehen, in welchem gegebenenfalls ein bis zwei Kohlenstoffatome durch Sauerstoff ersetzt sein können,
A für Wasserstoff, für jeweils gegebenenfalls einfach bis dreifach durch Fluor substituiertes C₁-C₈-Alkyl oder C₁-C₄-Alkoxy-C₁-C₂-alkyl, für jeweils gegebenenfalls einfach bis zweifach durch Fluor, Chlor, Methyl, Ethyl oder Methoxy substituiertes C₅-C₆-Cycloalkyl oder C₃₋C₆-Cycloalkyl-C₁-C₂-alkyl, in welchem gegebenenfalls ein Ringglied durch Sauerstoff oder Schwefel ersetzt ist oder jeweils gegebenenfalls einfach bis zweifach durch Fluor, Chlor, Brom, C₁-C₄-Alkyl, C₁-C₂₋Halogenalkyl, C ₁-C₄-Alkoxy oder C ₁ -C₂-Halogenalkoxy substituiertes Phenyl oder Benzyl steht,
B für Wasserstoff oder C₁ -C₄-Alkyl steht,
A, B und das Kohlenstoffatom an das sie gebunden sind, für gesättigtes C₅₋C₆-Cycloalkyl stehen, worin gegebenenfalls ein Ringglied durch Sauerstoff ersetzt ist und welches gegebenenfalls einfach durch C₁₋C₄-Alkyl, Trifluormethyl oder C ₁-C₄-Alkoxy substituiert ist,
mit der Maßgabe, dass dann Q¹ nur für Wasserstoff oder C₁-C₄-Alkyl steht,
A und Q¹ gemeinsam für gegebenenfalls einfach oder zweifach durch Methyl, Ethyl, Methoxy oder Ethoxy substitutiertes C₃-C₄-Alkandiyl stehen,
Q¹ für Wasserstoff, C₁-C₄-Alkyl, C₁-C₄-Alkoxy-C₁-C₂-alkyl, oder gegebenenfalls durch Methyl oder Methoxy substituiertes C₃-C₆₋Cycloalkyl, worin gegebenenfalls eine Methylengruppe durch Sauerstoff ersetzt ist, oder jeweils gegebenenfalls einfach bis zweifach durch Fluor, Chlor, Brom, C₁-C₄-Alkyl, C₁-C₄-Alkoxy, Trifluormethyl oder Trifluormethoxy substituiertes Phenyl oder Benzyl steht,
Q², Q³, Q⁴ unabhängig voneinander für Wasserstoff, Methyl oder Ethyl stehen,
Q ¹ und Q² gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl stehen, in welchem gegebenenfalls ein Ringglied durch Sauerstoff ersetzt ist,
mit der Maßgabe, dass dann A nur für Wasserstoff oder C₁-C₄-Alkyl steht,
G für Wasserstoff (a) oder für eine der Gruppen 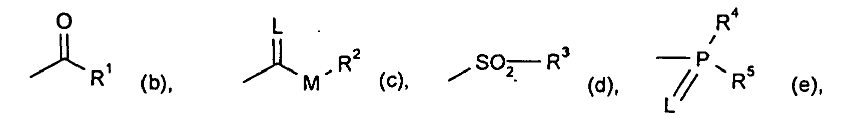 E (f) oder 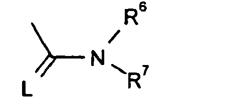 (g) steht
in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis fünffach durch Fluor oder Chlor substituiertes C₁-C₁₆-Alkyl, C₂-C₁₆-Alkenyl, C₁-C₄-Alkoxy-C₁₋C₂-alkyl, C₁-C₄-Alkylthio-C₁-C₂-alkyl, oder gegebenenfalls einfach bis zweifach durch Fluor, Chlor, C₁-C₄-Alkyl oder C₁-C₅-Alkoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls einfach bis zweifach durch Fluor, Chlor, Brom, Cyano, Nitro, C₁-C₄-Alkyl, C₁-C₄-Alkoxy, Trifluormethyl oder Trifluormethoxy substituiertes Phenyl,
für jeweils gegebenenfalls einfach durch Fluor, Chlor, Brom, Methyl, Ethyl oder Trifluormethyl substituiertes Pyridyl oder Thienyl steht,
R² für C₁-C₁₆-Alkyl, C₂-C₁₆-Alkenyl oder C -C₄-Alkoxy-C₂-C₄-alkyl,
für gegebenenfalls einfach bis zweifach durch Methyl, Ethyl oder Methoxy substituiertes C₃-C₆-Cycloalkyl,
für jeweils gegebenenfalls einfach bis zweifach, durch Fluor, Chlor, Brom, Cyano, Nitro, C₁-C₄-Alkyl, C₁-C₄-Alkoxy, Trifluormethyl oder Trifluormethoxy substituiertes Phenyl oder Benzyl steht,
R³ für gegebenenfalls einfach bis fünffach durch Fluor substituiertes C₁₋C₄-Alkyl oder für gegebenenfalls einfach bis zweifach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino,Di-(C₁-C₆₋alkyl)amino, C₁-C₆-Alkylthio, oder für jeweils gegebenenfalls einfach bis zweifach durch Fluor, Chlor, Brom, Nitro, Cyano, C₁-C₃-Alkoxy, Trifluormethoxy, C₁-C₃-Alkyl oder Trifluormethyl substituiertes Phenyl, Benzyl, Phenoxy oder Phenylthio steht,
R⁵ für C₁-C₄-Alkyl, C₁-C₄-Alkoxy oder C₁-C₄-Alkylthio steht,
R⁶ für Wasserstoff, C₁-C₆-Alkyl, C₃-C₆-Cycloalkyl, C₁-C₆-Alkoxy, C₃₋C₆-Alkenyl, C₁-C₆-Alkoxy-C₁-C₆-alkyl, für gegebenenfalls einfach bis zweifach durch Fluor, Chlor, Brom, Trifluormethyl, C₁-C₄-Alkyl oder C₁-C₄-Alkoxy substituiertes Phenyl, für gegebenenfalls durch Fluor, Chlor, Brom, Methyl, Ethyl, Trifluormethyl oder Methoxy substituiertes Benzyl steht,
R⁷ für Wasserstoff, C₁-C₆-Alkyl oder C₃-C₆-Alkenyl steht,
R⁶ und R⁷ zusammen mit dem Stickstoffatom, an das sie gebunden sind, für einen gegebenenfalls einfach bis zweifach durch Methyl oder Ethyl substituierten C₅-C₆-Alkylenrest stehen, in welchem gegebenenfalls eine Methylengruppe durch Sauerstoff oder Schwefel ersetzt ist.

2. Verbindungen der Formel (I) gemäß Anspruch 1, in welcher
Het für 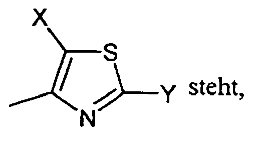
m für die Zahlen 0 oder 1 steht,
X für Wasserstoff, Chlor, Brom, Methyl, Ethyl, n-Propyl oder iso-Propyl steht,
Y für die Gruppe 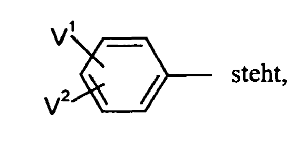
V¹ für Wasserstoff, Fluor, Chlor, Brom, Methyl, Ethyl, n-Propyl, iso-Propyl, n-Butyl, iso-Butyl, tert-Butyl, Methoxy, Ethoxy, n-Propoxy, iso-Propoxy, Trifluormethyl, Trifluormethoxy oder 4-Chlorphenoxy steht,
V² für Wasserstoff, Fluor, Chlor, Brom, Methyl, Methoxy oder Trifluormethyl steht,
A für Wasserstoff, Methyl, Ethyl, n-Propyl, iso-Propyl, n-Butyl, iso-Butyl, tert-Butyl, Methoxymethyl, Ethoxymethyl, Phenyl oder Cyclohexyl steht,
B für Wasserstoff, Methyl oder Ethyl steht,
A, B und das Kohlenstoffatom, an das sie gebunden sind, für gesättigtes C₅₋C₆-Cycloalkyl stehen, in welchem gegebenenfalls ein Ringglied durch Sauerstoff ersetzt ist und welches gegebenenfalls einfach durch Methyl, Ethyl, n-Propyl, Isopropyl, n-Butyl, Isobutyl, tert.-Butyl, Trifluormethyl, Methoxy, Ethoxy, n-Propoxy, n-Butoxy oder Isobutoxy substituiert ist,
mit der Maßgabe, dass dann Q ¹ nur für Wasserstoff steht,
A und Q¹ gemeinsam für C₃-C₄-Alkandiyl stehen,
Q¹ für Wasserstoff, Methyl, Ethyl, n-Propyl, iso-Propyl, Cyclopropyl, Cyclopentyl, Cyclohexyl oder 4-Chlorphenyl steht,
Q², Q³, Q⁴ unabhängig voneinander für Wasserstoff, Methyl oder Ethyl stehen,
Q¹ und Q² gemeinsam mit dem Kohlenstoffatom, an das sie gebunden sind, für gegebenenfalls durch Methyl, Ethyl, Methoxy, Ethoxy, n-Propoxy oder n-Butoxy substituiertes gesättigtes C₅-C₆-Cycloalkyl stehen, in welchem gegebenenfalls ein Ringglied durch Sauerstoff ersetzt ist,
mit der Maßgabe, dass dann A nur für Wasserstoff steht,
G für Wasserstoff (a) oder für eine der Gruppen 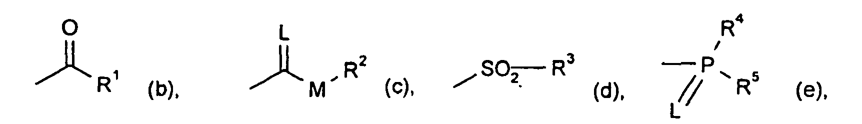 E (f) oder 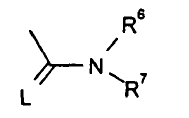 (g) steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁₋Cg-Alkyl, C₂-C₈-Alkenyl, C₁-C₂-Alkoxy-C₁-C₂-alkyl, C₁-C₂₋Alkylthio-C₁-C₂-alkyl oder jeweils gegebenenfalls einfach durch Fluor, Chlor, Methyl, Ethyl oder Methoxy substituiertes Cyclopropyl oder Cyclohexyl,
für gegebenenfalls einfach durch Fluor, Chlor, Brom, Cyano, Nitro, Methyl, Ethyl, iso-Propyl, tert-Butyl, Methoxy, Trifluormethyl oder Trifluormethoxy substituiertes Phenyl,
für jeweils gegebenenfalls einfach durch Fluor, Chlor, Brom oder Methyl substituiertes Thienyl oder Pyridyl steht,
R² für C₁-C₈-Alkyl, C₂-C₈-Alkenyl oder C₁-C₄-Alkoxy-C₂-alkyl,
für gegebenenfalls einfach durch Methyl, Ethyl oder Methoxy substituiertes C₃-C₆-Cycloalkyl,
für jeweils gegebenenfalls einfach durch Fluor, Chlor, Cyano, Nitro, Methyl, Ethyl, iso-Propyl, tert-Butyl, Methoxy, Trifluormethyl oder Trifluormethoxy substituiertes Phenyl oder Benzyl steht,
R³ für jeweils gegebenenfalls dreifach durch Fluor substituiertes Methyl oder Ethyl oder gegebenenfalls einfach durch Fluor, Chlor, Brom, Methyl, tert-Butyl, Methoxy, Trifluormethyl, Trifluormethoxy, Cyano oder Nitro substituiertes Phenyl steht,
R⁴ für C₁-C₄-Alkyl, C₁-C₄-Alkoxy, C₁-C₄-Alkylamino, Di-(C₁-C₄₋alkyl)amino, C₁-C₄-Alkylthio oder für jeweils gegebenenfalls einfach durch Fluor, Chlor, Brom, Nitro, Cyano, C₁-C₂-Alkoxy, Trifluormethoxy oder C₁-C₃-Alkyl substituiertes Phenyl, Phenoxy oder Phenylthio steht,
R⁵ für Methyl, Ethyl, Methoxy, Ethoxy, Methylthio oder Ethylthio steht,
R⁶ für Wasserstoff, für C₁-C₄-Alkyl, C₃-C₆-Cycloalkyl, C₁-C₄-Alkoxy, C₃-C₄-Alkenyl oder C₁-C₄-Alkoxy-C₁-C₄-alkyl steht,
R⁷ für Wasserstoff, C₁-C₄-Alkyl oder C₃-C₄-Alkenyl steht,
R⁶ und R⁷ zusammen mit dem Stickstoffatom, an das sie gebunden sind, für einen C₅-Alkylenrest stehen, in welchem gegebenenfalls eine Methylengruppe durch Sauerstoff ersetzt ist.

3. Verbindungen der Formel (I) gemäß Anspruch 1, in welcher
Het für 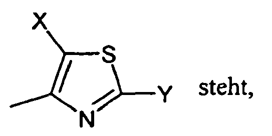
m für die Zahlen 0 oder. 1 steht,
X für Wasserstoff, Methyl oder Ethyl steht,
Y für die Gruppe 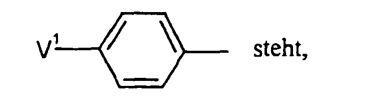
V¹ für Wasserstoff, Chlor, Methyl, Ethyl, n-Propyl, iso-Propyl, n-Butyl, iso-Butyl, tert.-Butyl, Trifluormethyl oder für 4-Chlorphenoxy steht,
A für Wasserstoff, Methyl, Ethyl, n-Propyl, iso-Propyl, n-Butyl, iso-Butyl, Phenyl oder Cyclohexyl steht,
B für Wasserstoff, Methyl oder Ethyl steht,
A, B und das Kohlenstoffatom, an das sie gebunden sind, für gesättigtes C₅₋C₆-Cycloalkyl stehen, in welchem gegebenenfalls ein Ringglied durch Sauerstoff ersetzt ist und welches gegebenenfalls einfach durch Methyl, Ethyl, n-Propyl, Isopropyl, n-Butyl, Isobutyl, tert.-Butyl, Methoxy, Ethoxy, n-Propoxy, n-Butoxy oder Isobutoxy substituiert ist,
mit der Maßgabe, dass dann Q¹ nur für Wasserstoff steht,
A und Q¹ gemeinsam für C₃-C₄-Alkandiyl stehen,
Q¹ für Wasserstoff, Methyl, Ethyl, n-Propyl, iso-Propyl oder 4-Chlorphenyl steht,
Q² für Wasserstoff, Methyl oder Ethyl steht,
Q³ für Wasserstoff steht,
Q⁴ für Wasserstoff steht,
Q¹ und Q² gemeinsam mit dem Kohlenstoffatom, an das sie gebunden sind, für gesättigtes C₅-C₆-Cycloalkyl stehen,
mit der Maßgabe, dass dann A nur für Wasserstoff steht,
G für Wasserstoff (a) oder für eine der Gruppen 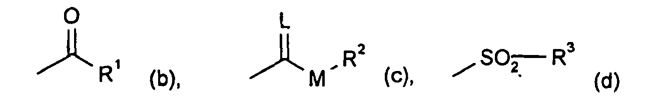 oder 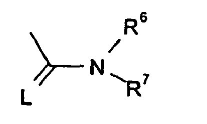 (g) steht,
in welchen
L für Sauerstoff steht und
M für Sauerstoff oder Schwefel steht,
R¹ für C₁-C₈-Alkyl, C₁-C₂-Alkoxy-C₁-C₂-alkyl oder gegebenenfalls einfach durch Chlor substituiertes Cyclopropyl,
für gegebenenfalls einfach durch Chlor substituiertes Phenyl,
für gegebenenfalls einfach durch Chlor substituiertes Pyridyl steht,
R² für C₁-C₈-Alkyl,
für Phenyl oder Benzyl steht,
R³ für Methyl oder Ethyl steht,
R⁶ und R⁷ zusammen mit dem Stickstoffatom, an das sie gebunden sind, für einen C₅-Alkylenrest stehen, in welchem gegebenenfalls eine Methylengruppe durch Sauerstoff ersetzt ist.

4. Verfahren zur Herstellung von Verbindungen der Formel (I) gemäß Anspruch 1, **dadurch gekennzeichnet, dass** man zum Erhalt von
(A) Verbindungen der Formel (I-1-a) 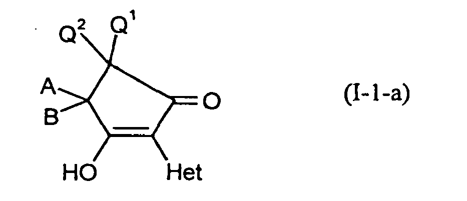 in welcher
A, B, Q¹, Q² und Het die oben angegebenen Bedeutungen haben,
Verbindungen der Formel (II) 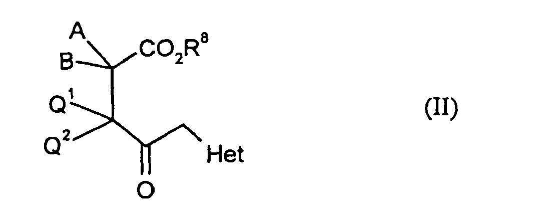 in welcher
A, B, Q¹, Q² und Het die oben angegebenen Bedeutungen haben,
und
R⁸ für Alkyl steht,
gegebenenfalls in Gegenwart eines Verdünnungsmittels und in Gegenwart einer Base intramolekular kondensiert,
(B) Verbindungen der Formel (I-2-a) 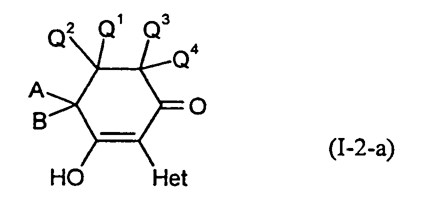 in welcher
A, B, Q¹, Q², Q³, Q⁴, und Het die oben angegebene Bedeutungen haben,
Verbindungen der Formel (III) 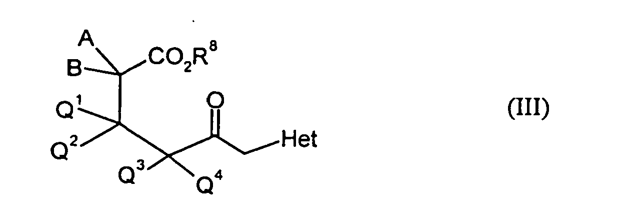 in welcher
A, B, Q¹, Q², Q³, Q⁴ und Het die oben angegebenen Bedeutungen haben,
und
R⁸ für Alkyl steht,
in Gegenwart eines Verdünnungsmittels und in Gegenwart einer Base intramolekular kondensiert,
(C) Verbindungen der oben gezeigten Formeln (I-1-b) bis (1-2-b), in welcher A, B, Q¹, Q², Q³, Q⁴, m, R¹ und Het die oben angebenen Bedeutungen haben, Verbindungen der oben gezeigten Formeln (I-1-a) bis (I-2-a), in welchen A, B, Q¹, Q², Q³, Q⁴, m und Het die oben angegebenen Bedeutungen haben, jeweils
(α) mit Säurehalogeniden der Formel (IV) 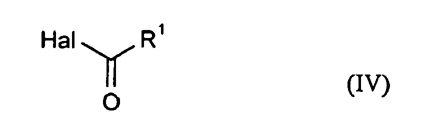 in welcher
R¹ die oben angegebene Bedeutung hat und
Hal für Halogen steht
oder
(β) mit Carbonsäureanhydriden der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der oben gezeigten Formeln (I-1-c) bis (I-2-c), in welchen A, B, Q¹, Q², Q³, Q⁴, m, R², M und Het die oben angegebenen Bedeutungen haben und L für Sauerstoff steht, Verbindungen der oben gezeigten Formeln (I-1-a) bis (I-2-a), in welcher A, B, Q¹, Q², Q³, Q⁴, m und Het die oben angegebenen Bedeutungen haben, jeweils
mit Chlorameisensäureestern oder Chlorameisensäurethioestern der Formel (VI)
R²-M-CO-Cl (VI)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formeln (I-1-c) bis (I-2-c), in welcher A, B, Q¹, Q², Q³, Q⁴, m, R², M und Het die oben angegebenen Bedeutungen haben und L für Schwefel steht, Verbindungen der oben gezeigten Formeln (I-1-a) bis (I-2-a), in welcher A, B, Q¹, Q², Q³, Q⁴, m und Het die oben angegebenen Bedeutungen haben, jeweils
mit Chlormonothioameisensäureestern oder Chlordithioameisensäureestem der Formel (VII) 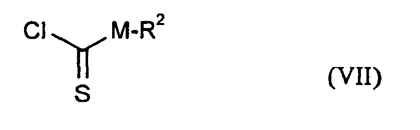 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(F) Verbindungen der oben gezeigten Formeln (I-1-d) bis (I-2-d), in welcher A, B, Q¹, Q², Q³, Q⁴, m, R³ und Het die oben angegebenen Bedeutungen haben, Verbindungen der oben gezeigten Formeln (I-1-a) und (I-2-a), in welcher A, B, Q¹, Q², Q³, Q⁴, m und Het die oben angegebenen Bedeutungen haben, jeweils
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formeln (I-1-e) bis (I-2-e), in welcher A, B, L, Q¹, Q², Q³, Q⁴, m, R⁴, R⁵ und Het die oben angegebenen Bedeutungen haben, Verbindungen der oben gezeigten Formeln (I-1-a) bis (I-2-a), in welcher A, B, Q¹, Q², Q³, Q⁴, m und Het die oben angegebenen Bedeutungen haben, jeweils
mit Phosphorverbindungen der Formel (IX) 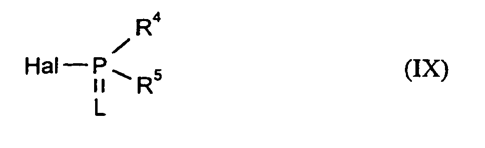 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formeln (I-1-f) bis (I-2-f), in welcher A, B, E, Q¹, Q², Q³, Q⁴, m und Het die oben angegebenen Bedeutungen haben, Verbindungen der Formeln (I-1-a) bis (I-2-a), in welchen A, B, Q¹, Q², Q³, Q⁴, m und Het die oben angegebenen Bedeutungen haben, jeweils
mit Metallverbindungen oder Aminen der Formeln (X) oder (XI)
Me(OR¹¹)ₜ (X) 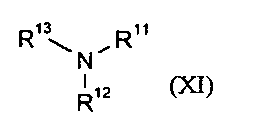
in welchen
Me für ein ein- oder zweiwertiges Metall,
t für die Zahl 1 oder 2 und
R¹¹, R¹², R¹³ unabhängig voneinander für Wasserstoff oder Alkyl stehen,
gegebenenfalls in Gegenwart eines Verdünnungsmittels umsetzt,
(I) Verbindungen der oben gezeigten Formeln (I-1-g) bis (I-2-g), in welcher A, B, L, Q¹, Q², Q³, Q⁴, m, R⁶, R⁷ und Het die oben angegebenen Bedeutungen haben, Verbindungen der oben gezeigten Formeln (I-1-a) bis (I-2-a), in welcher A, B, Q¹, Q², Q³, Q⁴, m und Het die oben angegebenen Bedeutungen haben, jeweils
(α) mit Isocyanaten oder Isothiocyanaten der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XIII) 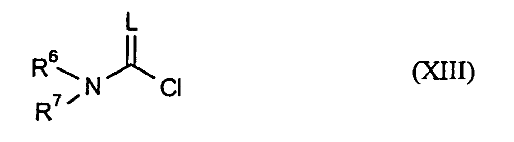 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 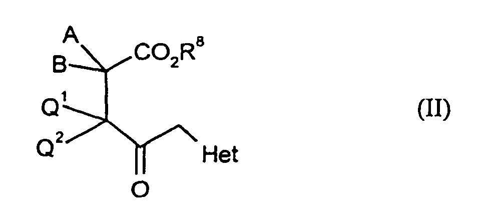 in welcher
A, B, Q¹, Q² und Het die oben angegebenen Bedeutungen haben und
R⁸ für Alkyl steht.

6. Verbindungen der Formel (III) 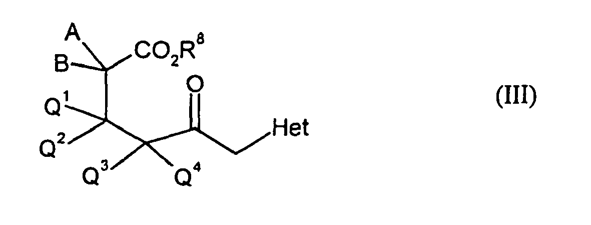 in welcher
A, B, Q¹, Q², Q³, Q⁴, Het und R⁸ die oben angegebene Bedeutung haben.

7. Verbindungen der Formel (XIV) 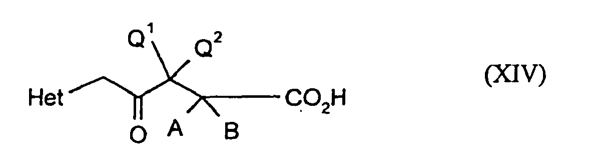 in welcher
A, B, Het, Q¹ und Q² die oben angegebene Bedeutung haben.

8. Verbindungen der Formel (XV) 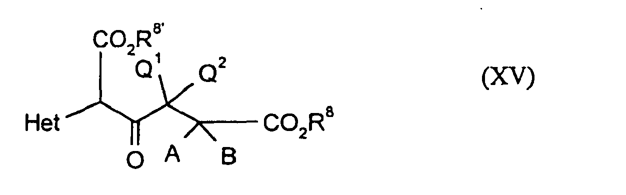 in welcher
A, B, Het, Q¹ und Q² die oben angegebene Bedeutung haben und
R⁸ und R^{8'} für Alkyl stehen.

9. Verbindungen der Formel (XIX) 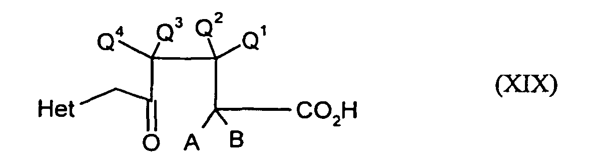 in welcher
A, B, Het, Q¹, Q², Q³ und Q⁴ die oben angegebene Bedeutung haben.

10. Verbindungen der Formel (XX) 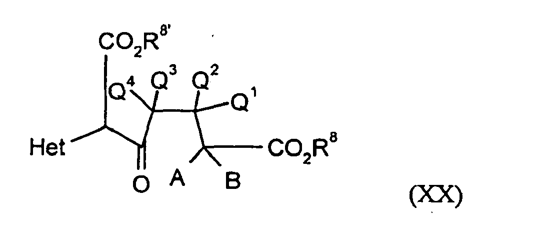 in welcher
A, B, Het, Q¹, Q², Q³, Q⁴, R⁸ und R^{8'} die oben angegebene Bedeutung haben.

11. Schädlingsbekämpfungsmittel, Herbizide und Fungizide, **gekennzeichnet durch** einen Gehalt an mindestens einer Verbindung der Formel (I) gemäß Anspruch 1.

12. Verfahren zur Bekämpfung von tierischen Schädlingen, unerwünschten Pflanzenbewuchs und Pilzen, **dadurch gekennzeichnet, daß** man Verbindungen der Formel (I) gemäß Anspruch 1 auf Schädlinge und/oder ihren Lebensraum einwirken läßt.

13. Verwendung von Verbindungen der Formel (I) gemäß Anspruch 1 zur Bekämpfung von tierischen Schädlingen, unerwünschtem Pflanzenbewuchs und Pilzen.

14. Verfahren zur Herstellung von Schädlingsbekämpfungsmitteln, Herbiziden und Fungiziden, **dadurch gekennzeichnet, daß** man Verbindungen der Formel (I) gemäß Anspruch 1 mit Streckmitteln und/oder oberflächenaktiven Stoffen vermischt.

15. Verwendung von Verbindungen der Formel (I) gemäß Anspruch 1 zur Herstellung von Schädlingsbekämpfungsmitteln, Herbiziden und Fungiziden.

## Claims

1. Compounds of the formula (I) 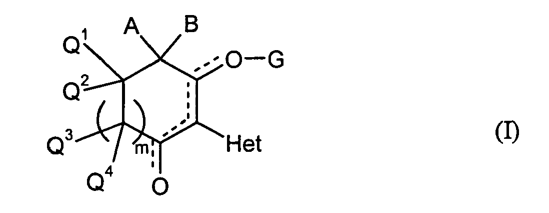 in which
Het represents 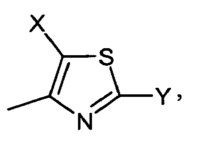
m represents the number 0 or 1,
X represents hydrogen, chlorine, bromine or C₁-C₄-alkyl,
Y represents chlorine, bromine, C₁-C₄-alkyl, C₁-C₄-halogenoalkyl, C₁-C₄-alkoxy, C₁-C₄-halogenoalkoxy or the group 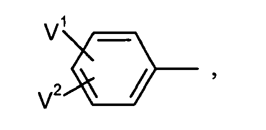
V¹ represents hydrogen, fluorine, chlorine, C₁-C₆-alkyl, C₁-C₄-alkoxy, C₁-C₂-halogenoalkyl, C₁-C₂-halogenoalkoxy, nitro, cyano or phenoxy which is optionally mono- or disubstituted by fluorine, chlorine, bromine, C₁-C₄-alkyl, C₁-C₄-alkoxy, C₁-C₂₋halogenoalkyl, C₁-C₂-halogenoalkoxy, nitro or cyano,
V² represents hydrogen, fluorine, chlorine, bromine, C₁-C₄-alkyl, C₁₋C₄-alkoxy, C₁-C₂-halogenoalkyl or C₁-C₂-halogenoalkoxy,
V¹ and V² jointly, together with the carbon atoms to which they are attached, represent an optionally fluorine- or methyl-substituted 5-or 6-membered cycle in which optionally one or two carbon atoms may be replaced by oxygen,
A represents hydrogen, represents C₁-C₈-alkyl or C₁-C₄-alkoxy-C₁₋C₂-alkyl, each of which is optionally mono- to trisubstituted by fluorine, represents C₅-C₆-cycloalkyl or C₃-C₆-cycloalkyl-C₁-C₂₋alkyl in which optionally one ring member is replaced by oxygen or sulphur, each of which radicals is optionally mono- or disubstituted by fluorine, chlorine, methyl, ethyl or methoxy, or represents phenyl or benzyl, each of which is optionally mono- or disubstituted by fluorine, chlorine, bromine, C₁-C₄-alkyl, C₁-C₂-halogenoalkyl, C₁₋C₄-alkoxy or C₁-C₂-halogenoalkoxy,
B represents hydrogen or C₁-C₄-alkyl,
A, B and the carbon atom to which they are attached represent saturated C₅-C₆-cycloalkyl in which optionally one ring member is replaced by oxygen and which is optionally monosubstituted by C₁-C₄-alkyl, trifluoromethyl or C₁-C₄-alkoxy, with the proviso that in this case Q¹ represents only hydrogen or C₁-C₄-alkyl,
A and Q¹ together represent C₃-C₄-alkanediyl which is optionally mono- or disubstituted by methyl, ethyl, methoxy or ethoxy,
Q¹ represents hydrogen, C₁-C₄-alkyl, C₁-C₄-alkoxy-C₁-C₂-alkyl, or optionally methyl- or methoxy-substituted C₃-C₆-cycloalkyl, in which optionally one methylene group is replaced by oxygen, or represents phenyl or benzyl, each of which is optionally mono- or disubstituted by fluorine, chlorine, bromine, C₁-C₄-alkyl, C₁-C₄₋alkoxy, trifluoromethyl or trifluoromethoxy,
Q², Q³, Q⁴ independently of one another represent hydrogen, methyl or ethyl,
Q¹ and Q² together with the carbon atom to which they are attached represent optionally C₁-C₄-alkyl- or C₁-C₄-alkoxy-substituted saturated C₅-C₆-cycloalkyl in which optionally one ring member is replaced by oxygen,
with the proviso that in this case A only represents hydrogen or C₁-C₄-alkyl,
G represents hydrogen (a) or represents one of the groups 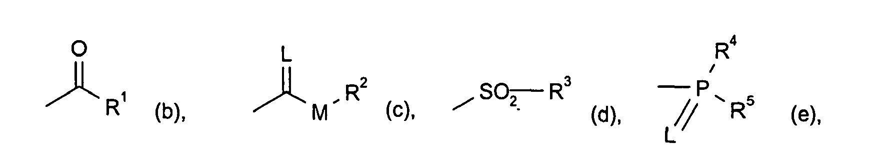 E (f) or 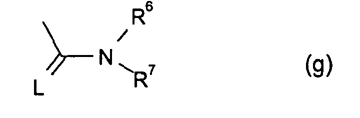 in which
E represents a metal ion or an ammonium ion,
L represents oxygen or sulphur and
M represents oxygen or sulphur,
R¹ represents C₁-C₁₆-alkyl, C₂-C₁₆-alkenyl, C₁-C₄-alkoxy-C₁-C₂₋alkyl, C₁-C₄-alkylthio-C₁-C₂-alkyl, each of which is optionally mono- to pentasubstituted by fluorine or chlorine, or C₃-C₆₋cycloalkyl in which optionally one or two not directly adjacent ring members are replaced by oxygen and/or sulphur and which is optionally mono- or disubstituted by fluorine, chlorine, C₁-C₄-alkyl or C₁-C₅-alkoxy,
represents phenyl which is optionally mono- or disubstituted by fluorine, chlorine, bromine, cyano, nitro, C₁-C₄-alkyl, C₁-C₄₋alkoxy, trifluoromethyl or trifluoromethoxy,
represents pyridyl or thienyl, each of which is optionally monosubstituted by fluorine, chlorine, bromine, methyl, ethyl or trifluoromethyl,
R² represents C₁-C₁₆-alkyl, C₂-C₁₆-alkenyl or C₁-C₄-alkoxy-C₂-C₄₋alkyl,
represents C₃-C₆-cycloalkyl which is optionally mono- or disubstituted by methyl, ethyl or methoxy,
represents phenyl or benzyl, each of which is optionally mono- or disubstituted by fluorine, chlorine, bromine, cyano, nitro, C₁-C₄₋alkyl, C₁-C₄-alkoxy, trifluoromethyl or trifluoromethoxy,
R³ represents C₁-C₄-akyl which is optionally mono- to pentasubstituted by fluorine or represents phenyl which is optionally mono- or disubstituted by fluorine, chlorine, bromine, C₁-C₄-alkyl, C₁-C₄-alkoxy, trifluoromethyl, trifluoromethoxy, cyano or nitro,
R⁴ represents C₁-C₆-akyl, C₁-C₆-alkoxy, C₁-C₆-alkylamino, di-(C₁-C₆-alkyl)amino, C₁-C₆-akylthio, or represents phenyl, benzyl, phenoxy or phenylthio, each of which is optionally mono- or disubstituted by fluorine, chlorine, bromine, nitro, cyano, C₁-C₃₋alkoxy, trifluoromethoxy, C₁-C₃-alkyl or trifluoromethyl,
R⁵ represents C₁-C₄-alkyl, C₁-C₄-alkoxy or C₁-C₄-alkylthio,
R⁶ represents hydrogen, C₁-C₆-alkyl, C₃-C₆-cycloakyl, C₁-C₆₋alkoxy, C₃-C₆-alkenyl, C₁-C₆-alkoxy-C₁-C₆-alkyl, represents phenyl which is optionally mono- or disubstituted by fluorine, chlorine, bromine, trifluoromethyl, C₁-C₄-alkyl or C₁-C₄-alkoxy, represents benzyl which is optionally substituted by fluorine, chlorine, bromine, methyl, ethyl, trifluoromethyl or methoxy,
R⁷ represents hydrogen, C₁-C₆-alkyl or C₃-C₆-alkenyl,
R⁶ and R⁷ together with nitrogen atom to which they are attached represent a C₅-C₆-alkylene radical in which optionally one methylene group is replaced by oxygen or sulphur and which is optionally mono- or disubstituted by methyl or ethyl.

2. Compounds of the formula (I) according to claim 1, in which
Het represents 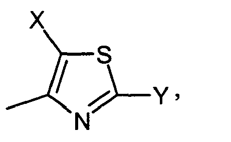
m represents the number 0 or 1,
X represents hydrogen, chlorine, bromine, methyl, ethyl, n-propyl or isopropyl,
Y represents the group 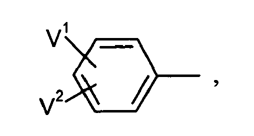
V¹ represents hydrogen, fluorine, chlorine, bromine, methyl, ethyl, n-propyl, isopropyl, n-butyl, isobutyl, tert-butyl, methoxy, ethoxy, n-propoxy, isopropoxy, trifluoromethyl, trifluoromethoxy or 4-chlorophenoxy,
V² represents hydrogen, fluorine, chlorine, bromine, methyl, methoxy or trifluoromethyl,
A represents hydrogen, methyl, ethyl, n-propyl, isopropyl, n-butyl, isobutyl, tert-butyl, methoxymethyl, ethoxymethyl, phenyl or cyclohexyl,
B represents hydrogen, methyl or ethyl,
A, B and the carbon atom to which they are attached represent saturated C₅-C₆-cycloalkyl in which optionally one ring member is replaced by oxygen and which is optionally monosubstituted by methyl, ethyl, n-propyl, isopropyl, n-butyl, isobutyl, tert-butyl, trifluoromethyl, methoxy, ethoxy, n-propoxy, n-butoxy or isobutoxy,
with the provisio that in this case Q¹ only represents hydrogen,
A and Q¹ together represent C₃-C₄-alkanediyl,
Q¹ represents hydrogen, methyl, ethyl, n-propyl, isopropyl, cyclopropyl, cyclopentyl, cyclohexyl or 4-chlorophenyl,
Q², Q³, Q⁴ independently of one another represent hydrogen, methyl or ethyl,
Q¹ and Q² together with the carbon atom to which they are attached represent optionally methyl-, ethyl-, methoxy-, ethoxy-, n-propoxy- or n-butoxy-substituted saturated C₅-C₆-cycloalkyl in which optionalloy one ring member is replaced by oxygen,
with the proviso that in this case A only represents hydrogen,
G represents hydrogen (a) or represents one of the groups 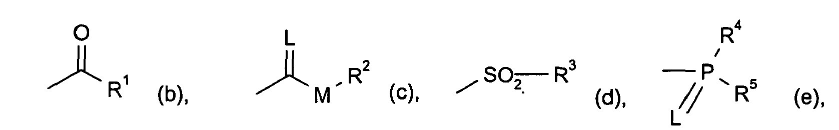 E (f) or 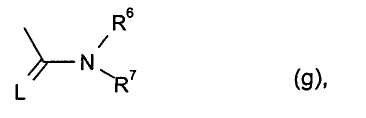 in which
E represents a metal ion or an ammonium ion,
L represents oxygen or sulphur and
M represents oxygen or sulphur,
R¹ represents in each case optionally fluorine- or chlorine-substituted C₁-C₈-alkyl, C₂-C₈-alkenyl, C₁-C₂-alkoxy-C₁-C₂-alkyl, C₁-C₂₋alkylthio-C₁-C₂-alkyl or cyclopropyl or cyclohexyl, each of which is optionally monosubstituted by fluorine, chlorine, methyl, ethyl or methoxy,
represents phenyl which is optionally monosubstituted by fluorine, chlorine, bromine, cyano, nitro, methyl, ethyl, isopropyl, tert-butyl, methoxy, trifluoromethyl or trifluoromethoxy,
represents thienyl or pyridyl, each of which is optionally monosubstituted by fluorine, chlorine, bromine or methyl,
R² represents C₁-C₈-alkyl, C₂-C₈-alkenyl or C₁-C₄-alkoxy-C₂-alkyl,
represents C₃-C₆-cycloalkyl which is optionally monosubstituted by methyl, ethyl or methoxy,
represents phenyl or benzyl, each of which is optionally monosubstituted by fluorine, chlorine, cyano, nitro, methyl, ethyl, isopropyl, tert-butyl, methoxy, trifluoromethyl or trifluoromethoxy,
R³ represents methyl or ethyl, each of which is optionally trisubstituted by fluorine or represents phenyl which is optionally monosubstituted by fluorine, chlorine, bromine, methyl, tert-butyl, methoxy, trifluoromethyl, trifluoromethoxy, cyano or nitro,
R⁴ represents C₁-C₄-alkyl, C₁-C₄-alkoxy, C₁-C₄-alkylamino, di-(C₁-C₄-alkyl)amino, C₁-C₄-alkylthio or represents phenyl, phenoxy or phenylthio, each of which is optionally monosubstituted by fluorine, chlorine, bromine, nitro, cyano, C₁-C₂-alkoxy, trifluoromethoxy or C₁-C₃-alkyl,
R⁵ represents methyl, ethyl, methoxy, ethoxy, methylthio or ethylthio,
R⁶ represents hydrogen, represents C₁-C₄-alkyl, C₃-C₆-ccycloalkyl, C₁-C₄-alkoxy, C₃-C₄-alkenyl or C₁-C₄-alkoxy-C₁-C₄-alkyl,
R⁷ represents hydrogen, C₁-C₄-alkyl or C₃-C₄-alkenyl,
R⁶ and R⁷ together with the nitrogen atom to which they are attached represent a C₅-alkylene radical in which optionally one methylene group is replaced by oxygen.

3. Compounds of the formula (I) according to claim 1, in which
Het represents 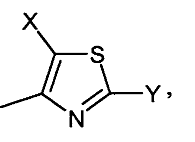
m represents the number 0 or 1,
X represents hydrogen, methyl or ethyl,
Y represents the group 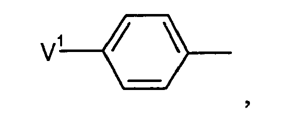
V¹ represents hydrogen, chlorine, methyl, ethyl, n-propyl, isopropyl, n-butyl, isobutyl, tert-butyl, trifluoromethyl or represents 4-chlorophenoxy,
A represents hydrogen, methyl, ethyl, n-propyl, isopropyl, n-butyl, isobutyl, phenyl or cyclohexyl,
B represents hydrogen, methyl or ethyl,
A, B and the carbon atom to which they are attached represent saturated C₅-C₆-cycloalkyl in which optionally one ring member is replaced by oxygen and which is optionally monosubstituted by methyl, ethyl, n-propyl, isopropyl, n-butyl, isobutyl, tert-butyl, methoxy, ethoxy, n-propoxy, n-butoxy or isobutoxy,
with the proviso that in this case Q¹ only represents hydrogen,
A and Q¹ together represent C₃-C₄-alkanediyl,
Q¹ represents hydrogen, methyl, ethyl, n-propyl, isopropyl or 4-chlorophenyl,
Q² represents hydrogen, methyl or ethyl,
Q³ represents hydrogen,
Q⁴ represents hydrogen,
Q¹ and Q² together with the carbon atom to which they are attached represent saturated C₅-C₆-cycloalkyl,
with the proviso that in this case A only represents hydrogen,
G represents hydrogen (a) or represents one of the groups 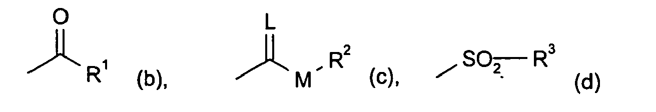 or 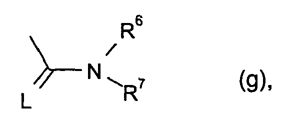 in which
L represents oxygen and
M represents oxygen or sulphur,
R¹ represents C₁-C₈-alkyl, C₁-C₂-alkoxy-C₁-C₂-alkyl or cyclopropyl which is optionally monosubstituted by chlorine,
represents phenyl which is optionally monosubstituted by chlorine,
represents pyridyl which is optionally monosubstituted by chlorine,
R² represents C₁-C₈-akyl,
represents phenyl or benzyl,
R³ represents methyl or ethyl,
R⁶ and R⁷ together with the nitrogen atom to which they are attached represent a C₅-alkylene radical in which optionally one methylene group is replaced by oxygen.

4. Process for preparing compounds of the formula (I) according to claim 1, **characterized in that**, to obtain
(A) compounds of the formula (I-1-a) 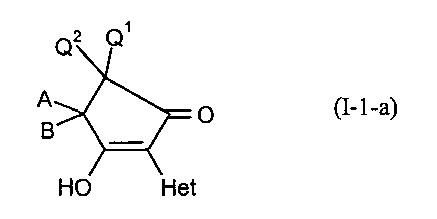 in which
A, B, Q¹, Q² and Het are as defined above,
compounds of the formula (II) 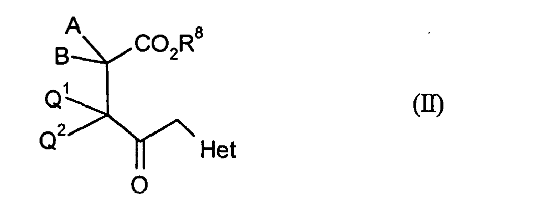 in which
A, B, Q¹, Q² and Het are as defined above
and
R⁸ represents alkyl,
are condensed intramolecularly, if appropriate in the presence of a diluent and in the presence of a base,
(B) compounds of the formula (I-2-a) 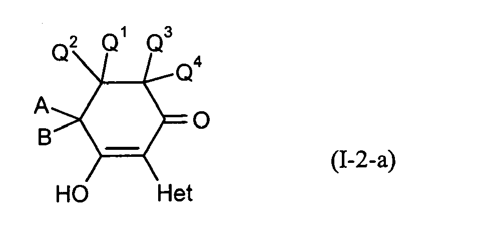 in which
A, B, Q¹, Q², Q³, Q⁴, and Het are as defined above,
compounds of the formula (III) 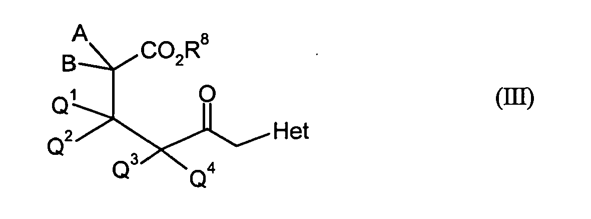 in which
A, B, Q¹, Q², Q³, Q⁴ and Het are as defined above
and
R⁸ represents alkyl,
are condensed intramolecularly in the presence of a diluent and in the presence of a base,
(C) compounds of the formulae (I-1-b) to (I-2-b) shown above in which A, B, Q¹, Q², Q³, Q⁴, m, R¹ and Het are as defined above, compounds of the formulae (I-1-a) to (1-2-a) shown above in which A, B, Q¹, Q², Q³, Q⁴, m and Het are as defined above are in each case
(α) reacted with acid halides of the formula (IV) 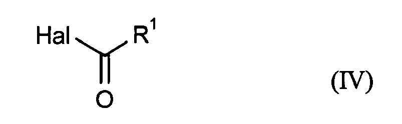 in which
R¹ is as defined above and
Hal represents halogen
or
(β) reacted with carboxylic anhydrides of the formula (V)
R¹-CO-O-CO-R¹ (V)
in which
R¹ is as defined above,
if appropriate in the presence of a diluent and if appropriate in the presence of an acid binder;
(D) compounds of the formulae (I-1-c) to (I-2-c) shown above in which A, B, Q¹, Q², Q³, Q⁴, m, R², M and Het are as defined above and L represents oxygen, compounds of the formulae (I-1-a) to (I-2-a) shown above in which A, B, Q¹, Q², Q³, Q⁴, m and Het are as defined above are in each case
reacted with chloroformic esters or chloroformic thioesters of the formula (VI)
R²-M-CO-Cl (VI)
in which
R² and M are as defined above,
if appropriate in the presence of a diluent and if appropriate in the presence of an acid binder;
(E) compounds of the formulae (I-1-c) to (I-2-c) shown above in which A, B, Q¹, Q², Q³, Q⁴, m, R², M and Het are as defined above and L represents sulphur, compounds of the formulae (I-1-a) to (I-2-a) shown above in which A, B, Q¹, Q², Q³, Q⁴, m and Het are as defined above are in each case
reacted with chloromonothioformic esters or chlorodithioformic esters of the formula (VII) 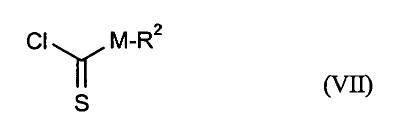 in which
M and R² are as defined above,
if appropriate in the presence of a diluent and if appropriate in the presence of an acid binder
and
(F) compounds of the formulae (I-1-d) to (I-2-d) shown above in which A, B, Q¹, Q², Q³, Q⁴, m, R³ and Het are as defined above, compounds of the formulae (I-1-a) to (I-2-a) shown above in which A, B, Q¹, Q², Q³, Q⁴, m and Het are as defined above are in each case
reacted with sulphonyl chlorides of the formula (VIII)
R³-SO₂-Cl (VIII)
in which
R³ is as defined above,
if appropriate in the presence of a diluent and if appropriate in the presence of an acid binder,
(G) compounds of the formulae (I-1-e) to (I-2-e) shown above in which A, B, L, Q¹, Q², Q³, Q⁴, m, R⁴, R⁵ and Het are as defined above, compounds of the formulae (I-1-a) to (I-2-a) shown above in which A, B, Q¹, Q², Q³, Q⁴, m and Het are as defined above are in each case
reacted with phosphorus compounds of the formula (IX) 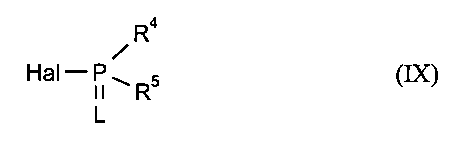 in which
L, R⁴ and R⁵ are as defined above and
Hal represents halogen,
if appropriate in the presence of a diluent and if appropriate in the presence of an acid binder,
(H) compounds of the formulae (I-1-f) to (I-2-f) shown above in which A, B, E, Q¹, Q², Q³, Q⁴, m and Het are as defined above, compounds of the formulae (I-1-a) to (I-2-a) in which A, B, Q¹, Q², Q³, Q⁴, m and Het are as defined above are in each case
reacted with metal compounds or amines of the formulae (X) and (XI), respectively,
Me(OR¹¹)ₜ (X) 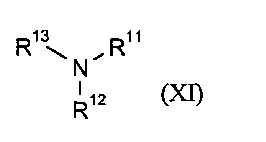
in which
Me represents a mono- or divalent metal,
t represents the number 1 or 2 and
R¹¹, R¹², R¹³ independently of one another represent hydrogen or alkyl,
if appropriate in the presence of a diluent,
(I) compounds of the formulae (I-1-g) to (I-2-g) shown above in which A, B, L, Q¹, Q², Q³, Q⁴, m, R⁶, R⁷ and Het are as defined above, compounds of the formulae (I-1-a) to (I-2-a) shown above in which A, B, Q¹, Q², Q³, Q⁴, m and Het are as defined above are in each case
(α) reacted with isocyanates or isothiocyanates of the formula (XII)
R⁶-N=C=L (XII)
in which
R⁶ and L are as defined above,
if appropriate in the presence of a diluent and if appropriate in the presence of a catalyst, or
(β) reacted with carbamoyl chlorides or thiocarbamoyl chlorides of the formula (XIII) 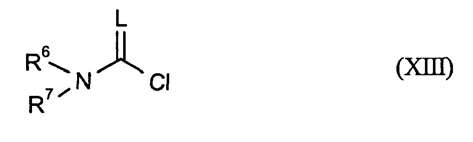
in which
L, R⁶ and R⁷ are as defined above,
if appropriate in the presence of a diluent and if appropriate in the presence of an acid binder.

5. Compounds of the formula (II) 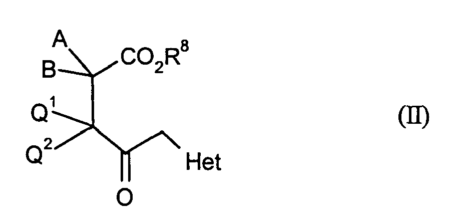 in which
A, B, Q¹, Q² and Het are as defined above and
R⁸ represents alkyl.

6. Compounds of the formula (III) 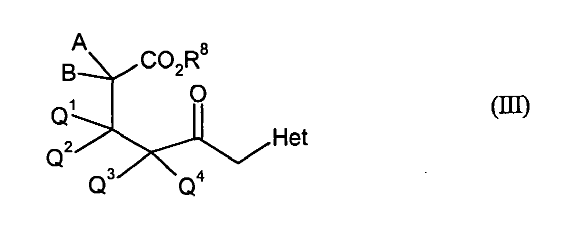 in which
A, B, Q¹, Q², Q³, Q⁴, Het and R⁸ are as defined above.

7. Compounds of the formula (XIV) 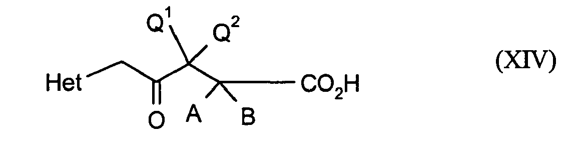 in which
A, B, Het, Q and Q² are as defined above.

8. Compounds of the formula (XV) 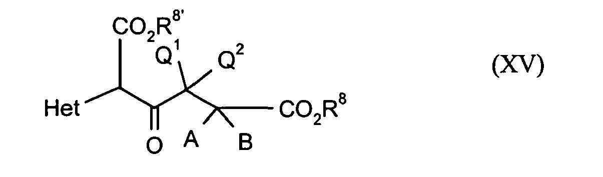 in which
A, B, Het, Q¹ and Q² are as defined above and
R⁸ and R^{8'} represent alkyl.

9. Compounds of the formula (XIX) 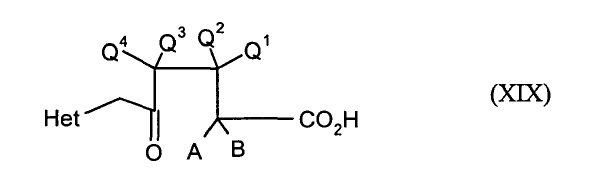 in which
A, B, Het, Q¹, Q², Q³ and Q⁴ are as defined above.

10. Compounds of the formula (XX) 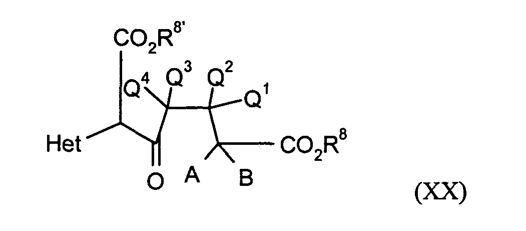 in which
A, B, Het, Q¹, Q², Q³, Q⁴, R⁸ and R⁸' are as defined above.

11. Pesticides, herbicides and fungicides, **characterized in that** they comprise at least one compound of the formula (I) according to claim 1.

12. Method for controlling animal pests, undesirable vegetation and fungi, **characterized in that** compounds of the formula (I) according to claim 1 are allowed to act on pests and/or their habitat.

13. Use of compounds of the formula (I) according to claim 1 for controlling animal pests, undesirable vegetation and fungi.

14. Process for preparing pesticides, herbicides and fungicides, **characterized in that** compounds of the formula (I) according to claim 1 are mixed with extenders and/or surfactants.

15. Use of compounds of the formula (I) according to claim 1 for preparing pesticides, herbicides and fungicides.

## Revendications

1. Composés de formule (I) 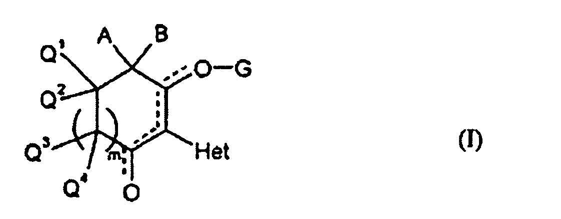 dans laquelle
Het représente 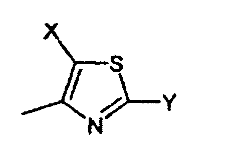
m représente les nombres 0 ou 1
X représente l'hydrogène, le chlore, le brome ou un reste alkyle en C₁ à C₄,
Y représente le chlore, le brome, un reste alkyle en C₁ à C₄, halogénalkyle en C₁ à C₄, alkoxy en C₁ à C₄, halogénalkoxy en C₁ à C₄ ou le groupe 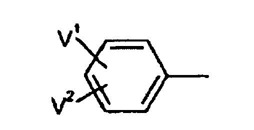
V¹ représente l'hydrogène, le fluor, le chlore, un reste alkyle en C₁ à C₆, alkoxy en C₁ à C₄, halogénalkyle en C₁ ou C₂, halogénalkoxy en C₁ ou C₂, nitro, cyano ou un reste phénoxy éventuellement substitué une ou deux fois par du fluor, du chlore, du brome, un radical alkyle en C₁ à C₄, alkoxy en C₁ à C₄, halogénalkyle en C₁ ou C₂, halogénalkoxy en C₁ ou C₂, nitro ou cyano,
V² représente l'hydrogène, le fluor, le chlore, le brome, un reste alkyle en C₁ à C₄, alkoxy en C₁ à C₄, halogénalkyle en C₁ ou C₂ ou halogénalkoxy en C₁ ou C₂,
V¹ et V² forment ensemble, conjointement avec l'atome de carbone auquel ils sont liés, un cycle pentagonal ou hexagonal éventuellement substitué par du fluor ou par un radical méthyle et dans lequel, le cas échéant, un ou deux atomes de carbone peuvent être remplacés par de l'oxygène,
A représente l'hydrogène, un reste alkyle en C₁ à C₈ ou (alkoxy en C₁ à C₄)-(alkyle en C₁ ou C₂), chacun éventuellement substitué une à trois fois par du fluor, un reste cycloalkyle en C₅ ou C₆ ou (cycloalkyle en C₃ à C₆) - (alkyle en C₁ ou C₂), chacun éventuellement substitué une ou deux fois par du fluor, du chlore, un radical méthyle, éthyle ou méthoxy et dans lequel, le cas échéant, un chaînon du noyau est remplacé par de l'oxygène ou du soufre, ou un reste phényle ou benzyle, chacun éventuellement substitué une ou deux fois par du fluor, du chlore, du brome, un radical alkyle en C₁ à C₄, halogénalkyle en C₁ ou C₂, alkoxy en C₁ à C₄ ou halogénalkoxy en C₁ ou C₂,
B représente l'hydrogène ou un reste alkyle en C₁ à C₄,
A, B et l'atome de carbone auquel ils sont liés forment un reste cycloalkyle saturé en C₅ ou C₆ dans lequel, le cas échéant, un chaînon du noyau est remplacé par de l'oxygène et qui est substitué éventuellement une fois par un radical alkyle en C₁ à C₄, trifluorométhyle ou alkoxy en C₁ à C₄,
sous réserve que Q¹ ne représente alors que l'hydrogène ou un radical alkyle en C₁ à C₄,
A et Q¹ forment ensemble un groupe alcanediyle en C₃ ou C₄ éventuellement substitué une ou deux fois par un radical méthyle, éthyle, méthoxy ou éthoxy,
Q¹ représente l'hydrogène, un reste alkyle en C₁ à C₄, (alkoxy en C₁ à C₄)-(alkyle en C₁ ou C₂), ou un reste cycloalkyle en C₃ à C₆ éventuellement substitué par un radical méthyle ou méthoxy, dans lequel le cas échéant un groupe méthylène est remplacé par de l'oxygène, ou bien un reste phényle ou benzyle, chacun éventuellement substitué une ou deux fois par du fluor, du chlore, du brome, un radical alkyle en C₁ à C₄, alkoxy en C₁ à C₄, trifluorométhyle ou trifluorométhoxy,
Q², Q³, Q⁴ représentent, indépendamment les uns des autres, l'hydrogène, un reste méthyle ou éthyle,
Q¹ et Q₂ forment, conjointement avec l'atome de carbone auquel ils sont liés, un reste cycloalkyle en C₅ ou C₆ saturé éventuellement substitué par un radical alkyle en C₁ à C₉ ou alkoxy en C₁ à C₉, dans lequel, le cas échéant, un chaînon du noyau est remplacé par de l'oxygène,
sous réserve que A ne représente alors que l'hydrogène ou un radical alkyle en C₁ à C₄,
G représente l'hydrogène (a) ou l'un des groupes 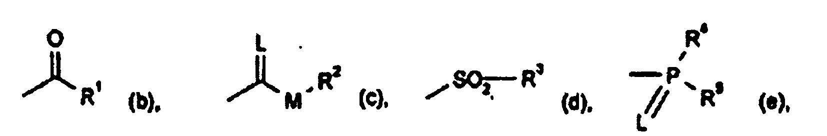 **E (f)** ou 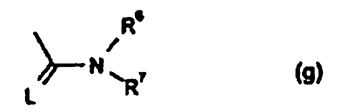 dans lesquels
E représente un ion métallique ou un ion ammonium
L représente l'oxygène ou le soufre et
M représente l'oxygène ou le soufre,
R¹ représente un reste alkyle en C₁ à C₁₆, alcényle en C₂ à C₁₆, (alkoxy en C₁ à C₄)-(alkyle en C₁ ou C₂), (alkylthio en C₁ à C₄)-(alkyle en C₁ ou C₂), chacun éventuellement substitué une à cinq fois par du fluor ou du chlore, ou un reste cycloalkyle en C₃ à C₆ éventuellement substitué une ou deux fois par du fluor, du chlore, un radical alkyle en C₁ à C₄ ou alkoxy en C₁ à C₅ et dans lequel, le cas échéant, un chaînon du noyau ou deux chaînons non contigus sont remplacés par de l'oxygène et/ou du soufre,
un reste phényle éventuellement substitué une ou deux fois par du fluor, du chlore, du brome, un radical cyano, nitro, alkyle en C₁ à C₄, alkoxy en C₁ à C₄, trifluorométhyle ou trifluorométhoxy,
un reste pyridyle ou thiényle chacun éventuellement substitué une fois par du fluor, du chlore, du brome, un radical méthyle, éthyle ou trifluorométhyle,
R² représente un reste alkyle en C₁ à C₁₆, alcényle en C₂ à C₁₆ ou (alkoxy en C₁ à C₄) -(alkyle en C₁ à C₄),
un reste cycloalkyle en C₃ à C₆ éventuellement substitué une ou deux fois par un radical méthyle, éthyle ou méthoxy,
un reste phényle ou benzyle chaun éventuellement substitué une ou deux fois par du fluor, du chlore, du brome, un radical cyano, nitro, alkyle en C₁ à C₄, alkoxy en C₁ à C₄, trifluorométhyle ou trifluorométhoxy,
R³ représente un reste alkyle en C₁ à C₄ éventuellement substitué une à cinq fois par du fluor ou un reste phényle éventuellement substitué une ou deux fois par du fluor, du chlore, du brome, un radical alkyle en C₁ à C₄, alkoxy en C₁ à C₄, trifluorométhyle, trifluorométhoxy, cyano ou nitro,
R⁴ représente un reste alkyle en C₁ à C₆, alkoxy en C₁ à C₆, alkylamino an C₁ à C₆, di (alkyle en C₁ à C₆) amino, alkylthio en C₁ à C₆, ou un reste phényle, benzyle, phénoxy ou phénylthio, chacun éventuellement substitué une ou deux fois par du fluor, du chlore, du brome, un radical nitro, cyano, alkoxy en C₁ à C₃, trifluorométhoxy, alkyle en C₁ à C₃ ou trifluorométhyle,
R⁵ représente un reste alkyle en C₁ à C₄, alkoxy en C₁ à C₄ ou alkylthio en C₁ à C₄,
R⁶ représente l'hydrogène, un reste alkyle en C₁ à C₆, cycloalkyle en C₃ à C₆, alkoxy en C₁ à C₆, alcényle en C₃ à C₆, (alkoxy en C₁ à C₆)-(alkyle en C₁ à C₆), un reste phényle éventuellement substitué une ou deux fois par du fluor, du chlore, du brome, un radical trifluorométhyle, alkyle en C₁ à C₄ ou alkoxy en C₁ à C₄, un reste benzyle éventuellement substitué par du fluor, du chlore, du brome, un radical méthyle, éthyle, trifluorométhyle ou méthoxy,
R⁷ représente l'hydrogène, un radical alkyle en C₁ à C₆ ou alcényle en C₃ à C₆,
R⁶ et R⁷ forment, conjointement avec l'atome d'azote auquel ils sont liés, un reste alkylène en C₅ ou C₆, éventuellement substitué une ou deux fois par un radical méthyle ou éthyle et dans lequel un groupe méthylène est éventuellement remplacé par l'oxygène ou le soufre.

2. Composés de formule (I) suivant la revendication 1, dans laquelle
Het représente 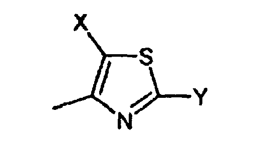
m représente les nombres 0 ou 1,
X représente l'hydrogène, le chlore, le brome, un reste méthyle, éthyle, n-propyle ou isopropyle,
Y représente le groupe 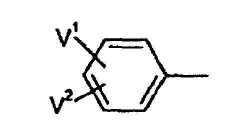
V¹ représente l'hydrogène, le fluor, le chlore, le brome, un reste méthyle, éthyle, n-propyle, isopropyle, n-butyle, isobutyle, tertiobutyle, méthoxy, éthoxy, n-propoxy, isopropoxy, trifluorométhyle, trifluorométhoxy ou 4-chlorophénoxy,
v² représente l'hydrogène, le fluor, le chlore, un reste méthyle, méthoxy ou trifluotométhyle,
A représente l'hydrogène, un reste méthyle, éthyle, n-propyle, isopropyle, n-butyle, isobutyle, tertio-butyle, méthoxyméthyle éthoxyméthyle, phényle ou cyclohexyle,
B représente l'hydrogène, un reste méthyle ou éthyle,
A, B et l'atome de carbone auquel ils sont liés forment un reste cycloalkyle en C₅ ou C₆ dans lequel, le cas échéant, un chaînon du noyau est remplacé par de l'oxygène et qui est substitué éventuellement une fois par un radical méthyle, éthyle, n-propyle, isopropyle, n-butyle, isobutyle, tertiobutyle, trifluorométhyle, méthoxy, éthoxy, n-propoxy, n-butoxy ou isobutoxy,
sous réserve que Q¹ ne représente alors que l'hydrogène,
A et Q¹ forment ensemble un groupe alcanediyle en C₃ ou C₄,
Q¹ représente l'hydrogène, un reste méthyle, éthyle, n-propyle, isopropyle, cyclopropyle, cyclopentyle, cyclohexyle ou 4-chlorophényle,
Q², Q³, Q⁴ représentent, indépendamment les uns des autres, l'hydrogène, un reste méthyle ou éthyle,
Q¹ et Q₂ forment, conjointement avec l'atome de carbone auquel ils sont liés, un reste cycloalkyle en C₅ ou C₆ saturé éventuellement substitué par un radical méthyle, éthyle, méthoxy, éthoxy, n-propoxy ou n-butoxy, dans lequel, le cas échéant, un chaînon du noyau est remplacé par de l'oxygène,
sous réserve que A ne représente alors que l'hydrogène,
G représente l'hydrogène (a) ou l'un des groupes 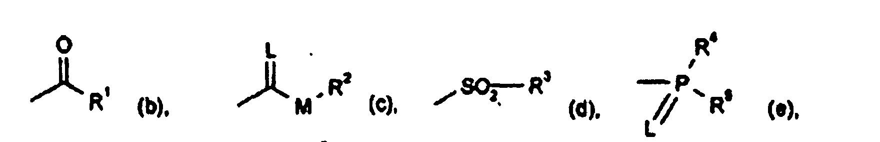 **E (f)** ou 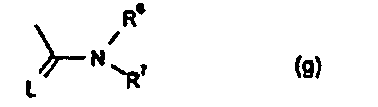 dans lesquels
E représente un ion métallique ou un ion ammonium
L représente l'oxygène ou le soufre et
M représente l'oxygène ou le soufre,
R¹ représente un reste alkyle en C₁ à C₈, alcényle en C₂ à C₈, (alkoxy en C₁ ou C₂) - (alkyle en C₁ ou C₂), (alkylthio en C₁ ou C₂)-(alkyle en C₁ ou C₂) chacun éventuellement substitué par du fluor ou du chlore, ou un reste cyclopropyle ou cyclohexyle chacun éventuellement substitué une ou deux fois par du fluor, du chlore, un radical méthyle, éthyle ou méthoxy,
un reste phényle éventuellement substitué une ou deux fois par du fluor, du chlore, du brome, un radical cyano, nitro, méthyle, éthyle, isopropyle, tertio-butyle, méthoxy, trifluorométhyle ou trifluorométhoxy, un reste thiényle ou pyridyle chacun éventuellement substitué une fois par du fluor, du chlore, du brome ou un radical méthyle,
R² représente un reste alkyle en C₁ à C₈, alcényle en C₂ à C₈ ou (alkoxy en C₁ ou C₄) - (alkyle en C₂),
un reste cycloalkyle en C₃ à C₆ éventuellement substitué une fois par un radical méthyle, éthyle ou méthoxy,
un reste phényle ou benzyle chacun éventuellement substitué une fois par du fluor, du chlore, un radical cyano, nitro, méthyle, éthyle, iropropyle, tertio-butyle, méthoxy, trifluorométhyle ou trifluorométhoxy,
R³ représente un reste méthyle ou éthyle éventuellement substitué trois fois par du fluor ou un reste phényle éventuellement substitué une fois par du fluor, du chlore, du brome, un radical méthyle, tertiobutyle, méthoxy, trifluorométhoxy, cyano ou nitro,
R⁴ représente un reste alkyle en C₁ à C₄, alkoxy en C₁ à C₄, alkylamino an C₁ à C₄, di (alkyle en C₁ à C₄)amino, alkylthio en C₁ à C₄, ou un reste phényle, phénoxy ou phénylthio, chacun éventuellement substitué une fois par du fluor, du chlore, du brome, du nitro, un radical cyano, alkoxy en C₁ ou C₂, trifluorométhoxy ou alkyle en C₁ à C₃,
R⁵ représente un reste méthyle, éthyle, méthoxy, méthylthio ou éthylthio,
R⁶ représente l'hydrogène, un reste alkyle en C₁ à C₄, cycloalkyle en C₃ à C₆, alkoxy en C₁ à C₄, alcényle en C₃ ou C₄ ou (alkoxy en C₁ à C₄) - (alkyle en C₁ à C₄),
R⁷ représente l'hydrogène, un radical alkyle en C₁ à C₄ ou alcényle en C₃ ou C₄,
R⁶ et R⁷ forment, conjointement avec l'atome d'azote auquel ils sont liés, un reste alkylène en C₅ dans lequel un groupe méthylène est éventuellement remplacé par l'oxygène.

3. Composés de formule (I) suivant la revendication 1, dans laquelle
Het représente 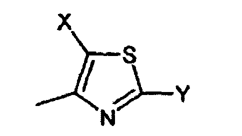
m représente les nombres 0 ou 1
X représente l'hydrogène, un reste méthyle ou éthyle,
Y représente le groupe 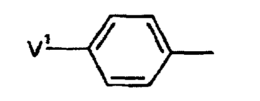
V¹ représente l'hydrogène, le chlore, un reste méthyle, éthyle, n-propyle, isopropyle, n-butyle, isobutyle, tertio-butyle, trifluorométhyle ou 4-chlorophénoxy,
A représente l'hydrogène, un reste méthyle, éthyle, n-propyle, isopropyle, n-butyle, phényle ou cyclohexyle,
B représente l'hydrogène, un reste méthyle ou éthyle,
A, B et l'atome de carbone auquel ils sont liés forment un reste cycloalkyle saturé en C₅ ou C₆ dans lequel, le cas échéant, un chaînon du noyau est remplacé par de l'oxygène et qui est substitué éventuellement une fois par un radical méthyle, éthyle, n-propyle, isopropyle, n-butyle, isobutyle, tertiobutyle, méthoxy, éthoxy, n-propoxy, n-butoxy ou isobutoxy,
sous réserve que Q¹ ne représente alors que l'hydrogène
A et Q¹ forment ensemble un groupe alcanediyle en C₃ ou C₄
Q¹ représente l'hydrogène, un reste méthyle, éthyle, n-propyle, isopropyle ou 4-chlorophényle,
Q² est l'hydrogène, un reste méthyle ou éthyle,
Q³ représente l'hydrogène,
Q⁴ représente l'hydrogène,
Q¹ et Q₂ forment, conjointement avec l'atome de carbone auquel ils sont liés, un reste cycloalkyle en C₅ ou C₆ saturé,
sous réserve que A ne représente alors que l'hydrogène,
G représente l'hydrogène (a) ou l'un des groupes 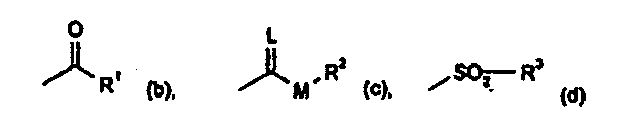 ou 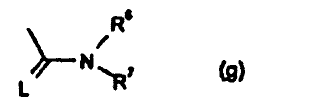 dans lesquels
L représente l'oxygène et
M représente l'oxygène ou le soufre,
R¹ représente un reste alkyle en C₁ à C₈, (alkoxy en C₁ ou C₂) -(alkyle en C₁ ou C₂) ou un reste cyclopropyle éventuellement substitué une fois par du chlore,
un reste phényle éventuellement substitué une fois par du chlore,
un reste pyridyle éventuellement substitué une fois par du chlore,
R² représente un reste alkyle en C₁ à C₈,
un reste phényle ou benzyle,
R³ représente un reste méthyle ou éthyle,
R⁶ et R⁷ forment, conjointement avec l'atome d'azote auquel ils sont liés, un reste alkylène en C₅ dans lequel un groupe méthylène est éventuellement remplacé par l'oxygène.

4. Procédé de production de composés de formule (I) suivant la revendication 1, **caractérisé en ce que**,
(A) pour l'obtention de composés de formule (I-1-a) 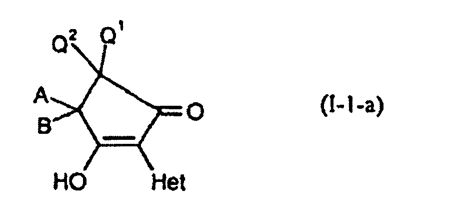 dans laquelle
A, B, Q¹, Q², et Het ont les définitions indiquées ci-dessus,
on conduit la condensation intramoléculaire de composés de formule (II) 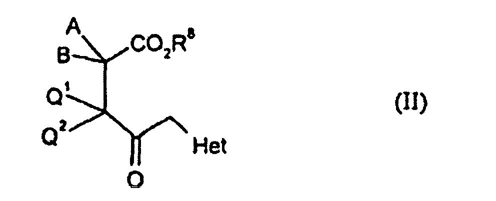 dans laquelle
A, B, Q¹ et Q² ont les définitions indiquées ci-dessus,
et
R⁸ est un reste alkyle,
éventuellement en présence d'un diluant et en présence d'une base,
(B) pour l'obtention de composés de formule (I-2-a) 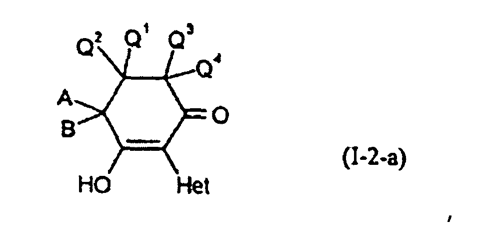 dans laquelle
A, B, Q¹, Q², Q³, Q⁴ et Het ont les définitions indiquées ci-dessus,
on conduit la condensation intramoléculaire de composés de formule (III) 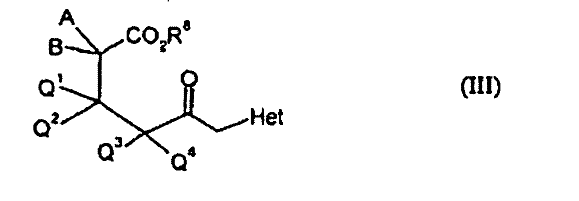 dans laquelle
A, B, Q¹ Q², Q³, Q⁴ et Het ont les définitions indiquées ci-dessus,
et
R⁸ est un reste alkyle,
en présence d'un diluant et en présence d'une base,
(C) pour l'obtention de composés des formules (I-1-b) à (I-2-b) représentées ci-dessus, dans lesquelles A, B, Q¹, Q², Q³, Q⁴, m, R¹ et Het ont les définitions indiquées ci-dessus, on fait réagir des composés des formules (I-1-a) à (I-2-a) dans lesquelles A, B, Q¹, Q², Q³, Q⁴, m et Het ont les définitions indiquées ci-dessus, respectivement
(α) avec des halogénures d'acides de formule (IV) 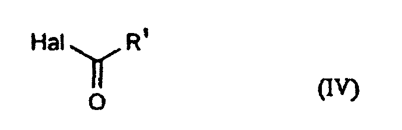 dans laquelle
R¹ a la définition indiquée ci-dessus et
Hal représente un halogène
ou bien
(β) avec des anhydrides d'acides carboxyliques de formule (V)
R¹-CO-O-CO-R¹ (V)
dans laquelle
R¹ a la définition indiquée ci-dessus
éventuellement en présence d'un diluant et, le cas échéant, en présence d'un accepteur d'acide ;
(D) pour l'obtention de composés des formules (I-1-c) à (I-2-c) représentées ci-dessus, dans lesquelles A, B, Q¹, Q², Q³, Q⁴, m, R², M et Het ont les définitions indiquées ci-dessus et L représente l'oxygène, on fait réagir des composés des formules (I-1-a) à (I-2-a) représentées ci-dessus dans lesquelles A, B, Q¹, Q², Q³, Q⁴, m et Het ont les définitions indiquées ci-dessus, respectivement
avec des esters d'acide chloroformique ou des thioesters d'acide chloroformique de formule (VI)
R²-M-CO-Cl (VI)
dans laquelle
R² et M ont les définitions indiquées ci-dessus,
éventuellement en présence d'un diluant et, le cas échéant, en présence d'un accepteur d'acide ;
(E) pour l'obtention de composés des formules (I-1-c) à (I-2-c) représentées ci-dessus, dans lesquelles A, B, Q¹, Q², Q³, Q⁴, m, R², M et Het ont les définitions indiquées ci-dessus, on fait réagir des composés des formules (I-1-a) à (I-2-a) dans lesquelles A, B, Q¹, Q², Q³, Q⁴, m et Het ont les définitions indiquées ci-dessus, respectivement
avec des esters d'acide chloromonothioformique ou des esters d'acide chlorodithioformique de formule (VII) 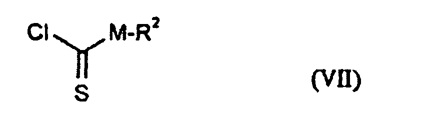 dans laquelle
M et R² ont les définitions indiquées ci-dessus,
éventuellement en présence d'un diluant et, le cas échéant, en présence d'un accepteur d'acide ;
et
(F) pour l'obtention de composés des formules (I-1-d) à (I-2-d) représentées ci-dessus, dans lesquelles A, B, Q¹, Q², Q³, Q⁴, m, R³ et Het ont les définitions indiquées ci-dessus, on fait réagir des composés des formules (I-1-a) à (I-2-a) dans lesquelles A, B, Q¹, Q², Q³, Q⁴, m et Het ont les définitions indiquées ci-dessus, respectivement
avec des chlorures d'acides sulfoniques de formule (VIII)
R³-SO₂-Cl (VIII)
dans laquelle
R³ a la définition indiquée ci-dessus,
éventuellement en présence d'un diluant et, le cas échéant, en présence d'un accepteur d'acide ;
(G) pour l'obtention de composés des formules (I-1-e) à (I-2-e) représentées ci-dessus, dans lesquelles A, B, L, Q¹, Q², Q³, Q⁴, m , R⁴, R⁵ et Het ont les définitions indiquées ci-dessus, on fait réagir des composés des formules (I-1-a) à (I-2-a)représentées ci-dessus dans lesquelles A, B, Q¹, Q², Q³, Q⁴, m et Het ont les définitions indiquées ci-dessus, respectivement avec des composés de phosphore de formule (IX) 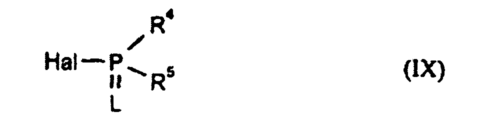 dans laquelle
L, R⁴ et R⁵ ont les définitions indiquées ci-dessus et Hal représente un halogène,
éventuellement en présence d'un diluant et, le cas échéant en présence d'un accepteur d'acide,
(H) pour l'obtention de composés des formules (I-1-f) à (I-2-f) représentées ci-dessus, dans lesquelles A, B, E, Q¹, Q², Q³, Q⁴, m et Het ont les définitions indiquées ci-dessus, on fait réagir des composés des formules (I-1-a) à (I-2-a) représentées ci-dessus, dans lesquelles A, B, Q¹, Q², Q³, Q⁴, m et Het ont les définitions indiquées ci-dessus, respectivement avec des composés métalliques ou des amines de formules (X) ou (XI)
Me(OR¹¹)ₜ (X) 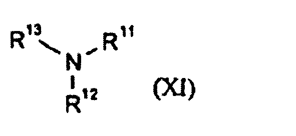
dans lesquelles
Me représente un métal monovalent ou divalent,
t représente le nombre 1 ou 2 et
R¹¹, R¹², R¹³ représentent, indépendamment les uns des autres, l'hydrogène ou un reste alkyle,
éventuellement en présence d'un diluant ;
(I) pour l'obtention de composés des formules (I-1-g) à (1-2-g) représentées ci-dessus, dans lesquelles A, B, L, Q¹, Q², Q³, Q⁴, m , R⁶, R⁷ et Het ont les définitions indiquées ci-dessus, on fait réagir des composés des formules (I-1-a) à (I-2-a) dans lesquelles A, B, Q¹, Q², Q³, Q⁴, m et Het ont les définitions indiquées ci-dessus, respectivement
(α) avec des isocyanates ou des isothiocyanates de formule (XII)
**R**^{**6**}**-N=C=L** **(XII)**
dans laquelle
R⁶ et L ont les définitions indiquées ci-dessus,
éventuellement en présence d'un diluant et, le cas échéant, en présence d'un catalyseur, ou bien
(β) avec des chlorures d'acides carbamiques ou des chlorures d'acides thiocarbamiques de formule (XIII) 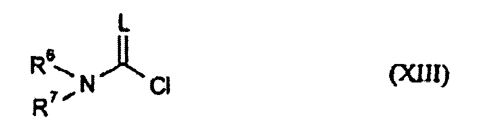
dans laquelle
L, R⁶ et R⁷ ont les définitions indiquées ci-dessus,
éventuellement en présence d'un diluant et, le cas échéant, en présence d'un accepteur d'acide.

5. Composés de formule (II) 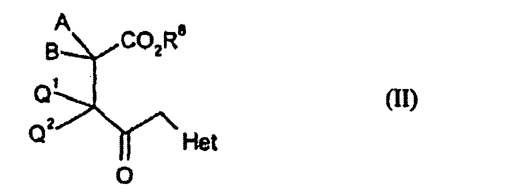 dans laquelle
A, B, Q¹, Q² et Het ont les définitions indiquées ci-dessus et
R⁸ est un reste alkyle.

6. Composés de formule (III) 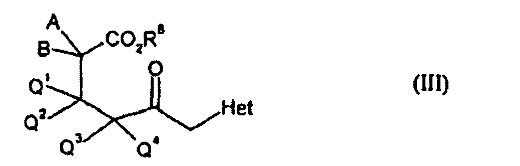 dans laquelle
A, B, Q¹, Q², Q³, Q⁴, Het et R⁸ ont la définition indiquée ci-dessus.

7. Composés de formule (XIV) 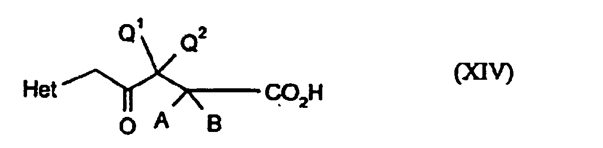 dans laquelle
A, B, Het, Q¹ et Q² ont la définition indiquée ci-dessus.

8. Composés de formule (XV) 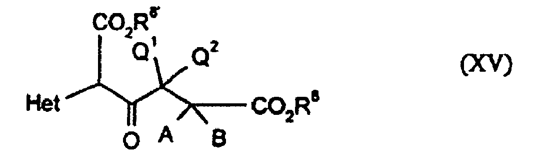 dans laquelle
A, B, Het, Q¹ et Q² ont la définition indiquée ci-dessus et
R⁸ et R^{8'} sont des groupes alkyle.

9. Composés de formule (XIX) 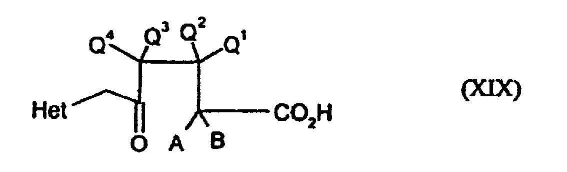 dans laquelle
A, B, Het, Q¹, Q², Q³ et Q⁴ ont la définition indiquée ci-dessus.

10. Composés de formule (XX) 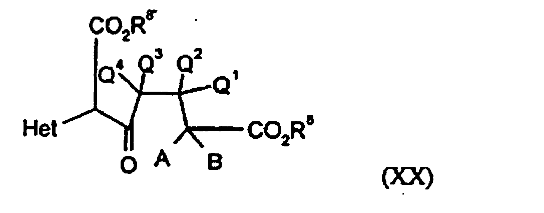 dans laquelle
A, B, H et, Q¹, Q², Q³, Q⁴, R⁸ et R^{8'} ont la définition indiquée ci-dessus

11. Pesticides, herbicides et fongicides, **caractérisés par** une teneur en au moins un composé de formule (I) suivant la revendication 1.

12. Procédé pour combattre des parasites animaux, la croissance de plantes indésirables et des champignons, **caractérisé en ce qu'**on fait agir des composés de formule (I) suivant la revendication 1 sur les parasites et/ou sur leur milieu.

13. Utilisation de composés de formule (I) suivant la revendication 1 pour combattre des parasites animaux, la croissance de plantes indésirables et des champignons.

14. Procédé de préparation de pesticides, d'herbicides et de fongicides, **caractérisé en ce qu'**on mélange des composés de formule (I) suivant la revendication 1 avec des diluants et/ou des agents tensioactifs.

15. Utilisation de composés de formule (I) suivant la revendication 1 pour la préparation de pesticides, d'herbicides et de fongicides.
